# EUROPEAN PATENT APPLICATION

(11) **EP 4 825 852 A1**
(43) Date of publication of application: **21.10.2026**
(21) Application number: 25747778.6
(22) Date of filing: 24.01.2025
(51) Int. Cl.: C07D 401/14, A61K 31/506, A61P 35/00

(54) **COMPOUND HAVING PROTEIN TYROSINE KINASE BTK-DEGRADING ACTIVITY, AND PREPARATION METHOD THEREFOR AND USE THEREOF**

(30) Priority: 04.02.2024 CN 202410158821; 02.07.2024 CN 202410875903
(71) Applicant: Shanghai Helioson Pharmaceutical Co., Ltd., Shanghai 201114 (CN)
(72) Inventor: ZHAO, Liwen, Shanghai 201114 (CN); WANG, Dihuang, Shanghai 201114 (CN); ZHOU, Gang, Shanghai 201114 (CN)
(74) Representative: Puchberger & Partner Patentanwälte
(86) International application number: PCT/CN2025/074526
(87) International publication number: WO 2025/162210

(57) **Abstract**

The present application relates to the field of pharmaceutical chemistry and specifically to a Bruton's tyrosine kinase (BTK) shown in formula (I) and a mutant degrader thereof, a pharmaceutically acceptable salt thereof, a deuterated compound thereof, a stereoisomer thereof, a tautomer thereof, or a mixture thereof, and a use thereof in the preparation of a drug for treating diseases or disorders mediated by degradation of BTK.

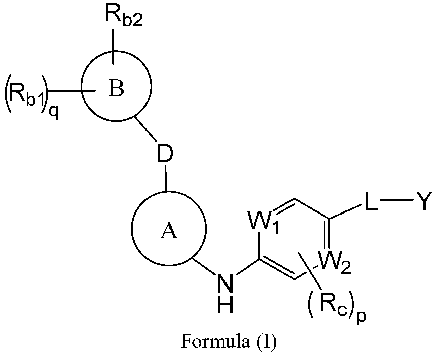

## Description

### TECHNICAL FIELD

The present application relates to the field of pharmaceutical chemistry and specifically to a compound having protein tyrosine kinase BTK-degrading activity, and preparation method therefor and use thereof.

### BACKGROUND

Bruton's tyrosine kinase (BTK), as a member of the non-receptor tyrosine kinase Tec family, plays a pivotal role in antigen-dependent BCR (B cell receptor) and antigen-independent TLR (Toll-like receptor) signaling pathways (Rip et al., 2019). Upon antigen binding to the BCR, cytoplasmic spleen tyrosine kinase (Syk) is activated, recruiting and activating BTK, thereby initiating a kinase cascade that promotes the expression of transcription factors such as NF-κB, PI3K, and ERK (Ahn and Brown, 2021), thereby regulating gene transcription, proliferation, differentiation, and maturation of B cells. Studies have demonstrated that aberrant or persistent activation of BTK is a key factor in the pathogenesis of B-cell lymphomas and autoimmune diseases (e.g., rheumatoid arthritis) (Wang et al., 2022; Zhang et al., 2021). Therefore, targeted inhibition of BTK has become one of the primary therapeutic strategies for B-cell lymphomas, including chronic lymphocytic leukemia (CLL) and mantle cell lymphoma (MCL).

Currently, several covalent inhibitors based on the BTK C481 site have been approved by the FDA for the treatment of B-cell lymphoma, including first-generation covalent inhibitors such as ibrutinib, second-generation covalent inhibitors such as acalabrutinib and zanubrutinib, and third-generation non-covalent inhibitors such as pirtobrutinib. Although BTK inhibitors have demonstrated significant efficacy in clinical practice (e.g., pirtobrutinib achieves an objective response rate (ORR) of 51% in MCL patients, with complete response (CR) and partial response (PR) rates of 25% and 26%, respectively), drug-induced mutations in the BTK kinase domain (such as C481S, L528W, T474I, V416L) can lead to resistance to BTK inhibitors in patients during long-term treatment (Nakhoda et al., 2023). Among them, C481S is the main mutation type for covalent inhibitors, accounting for approximately 70% of clinical mutations (Nakhoda et al., 2023), while for non-covalent inhibitors, L528W and T474I mutations account for approximately 50% and 20% of mutation types, respectively (Wang et al., 2022). In a clinical trial of zanubrutinib for the treatment of CLL, researchers found a significant enrichment of the BTK-L528W mutation in CLL patients treated with zanubrutinib compared with those treated with ibrutinib (Blombery et al., 2022). With the widespread use of second-generation and third-generation BTK inhibitors, addressing the subsequent enrichment of more diverse resistance mutations has become an urgent clinical need in the future treatment of B-cell lymphomas.

Proteolysis targeting chimeras (protacs) are a recently emerging biotechnology that utilizes the ubiquitin-proteasome system to induce target protein degradation. Unlike traditional small molecule inhibitors that only inhibit target proteins by occupying their sites, protacs can be recycled to recruit and induce target protein degradation, enabling efficient degradation of disease-related targets with low-dose protacs (Moreau et al., 2020).

The present application aims to develop a novel small-molecule degrader that can efficiently and specifically induce the degradation of both wild-type and mutant BTK proteins, exhibiting excellent metabolic stability. It achieves potent antitumor effects against B-cell lymphoma while minimizing safety risks associated with off-target toxicities.

### SUMMARY OF THE INVENTION

One of the purposes of the present application is to provide a compound of formula (I), a pharmaceutically acceptable salt thereof, a deuterated compound thereof, a stereoisomer thereof, a tautomer thereof, or a mixture thereof, 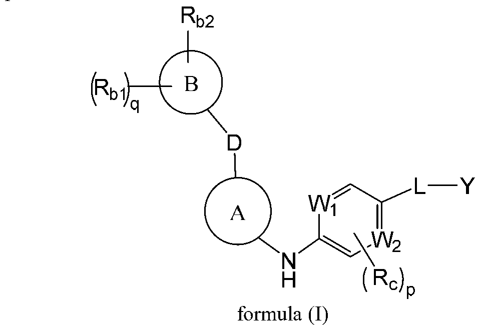 wherein
ring A is selected from the group consisting of 5-12 membered heteroaryl and 5-12 membered heterocyclyl, each optionally substituted with 1, 2, or 3 Rₐ, preferably, ring A is selected from the group consisting of 5-6 membered heteroaryl and 5-6 membered heterocyclyl, each optionally substituted with 1 or 2 Rₐ;
D is selected from the group consisting of a bond, -CR₁R₁^{'}-, -S-, -O-, and -NR₂-(CH₂)ₘ-;
ring B is selected from the group consisting of 6-12 membered aryl, 5-12 membered heteroaryl, 5-12 membered heterocyclyl, and C₃-C₁₂ cycloalkyl;
W₁ and W₂ are each independently selected from the group consisting of C and N;
Rₐ is selected from the group consisting of H, C₁-C₆ alkyl, and -C(=O)-NH₂;
R_{b1} is selected from the group consisting of C₁-C₆ haloalkyl, C₁-C₆ alkyl, and halogen;
R_{b2} is selected from the group consisting of 4-6 membered heterocyclyl, -C(=O)-R₃, -NR₄R₅, -S(=O)₂R₃ and -NR₆-C(=O)-R₇; wherein the 4-6 membered heterocyclyl is optionally substituted with 1 or 2 R₈;
R₁, R₁^{'}, R_{c} and R₂ are each independently selected from the group consisting of H, halogen, C₁-C₆ haloalkyl, C₁-C₆ haloalkoxy, C₁-C₆ alkoxy, and C₁-C₆ alkyl;
R₃ is each independently selected from the group consisting of C₃-C₁₀ cycloalkyl, 5-12 membered heterocyclyl, C₁-C₆ alkyl, C₁-C₆ haloalkyl, -S(=O)₂CH₃, and NR₄R₅; wherein the C₃-C₁₀ cycloalkyl, 5-12 membered heterocyclyl, C₁-C₆ alkyl, and C₁-C₆ haloalkyl are each optionally substituted with 1-3 substituents independently selected from the group consisting of halogen, cyano, -SF₅, C₁-C₆ alkyl, C₁-C₆ haloalkoxy, and C₁-C₆ haloalkyl;
R₄, R₅, and R₆ are each independently selected from the group consisting of H, C₁-C₆ alkyl, and -S(=O)₂CH₃;
R₇ is selected from the group consisting of 5-6 membered heteroaryl and phenyl, each optionally substituted with 1-3 R₉;
R₈ is each independently selected from the group consisting of H, C₁-C₆ alkyl, and -S(=O)₂CH₃;
R₉ is selected from the group consisting of halogen, C₁-C₆ alkyl, and C₁-C₆ hydroxyalkyl;
L is -X₁-X₂-X₃-X₄-X₅-X₆-;
X₁ is selected from the group consisting of a bond, -C(=O)-, C₁-C₆ alkylene, C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl; the C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl are each optionally substituted with 1-3 substituents independently selected from the group consisting of C₁-C₆ alkyl, C₁-C₆ haloalkyl, cyano, -C(O)NH₂, hydroxyl, and halogen;
X₂ is selected from the group consisting of a bond, -C(=O)-, and C₁-C₆ alkylene;
X₃, X₄, X₅, and X₆ are each independently selected from the group consisting of a bond, 3-12 membered heterocyclyl, C₁-C₆ alkylene, C₃-C₁₀ cycloalkyl, -O-, -NR₂-, and -C(=O)-; wherein not all of X₁, X₂, X₃, X₄, X₅, and X₆ are bonds;
Y is selected from the group consisting of 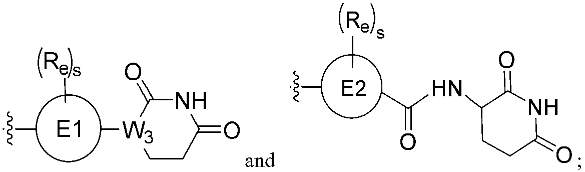 wherein W₃ is selected from the group consisting of N and C; 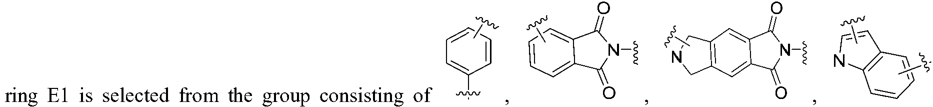 and 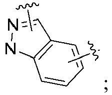 Rₑ is selected from the group consisting of halogen, methyl, methoxy, and trifluoromethyl;
ring E2 is selected from the group consisting of 5-10 membered heteroaryl; preferably 5-6 membered heteroaryl;
m, p, and q are selected from 0, 1, 2, or 3;
s is selected from 0, 1, or 2.

One of the purposes of the present application is to provide a compound of formula (I), a pharmaceutically acceptable salt thereof, a deuterated compound thereof, a stereoisomer thereof, a tautomer thereof, or a mixture thereof, 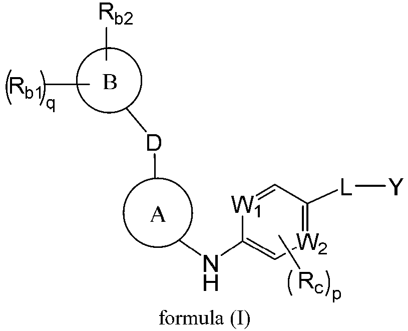 wherein
ring A is selected from the group consisting of 5-12 membered heteroaryl and 5-12 membered heterocyclyl, each optionally substituted with **1,** 2, or 3 Rₐ; preferably, ring A is selected from the group consisting of 5-6 membered heteroaryl and 5-6 membered heterocyclyl, each optionally substituted with 1 or 2 Rₐ;
D is selected from the group consisting of a bond, -CR₁R₁^{'}-, -S-, -O- and -NR₂-(CH₂)ₘ-;
ring B is selected from the group consisting of 6-12 membered aryl, 5-12 membered heteroaryl, 5-12 membered heterocyclyl, and C₃-C₁₂ cycloalkyl;
W₁ and W₂ are each independently selected from the group consisting of C and N;
Rₐ is selected from the group consisting of H, C₁-C₆ alkyl, and -C(=O)-NH₂;
R_{b1} is selected from the group consisting of C₁-C₆ haloalkyl, C₁-C₆ alkyl, and halogen;
R_{b2} is selected from the group consisting of 4-6 membered heterocyclyl, -C(=O)-R₃, -NR₄R₅, -S(=O)₂R₃ and -NR₆-C(=O)-R₇; wherein the 4-6 membered heterocyclyl is optionally substituted with 1 or 2 R₈;
R₁, R₁^{'}, R_{c} and R₂ are each independently selected from the group consisting of H, halogen, C₁-C₆ haloalkyl, C₁-C₆ haloalkoxy, C₁-C₆ alkoxy, and C₁-C₆ alkyl;
R₃ is each independently selected from the group consisting of C₃-C₁₀ cycloalkyl, 5-12 membered heterocyclyl, C₁-C₆ alkyl, C₁-C₆ alkoxy, C₁-C₆ haloalkyl, -S(=O)₂CH₃, and NR₄R₅; wherein the C₃-C₁₀ cycloalkyl, 5-12 membered heterocyclyl, C₁-C₆ alkyl, and C₁-C₆ haloalkyl are each optionally substituted with 1-3 substituents independently selected from the group consisting of halogen, cyano, -SF₅, C₁-C₆ alkyl, C₁-C₆ haloalkoxy, and C₁-C₆ haloalkyl;
R₄, R₅, and R₆ are each independently selected from the group consisting of H, C₁-C₆ alkyl, and -S(=O)₂CH₃;
R₇ is selected from the group consisting of 5-6 membered heteroaryl and phenyl, each optionally substituted with 1-3 R₉;
R₈ is each independently selected from the group consisting of H, C₁-C₆ alkyl, and -S(=O)₂CH₃;
R₉ is selected from the group consisting of halogen, C₁-C₆ alkyl, and C₁-C₆ hydroxyalkyl;
L is -X₁-X₂-X₃-X₄-X₅-X₆-;
X₁ is selected from the group consisting of a bond, -C(=O)-, C₁-C₆ alkylene, C₅-C₈ monocycloalkyl, C₇-C₁₂ spirocycloalkyl; 7-12 membered spiroheterocyclyl; 5-12 membered bridged heterocyclyl; and 3-8 membered monocyclic heterocyclyl; the C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl are each optionally substituted with 1-3 substituents independently selected from the group consisting of C₁-C₆ alkyl, C₁-C₆ haloalkyl, cyano, -C(O)NH₂, hydroxyl, and halogen;
X₂ is selected from the group consisting of a bond, -C(=O)-, and C₁-C₆ alkylene;
X₃, X₄, X₅, and X₆ are each independently selected from the group consisting of a bond, 3-12 membered heterocyclyl, C₁-C₆ alkylene, C₃-C₁₀ cycloalkyl, -O-, -NR₂-, and -C(=O)-; wherein not all of X₁, X₂, X₃, X₄, X₅, and X₆ are bonds;
Y is selected from the group consisting of 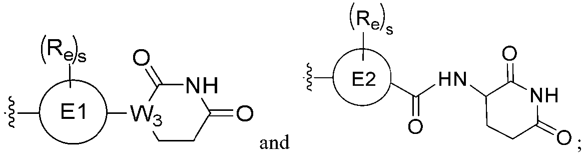 where W₃ is selected from the group consisting of N and C;
ring E1 is selected from the group consisting of 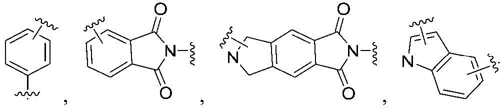 and 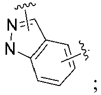 Rₑ is selected from the group consisting of halogen, methyl, methoxy, and trifluoromethyl;
ring E2 is selected from 5-10 membered heteroaryl; preferably 5-6 membered heteroaryl;
m, p, and q are each independently selected from 0, 1, 2, or 3;
s is selected from 0, 1, or 2.

In some preferred embodiments, ring A is selected from the group consisting of 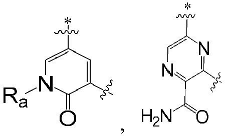 and 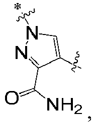 wherein * indicates the position of attachment to D, provided that: when ring A is selected from the group consisting of

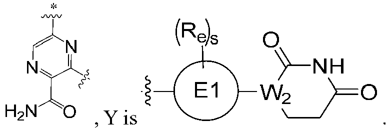

In some preferred embodiments, Rₐ is selected from the group consisting of H, C₁-C₃ alkyl and -C(=O)-NH₂, preferably, Rₐ is selected from the group consisting of C₁-C₃ alkyl and -C(=O)-NH₂, more preferably, Rₐ is selected from the group consisting of -CH₃ and -C(=O)-NH₂.

In some embodiments, R_{b1} is selected from the group consisting of C₁-C₆ haloalkyl, C₁-C₆ alkyl, and halogen, preferably, R_{b1} is selected from the group consisting of C₁-C₃ haloalkyl, C₁-C₃ alkyl, and halogen, more preferably, R_{b1} is selected from the group consisting of C₁-C₃ alkyl and halogen, further preferably, R_{b1} is selected from the group consisting of -CH₃ and F. R_{b2} is selected from the group consisting of 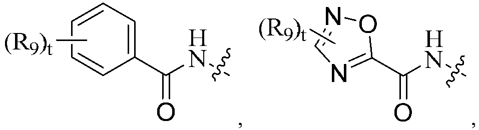 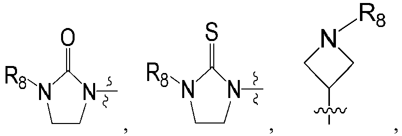 -C(=O)-R₃, -NR₄R₅ and -S(=O)₂R₃; or R_{b2} is selected from the group consisting of 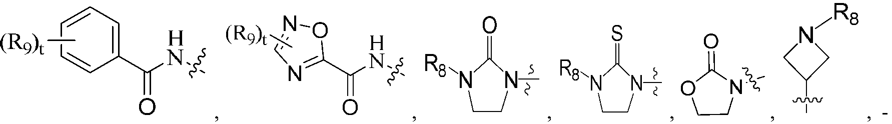 C(=O)-R₃, -NR₄R₅, and -S(=O)₂R₃; R₉ is selected from the group consisting of halogen, C₁-C₆ alkyl, and C₁-C₆ hydroxyalkyl, preferably, R₉ is selected from the group consisting of C₁-C₃ alkyl and C₁-C₃ hydroxyalkyl; R₈ is each independently selected from the group consisting of H, C₁-C₆ alkyl, C₁-C₆ haloalkyl, and -S(=O)₂CH₃, preferably, R₈ is each independently selected from the group consisting of H and C₁-C₃ alkyl; t is selected from 0, 1, or 2; preferably, R_{b2} is selected from the group consisting of 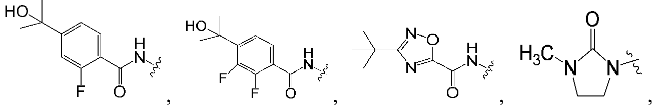 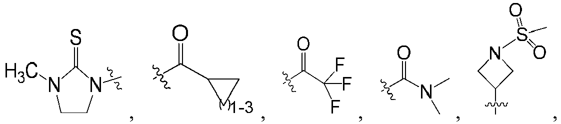 -NH₂, -NH-S(=O)₂CH₃ and -S(=O)₂CH₃; or R_{b2} is selected from selected from the group consisting of 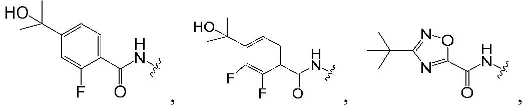 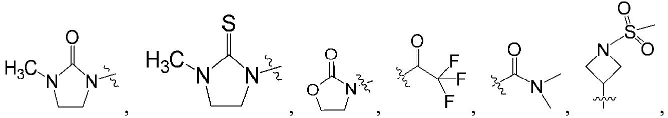 -C(=O)-R₃ and -S(=O)₂CH₃; R₃ is each independently selected from the group consisting of C₃-C₅ cycloalkyl, C₁-C₃ alkyl, and C₁-C₃ alkoxy, each optionally substituted with 1-3 substituents independently selected from the group consisting of halogen, cyano, -SF₅, C₁-C₆ alkyl, C₁-C₆ haloalkoxy, and C₁-C₆ haloalkyl.

In some preferred embodiments, R_{b2} is selected from the group consisting of 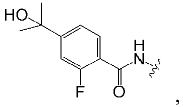 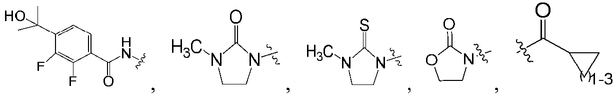 and -S(=O)₂CH₃, or R_{b2} is selected from the group consisting of 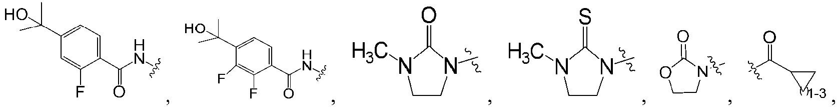 -C(=O)OCH₃ and -S(=O)₂CH₃.

In some preferred embodiments, R₃ is each independently selected from the group consisting of C₃-C₅ cycloalkyl, and C₁-C₃ alkyl; or R₃ is each independently selected from the group consisting of C₃-C₅ cycloalkyl, and C₁-C₃ alkoxy.

In some preferred embodiments, R₁, R₁', R_{c}, R₂ are each independently selected from the group consisting of H, halogen, C₁-C₃ haloalkyl, C₁-C₃ haloalkoxy, C₁-C₃ alkoxy and C₁-C₃ alkyl; preferably, each independently selected from the group consisting of H, C₁-C₃ alkoxy and C₁-C₃ alkyl.

In some preferred embodiments, ring E1 is selected from the group consisting of 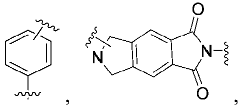 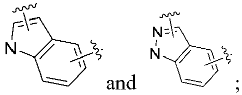 preferably, ring E1 is selected from the group consisting of 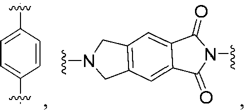 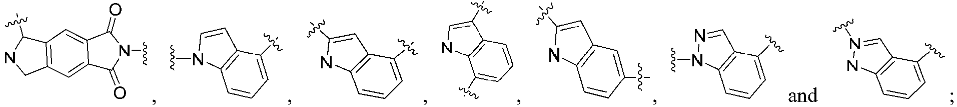 more preferably, ring E1 is selected from the group consisting of 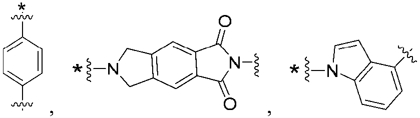 and 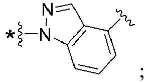 * indicates the position of attachment to L.

In some preferred embodiments, ring E1 is selected from the group consisting of 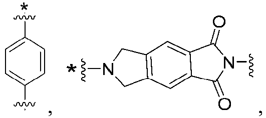 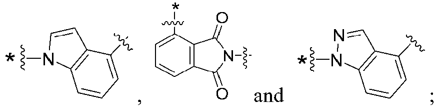 wherein * indicates the position of attachment to L.

In some preferred embodiments, ring E2 is selected from the group consisting of 5-6 membered heteroaryl, preferably pyridine.

In some preferred embodiments, Y is selected from the group consisting of 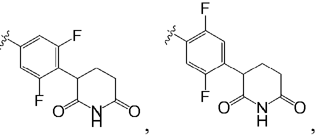 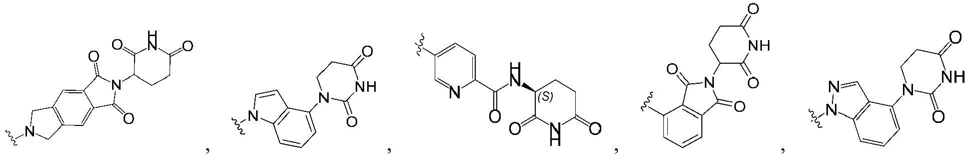 and 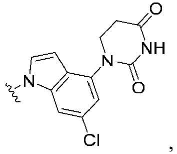 preferably, Y is selected from the group consisting of

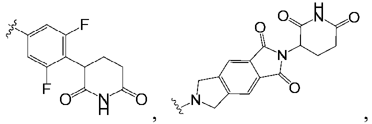

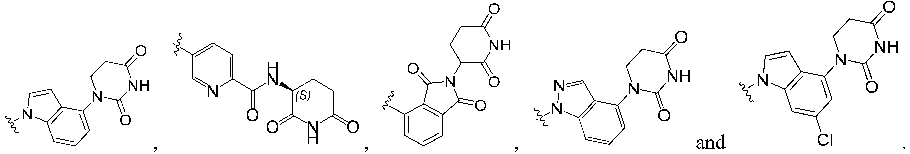

In some preferred embodiments, the compound of formula (I) has the structure shown in formula (II): 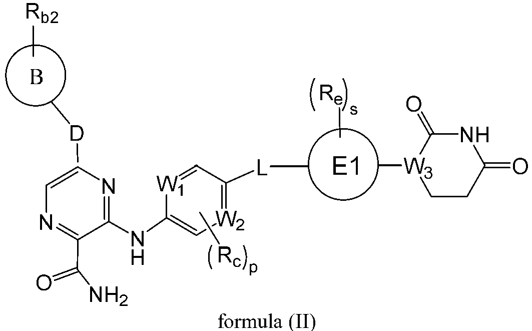 wherein
W₁, W₂, and W₃ are each independently selected from the group consisting of C and N;
D is selected from the group consisting of a bond, -CR₁R₁^{'}-, -S-, -O-, and -NR₂-(CH₂)ₘ-;
ring B is selected from the group consisting of 6-12 membered aryl, 5-12 membered heteroaryl, 5-12 membered heterocyclyl, and C₃-C₆ cycloalkyl;
R_{b2} is selected from the group consisting of 4-6 membered heterocyclyl, -C(=O)-R₃, -NR₄R₅, -S(=O)₂R₃, and -NR₆-C(=O)-R₇; wherein the 4-6 membered heterocyclyl is optionally substituted with 1 or 2 R₈;
R₁, R₁^{'}, Rc, and R₂ are each independently selected from the group consisting of H, halogen, C₁-C₆ haloalkyl, C₁-C₆ haloalkoxy, C₁-C₆ alkoxy, and C₁-C₆ alkyl;
R₃ is each independently selected from the group consisting of C₃-C₁₀ cycloalkyl, 5-12 membered heterocyclyl, C₁-C₆ alkyl, C₁-C₆ haloalkyl, -S(=O)₂CH₃, and NR₄R₅; wherein the C₃-C₁₀ cycloalkyl, 5-12 membered heterocyclyl, C₁-C₆ alkyl, and C₁-C₆ haloalkyl are each optionally substituted with 1-3 substituents independently selected from the group consisting of halogen, cyano, -SF₅, C₁-C₆ alkyl, C₁-C₆ haloalkoxy, and C₁-C₆ haloalkyl;
R₄, R₅, and R₆ are each independently selected from the group consisting of H, C₁-C₆ alkyl, and -S(=O)₂CH₃;
R₇ is selected from the group consisting of 5-6 membered heteroaryl and phenyl, each optionally substituted with 1-3 R₉;
R₈ is each independently selected from the group consisting of H, C₁-C₆ alkyl, and -S(=O)₂CH₃;
R₉ is selected from the group consisting of halogen, C₁-C₆ alkyl, and C₁-C₆ hydroxyalkyl;
L is -X₁-X₂-X₃-X₄-X₅-X₆-;
X₁ is selected from the group consisting of a bond, -C(=O)-, C₁-C₆ alkylene, C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl; the C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl are each optionally substituted with 1-3 substituents independently selected from the group consisting of C₁-C₆ alkyl, C₁-C₆ haloalkyl, cyano, -C(O)NH₂, hydroxyl, and halogen;
X₂ is selected from the group consisting of a bond, -C(=O)-, and C₁-C₆ alkylene;
X₃, X₄, X₅, and X₆ are each independently selected from the group consisting of a bond, 3-12 membered heterocyclyl, C₁-C₆ alkylene, C₃-C₁₀ cycloalkyl, -O-, -NR₂-, and -C(=O)-; wherein not all of X₁, X₂, X₃, X₄, X₅, and X₆ are bonds;
ring E1 is selected from the group consisting of 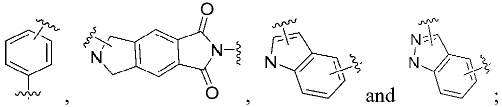
Rₑ is selected from the group consisting of halogen, methyl, methoxy, and trifluoromethyl;
m, s, and p are each independently selected from 0, 1, or 2.

In some preferred embodiments, the compound of formula (I) has the structure shown in formula (II): 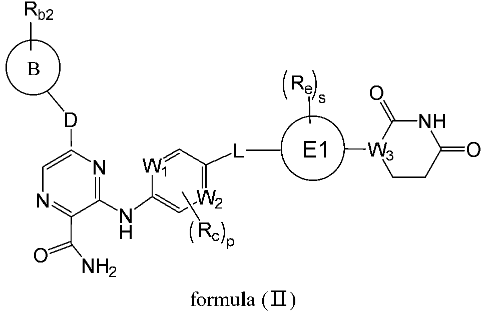 wherein
W₁, W₂, and W₃ are each independently selected from the group consisting of C and N;
D is selected from the group consisting of a bond, -CR₁R₁^{'}-, -S-, -O-, and -NR₂-(CH₂)ₘ-;
ring B is selected from the group consisting of 6-12 membered aryl, 5-12 membered heteroaryl, 5-12 membered heterocyclyl, and C₃-C₆ cycloalkyl;
R_{b2} is selected from the group consisting of 4-6 membered heterocyclyl, -C(=O)-R₃, -NR₄R₅, -S(=O)₂R₃, and -NR₆-C(=O)-R₇; wherein the 4-6 membered heterocyclyl is optionally substituted with 1 or 2 R₈;
R₁, R₁^{'}, Rc, and R₂ are each independently selected from the group consisting of H, halogen, C₁-C₆ haloalkyl, C₁-C₆ haloalkoxy, C₁-C₆ alkoxy, and C₁-C₆ alkyl;
R₃ is each independently selected from the group consisting of C₃-C₁₀ cycloalkyl, 5-12 membered heterocyclyl, C₁-C₆ alkyl, C₁-C₆ alkoxy, C₁-C₆ haloalkyl, -S(=O)₂CH₃, and NR₄R₅; wherein the C₃-C₁₀ cycloalkyl, 5-12 membered heterocyclyl, C₁-C₆ alkyl, and C₁-C₆ haloalkyl are each optionally substituted with 1-3 substituents independently selected from the group consisting of halogen, cyano, -SF₅, C₁-C₆ alkyl, C₁-C₆ haloalkoxy, and C₁-C₆ haloalkyl;
R₄, R₅, and R₆ are each independently selected from the group consisting of H, C₁-C₆ alkyl, and -S(=O)₂CH₃;
R₇ is selected from the group consisting of 5-6 membered heteroaryl and phenyl, each optionally substituted with 1-3 R₉;
R₈ is each independently selected from the group consisting of H, C₁-C₆ alkyl, and -S(=O)₂CH₃;
R₉ is selected from the group consisting of halogen, C₁-C₆ alkyl, and C₁-C₆ hydroxyalkyl;
L is -X₁-X₂-X₃-X₄-X₅-X₆-;
X₁ is selected from the group consisting of a bond, -C(=O)-, C₁-C₆ alkylene, C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl; the C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl are each optionally substituted with 1-3 substituents independently selected from the group consisting of C₁-C₆ alkyl, C₁-C₆ haloalkyl, cyano, -C(O)NH₂, hydroxyl, and halogen;
X₂ is selected from the group consisting of a bond, -C(=O)-, and C₁-C₆ alkylene;
X₃, X₄, X₅, and X₆ are each independently selected from the group consisting of a bond, 3-12 membered heterocyclyl, C₁-C₆ alkylene, C₃-C₁₀ cycloalkyl, -O-, -NR₂-, and -C(=O)-; wherein not all of X₁, X₂, X₃, X4, X₅, and X₆ are bonds;
ring E1 is selected from the group consisting of 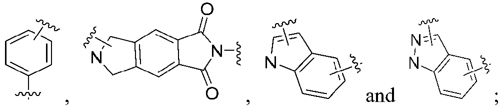
Rₑ is selected from the group consisting of halogen, methyl, methoxy, and trifluoromethyl;
m, s, and p are each independently selected from 0, 1, or 2.

In some preferred embodiments, in the compound of formula (I) and compound of formula (II), ring B is selected from the group consisting of phenyl, 5-6 membered heteroaryl, 5-6 membered heterocyclyl, and C₃-C₆ cycloalkyl; more preferably, ring B is selected from the group consisting of 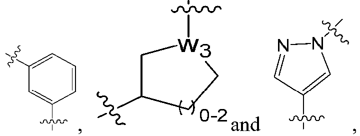 and W₃ is selected from the group consisting of C and N; further preferably, ring B is selected from the group consisting of 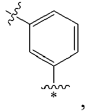 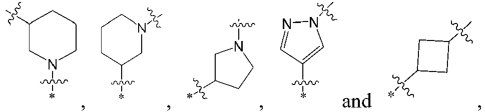 furthermore preferably, ring B is selected from the group consisting of 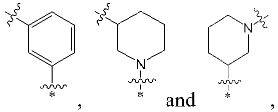 even more preferably, ring B is selected from the group consisting of 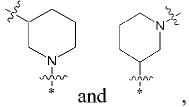 wherein * indicates the position of attachment to D.

In some preferred embodiments, in the compound of formula (I) and compound of formula (II), D is selected from the group consisting of a bond, -NH-, -N(CH₃)-, and -NH-CH₂-; preferably, D is selected from the group consisting of a bond, -NH-, and -N(CH₃)-.

In some preferred embodiments, in the compound of formula (II), D is selected from a bond, ring B is selected from the group consisting of 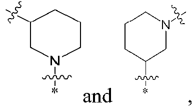 preferably 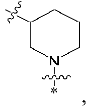 wherein * indicates the position of attachment to D.

In some preferred embodiments, in the compound of formula (II), D is selected from the group consisting of -NR₂-(CH₂)ₘ-; ring B is selected from the group consisting of 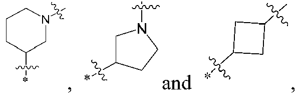 preferably 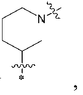 wherein * indicates the position of attachment to D.

In some preferred embodiments, in the compound of formula (II), R_{b2} is selected from the group consisting of 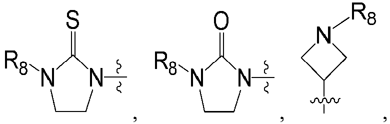 -C(=O)-R₃, -NR₄R₅ and -S(=O)₂R₃, preferably, R_{b2} is selected from the group consisting of 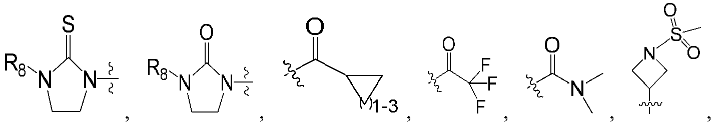 -NH₂, -NH-S(=O)₂CH₃ and -S(=O)₂CH₃.

In some preferred embodiments, in the compound of formula (II), R_{b2} is selected from the group consisting of 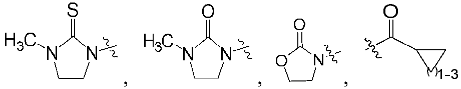 and -S(=O)₂CH₃; or R_{b2} is selected from the group consisting of 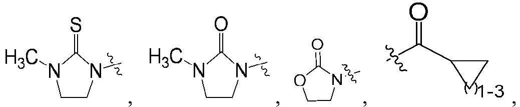 -C(=O)OCH₃ and -S(=O)₂CH₃.

In some preferred embodiments, in the compound of formula (II), D is selected from the group consisting of a bond, -NH-, -N(CH₃)- and -NH-CH₂-, preferably, D is selected from the group consisting of a bond, -NH- and -N(CH₃)-; W₁, W₂ and W₃ are each independently selected from the group consisting of C and N; ring B is selected from the group consisting of 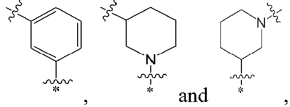 preferably, ring B is selected from the group consisting of 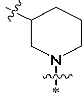 and 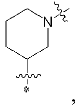 wherein * indicates the position of attachment to D;
R_{b2} is selected from the group consisting of 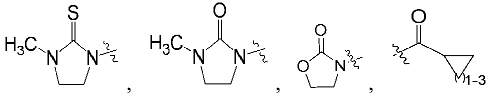 and - S(=O)₂CH₃; or R_{b2} is selected from the group consisting of 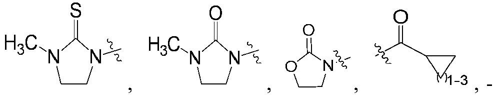 C(=O)OCH₃ and -S(=O)₂CH₃;
ring E1 is selected from the group consisting of 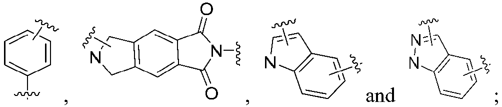
L is selected from the group consisting of 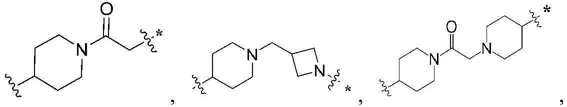 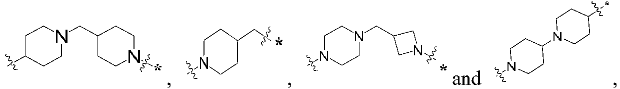 wherein * indicates the position of attachment to Y.

In some preferred embodiments, in the compound of formula (II), D is selected from the group consisting of a bond, -NH-, -N(CH₃)-, and -NH-CH₂-, preferably, D is selected from the group consisting of a bond, -NH-, and -N(CH₃)-;
W₁, W₂ and W₃ are each independently selected from the group consisting of C and N; ring B is selected from the group consisting of 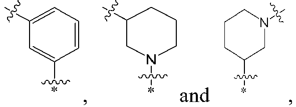 preferably, ring B is selected from the group consisting of 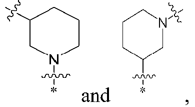 wherein * indicates the position of attachment to D;
R_{b2} is selected from the group consisting of 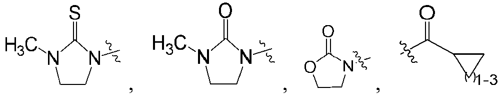 and - S(=O)₂CH₃; or R_{b2} is selected from the group consisting of 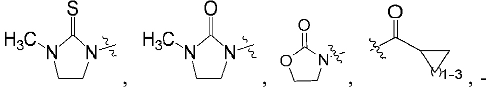 C(=O)OCH₃ and -S(=O)₂CH₃;
ring E1 is selected from the group consisting of 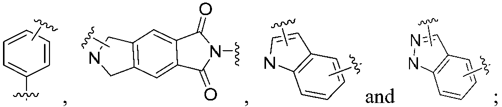
L is selected from the group consisting of 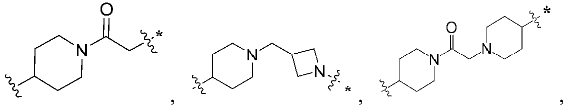 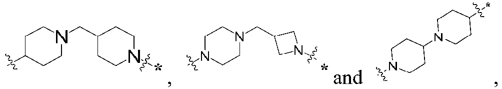 wherein * indicates the position of attachment to Y.

In some preferred embodiments, in the compound of formula (II), D is selected from the group consisting of a bond, -NH-, and -N(CH₃)-;
W₁ and W₂ are each independently selected from C or N; W3 is independently selected from C or N; ring B is selected from the group consisting of 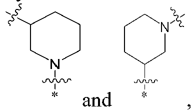 wherein * indicates the position of attachment to D;
R_{b2} is selected from the group consisting of 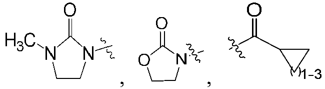 and -C(=O)OCH₃;
ring E1 is selected from the group consisting of 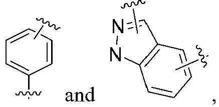 preferably, ring E1 is selected from the group consisting of 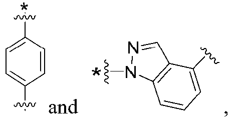 wherein * indicates the position of attachment to L;
L is selected from the group consisting of 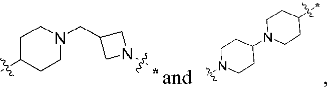 wherein * indicates the position of attachment to Y.

In some preferred embodiments, the present application provides a compound of formula (I-1)-(I-4), a pharmaceutically acceptable salt thereof, a deuterated compound thereof, a stereoisomer thereof, a tautomer thereof, or a mixture thereof, 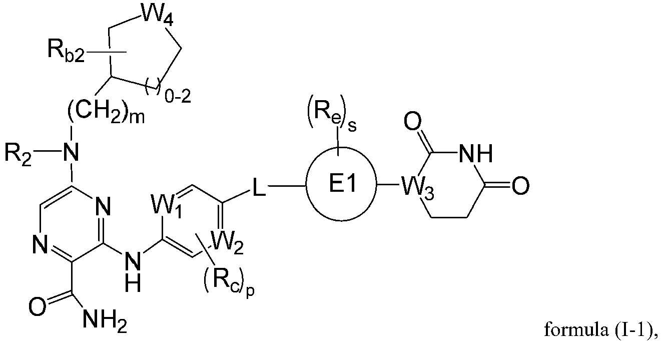 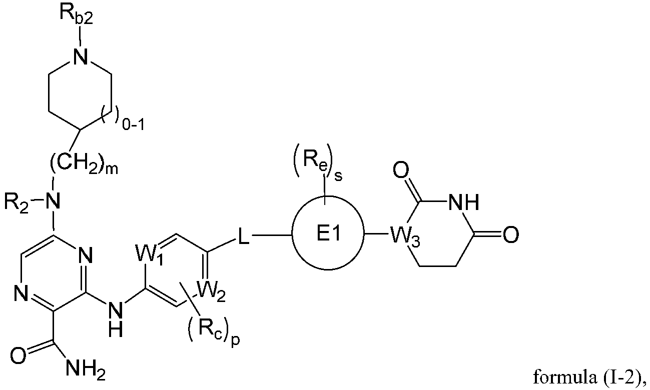 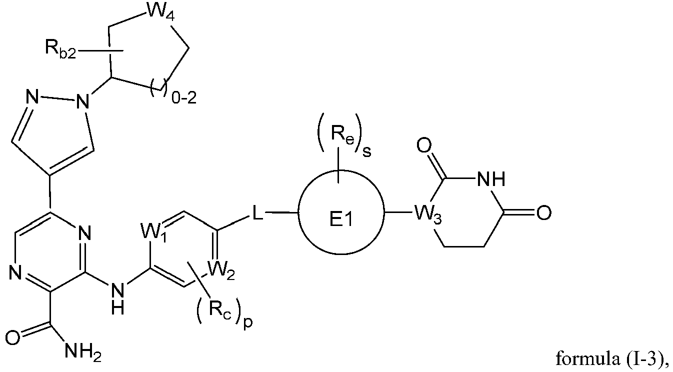 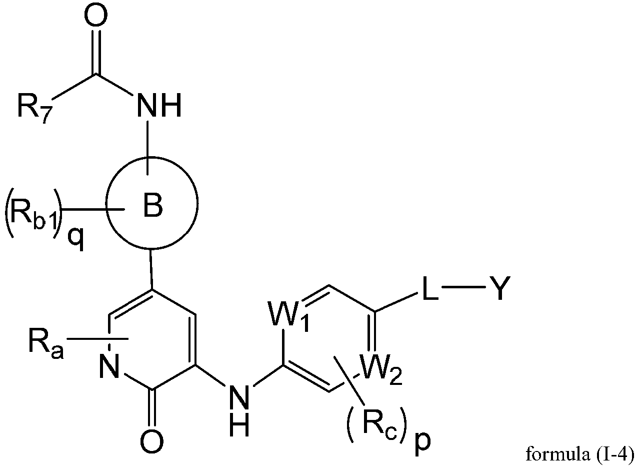 wherein
W₄ is selected from the group consisting of C and N;
W₁, W₂, W₃, R_{c}, R₂, R_{b2}, Rₑ, Rₐ, R₇, ring B, L, Y, ring E1, m, p, q, and s are defined as any one of the above.

In some embodiments, in the compounds of formula (I), (II), and formulas (I-1)-(I-4), L is -X₁-X₂-X₃-X₄-X₅-X₆-*, wherein * indicates the position of attachment to Y;
X₁ is selected from the group consisting of a bond, -C(=O)-, C₁-C₆ alkylene, C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl; wherein the C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl are each optionally substituted with 1-3 substituents independently selected from the group consisting of C₁-C₆ alkyl, C₁-C₆ haloalkyl, cyano, -C(O)NH₂, hydroxyl, and halogen; preferably, X₁ is selected from the group consisting of a bond, -C(=O)-, and 3-6 membered monocyclic heterocyclyl;
X₂ is selected from the group consisting of a bond, -C(=O) and C₁-C₃ alkylene; preferably, X₂ is selected from the group consisting of a bond, -C(=O) and C₁-C₂ alkylene;
X₃, X₄, X₅, and X₆ are each independently selected from the group consisting of a bond, 4-6 membered heterocyclyl, C₁-C₃ alkylene, C₃-C₆ cycloalkyl, -O-, -NH-, and -C(=O)-, and not all of X₁, X₂, X₃, X₄, X₅, and X₆ are bonds.

In some preferred embodiments, in the compound of formula (I), (II), and formulas (I-1)-(I-4), L is selected from the group consisting of

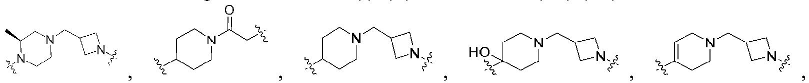

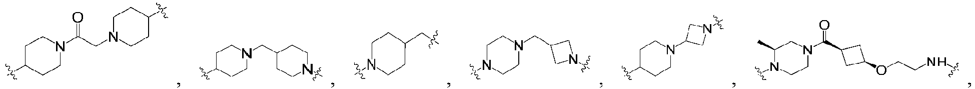

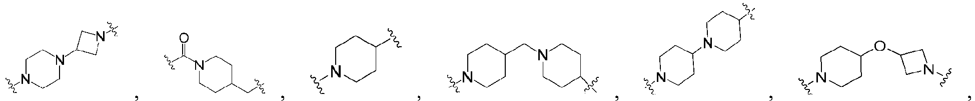

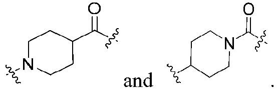

In some more preferred embodiments, in the compound of formula (I), (II), and formulas (I-1)-(I-4), L is selected from the group consisting of 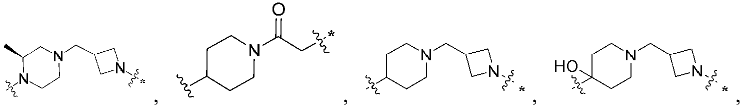 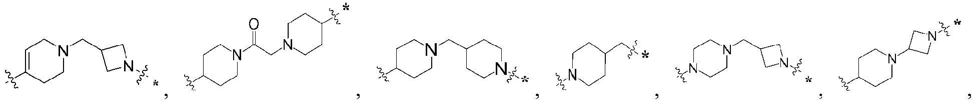 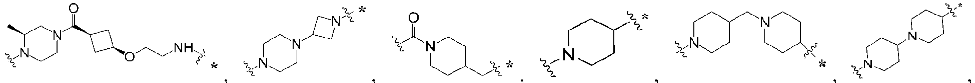 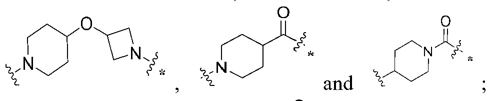 preferably, L is selected from the group consisting of 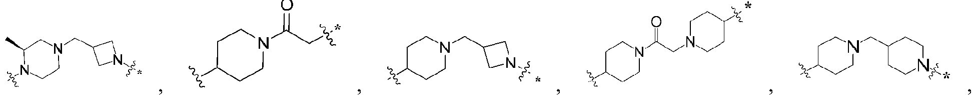 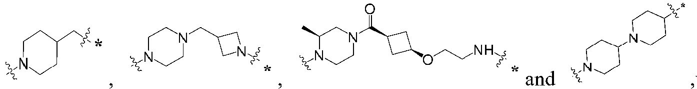 wherein * indicates the position of attachment to Y or E1.

In some preferred embodiments, in the compound of formula (I), ring A is selected from the group consisting of 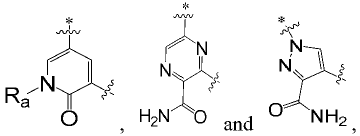 wherein * indicates the position of attachment to D, provided that when ring A is selected from the group consisting of 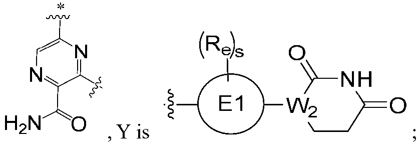
Rₐ is selected from the group consisting of C₁-C₃ alkyl and -C(=O)-NH₂, preferably, Rₐ is selected from the group consisting of -CH₃ and -C(=O)-NH₂;
D is selected from the group consisting of a bond, -NH-, -N(CH₃)-, and -NH-CH₂-; preferably, D is selected from the group consisting of a bond, -NH-, and -N(CH₃)-;
W₁ and W₂ are each independently selected from the group consisting of C and N;
ring B is selected from the group consisting of 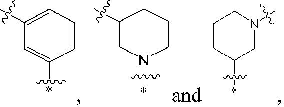 wherein * indicates the position of attachment to D;
R_{b1} is selected from the group consisting of C₁-C₃ alkyl and halogen, preferably, R_{b1} is selected from the group consisting of -CH₃ and F;
R_{b2} is selected from the group consisting of 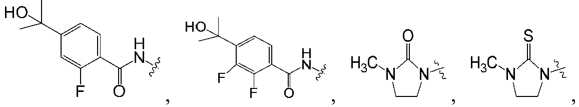 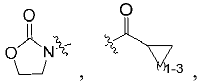 and -S(=O)₂CH₃, or R_{b2} is selected from the group consisting of 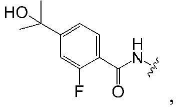 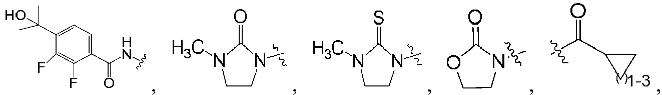 -C(=O)OCH₃ and -S(=O)₂CH₃
q is selected from the group consisting of 0, 1, or 2;
Y is selected from the group consisting of 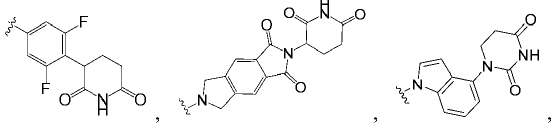 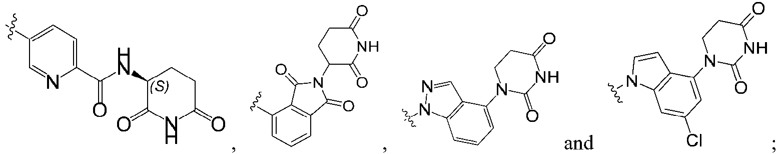
L is selected from the group consisting 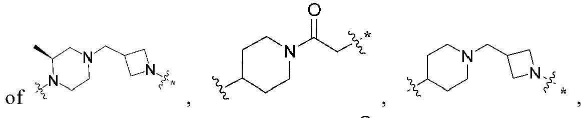 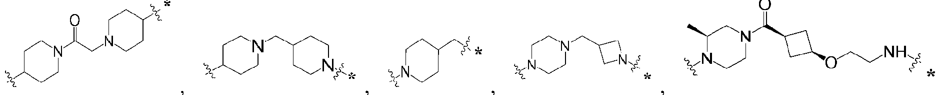 and 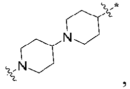 wherein * indicates the position of attachment to Y.

In some preferred embodiments, for any of the above compound, pharmaceutically acceptable salt thereof, deuterated compound thereof, stereoisomer thereof, tautomer thereof, or mixture thereof, the compound is selected from but not limited to:

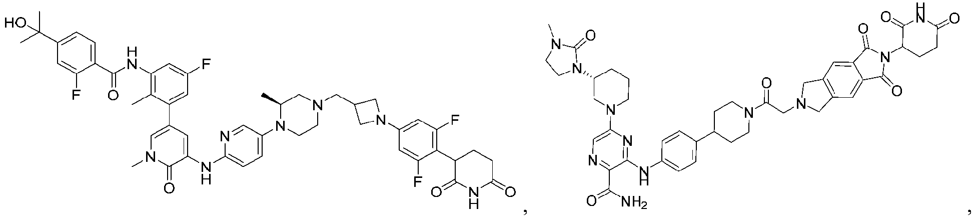

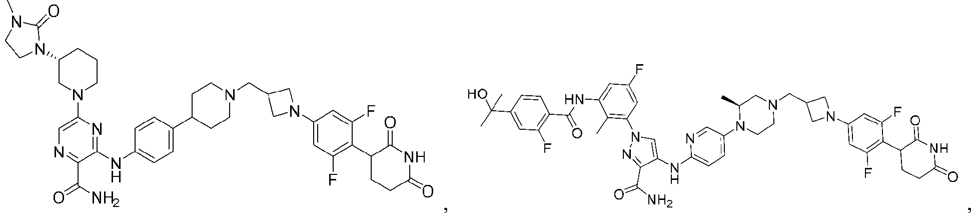

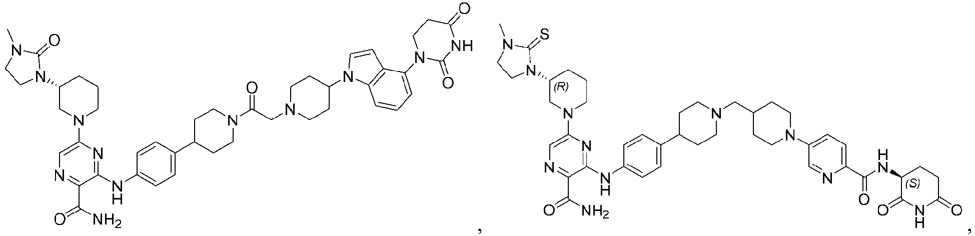

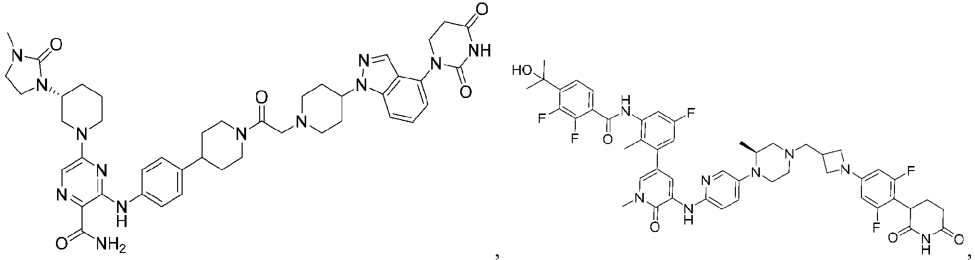

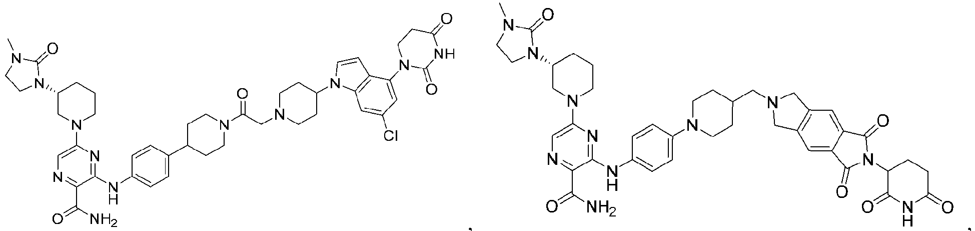

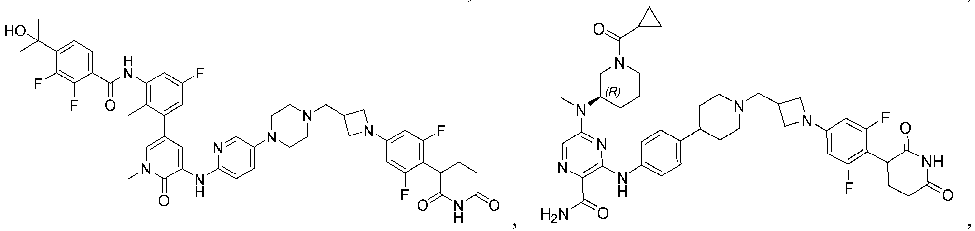

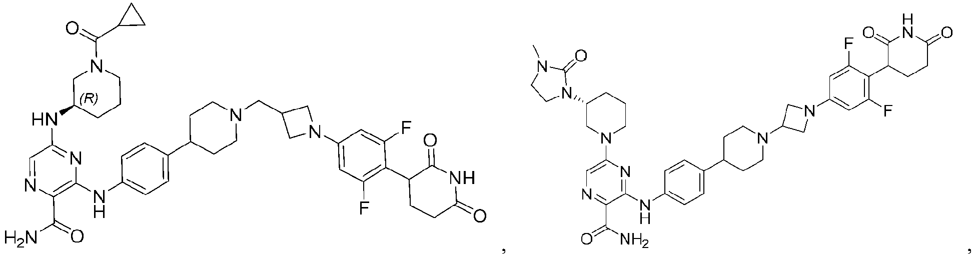

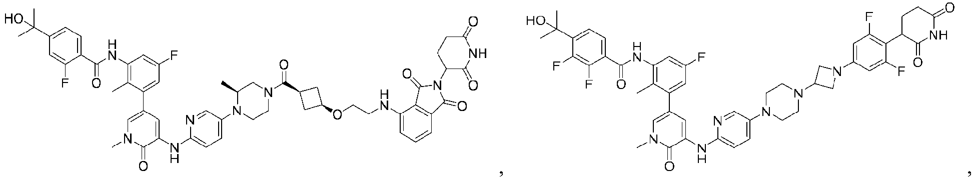

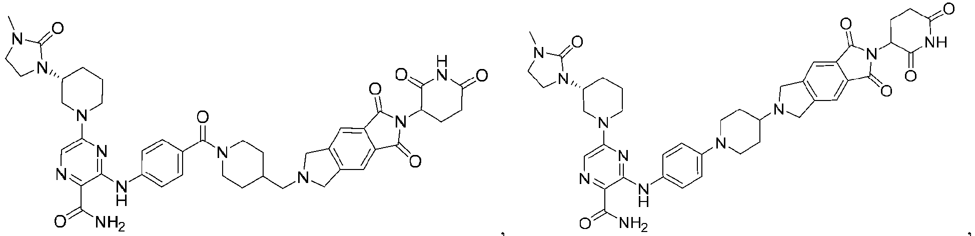

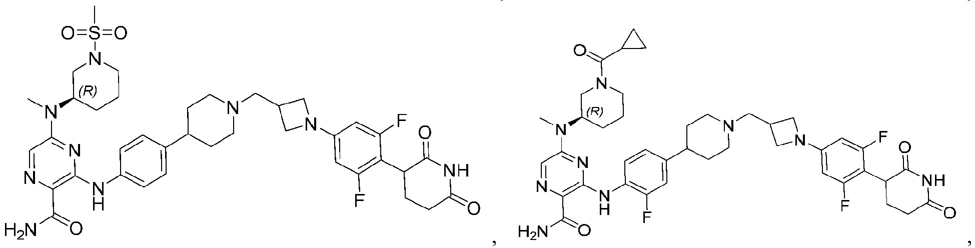

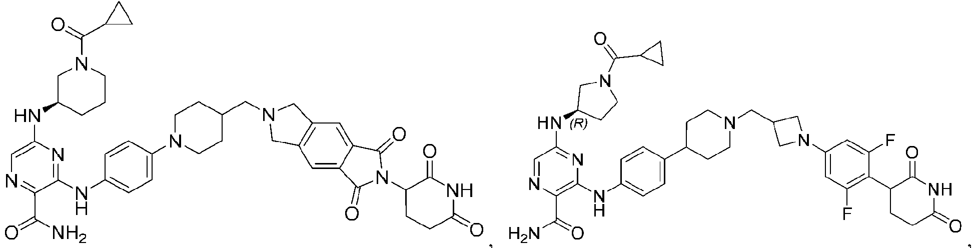

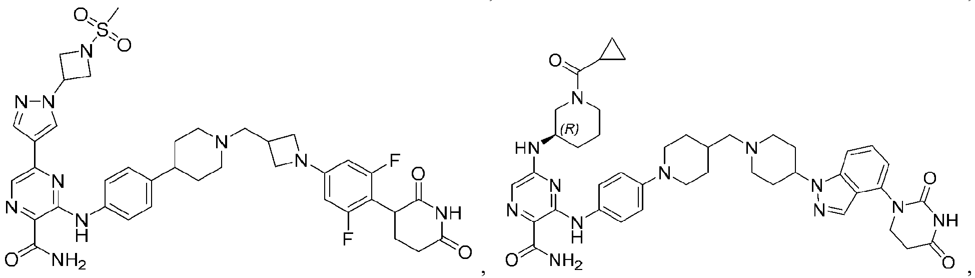

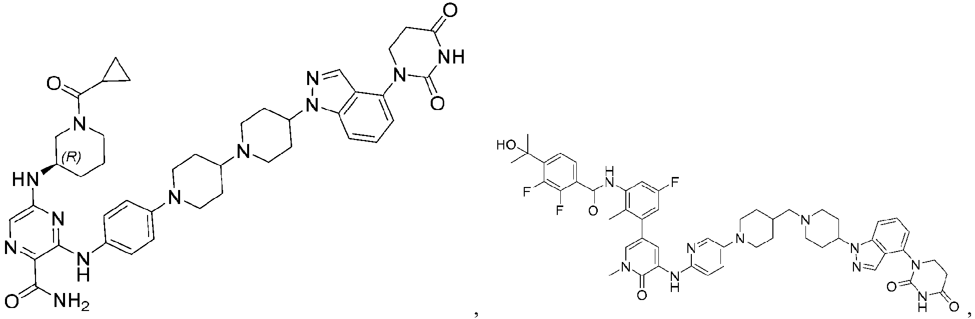

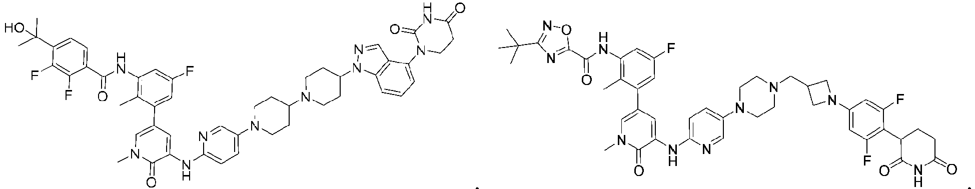

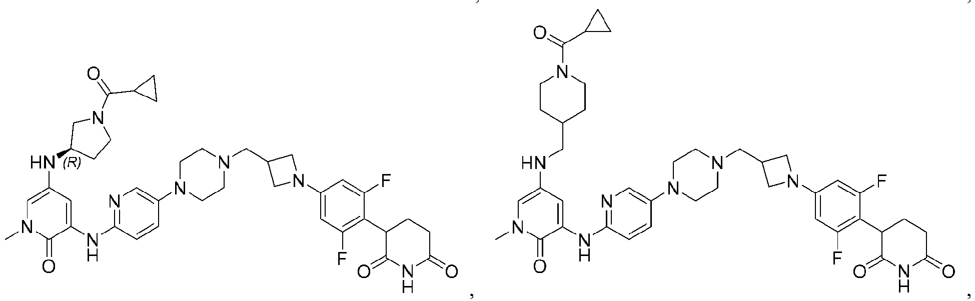

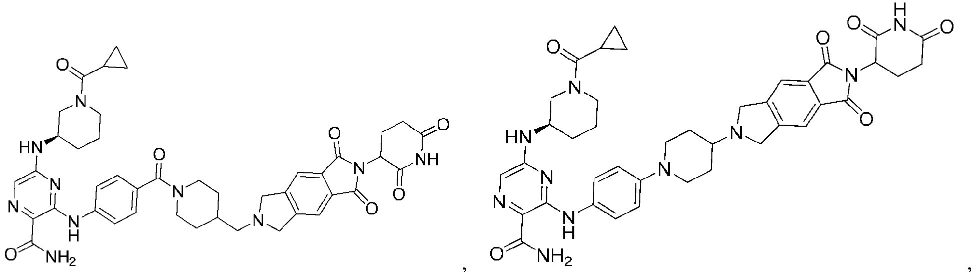

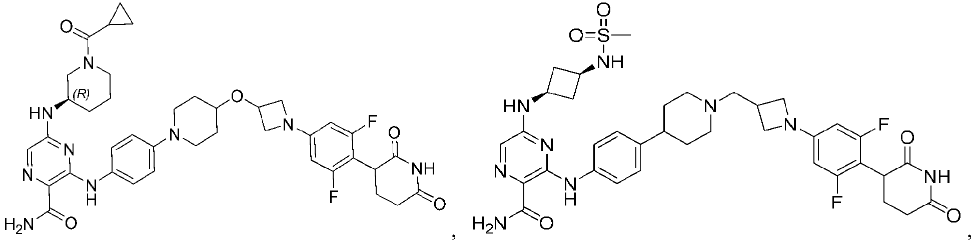

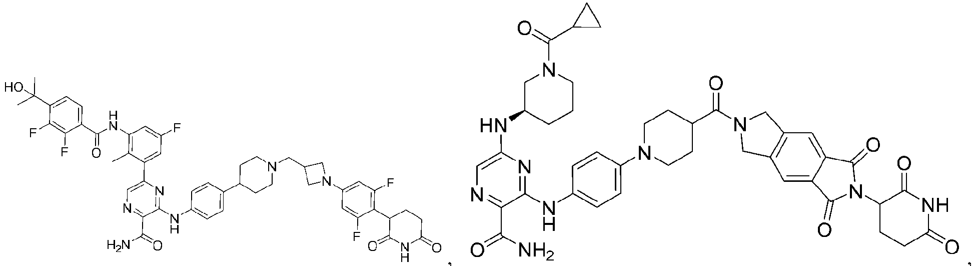

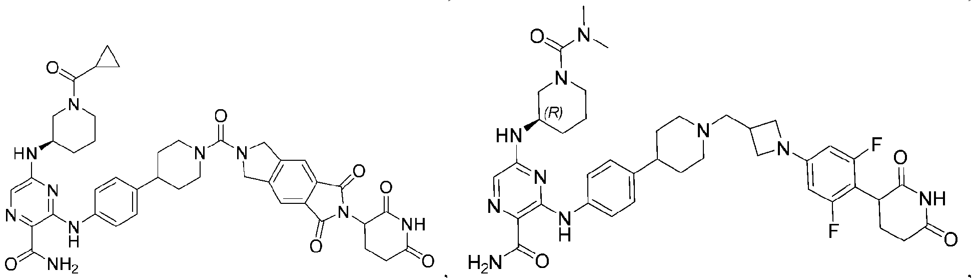

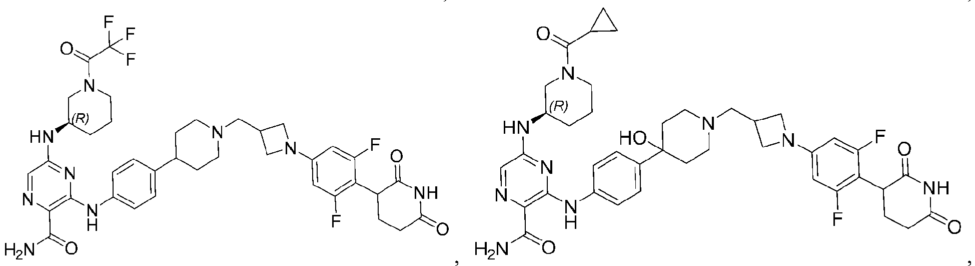

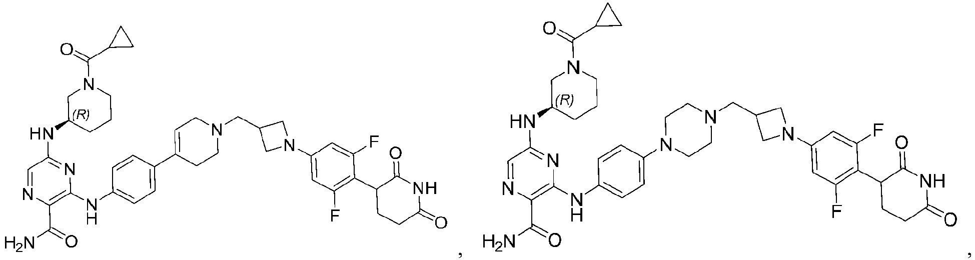

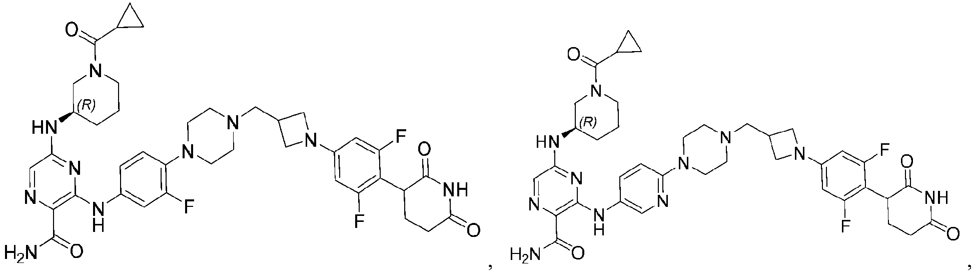

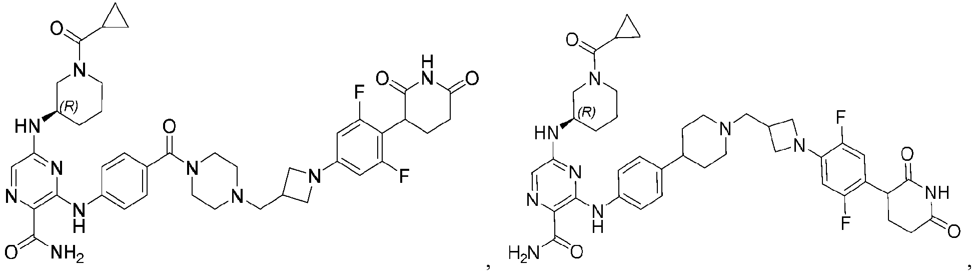

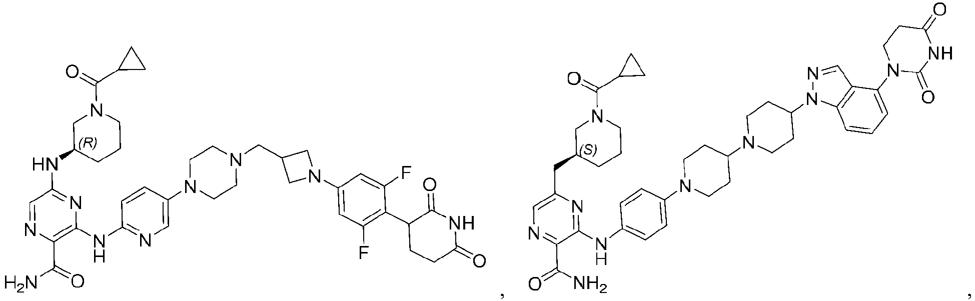

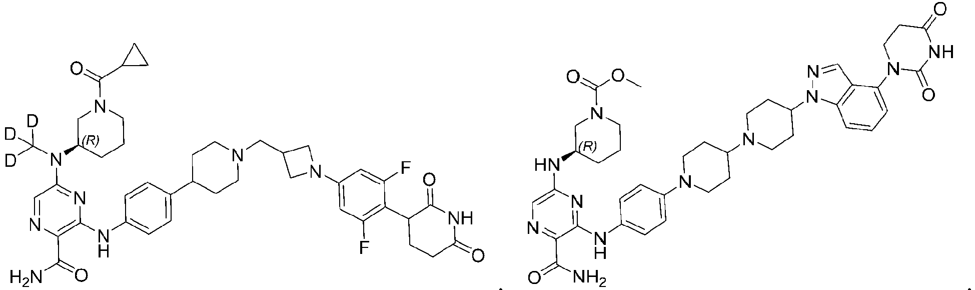

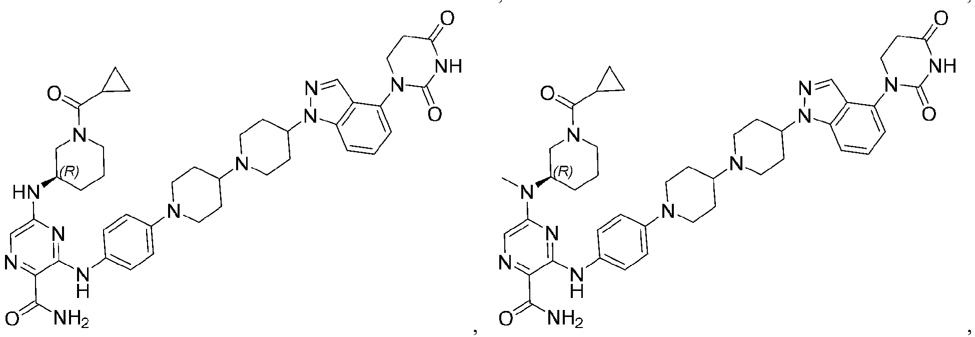

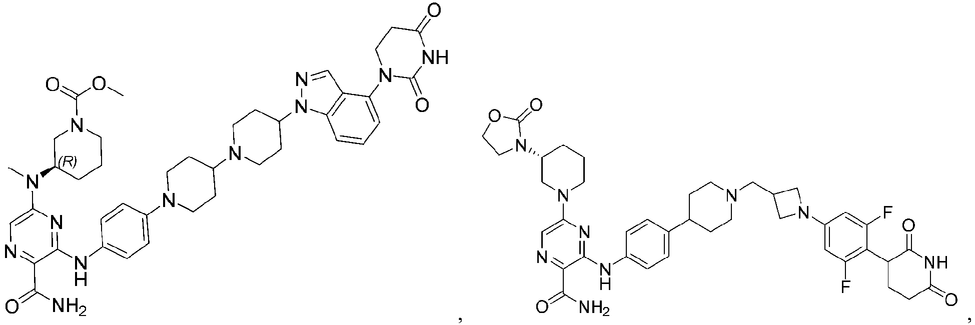

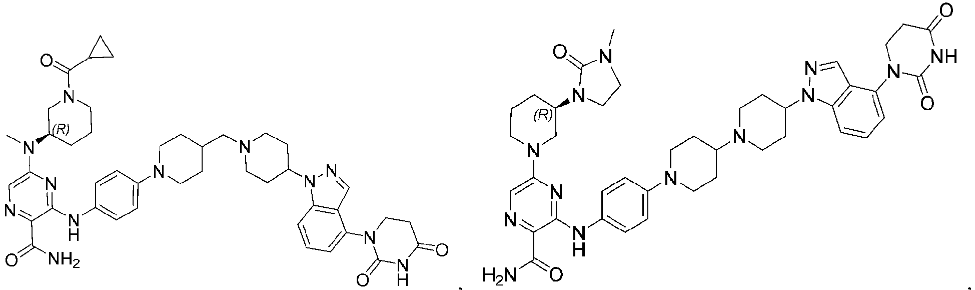

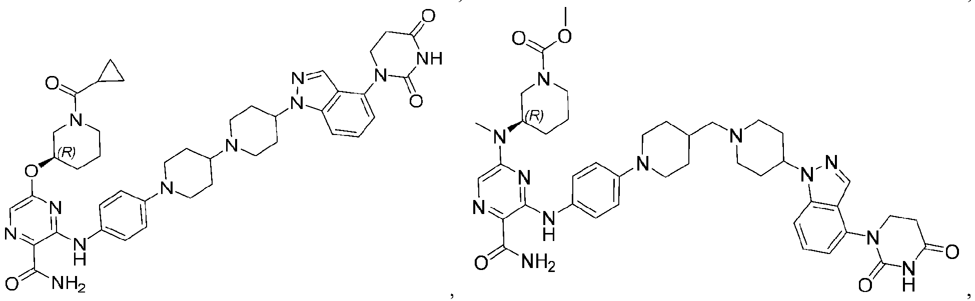

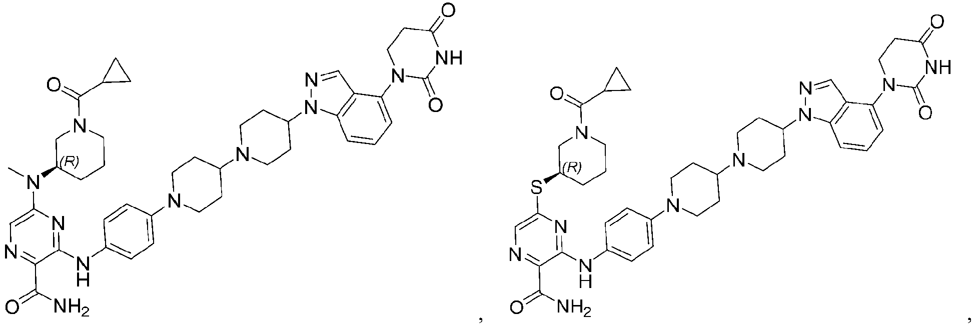

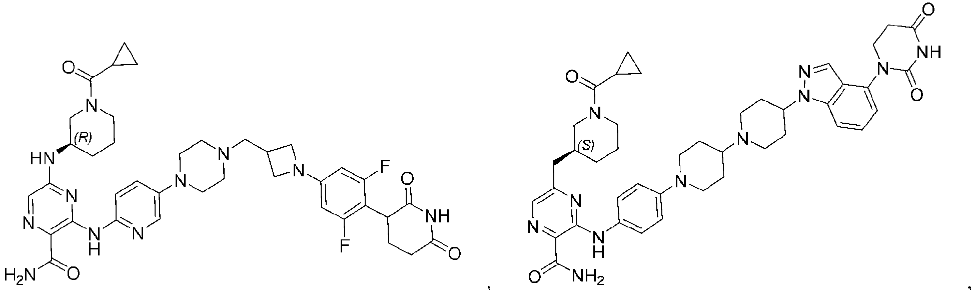

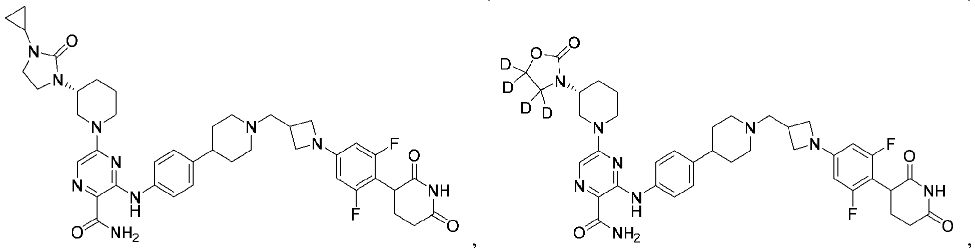

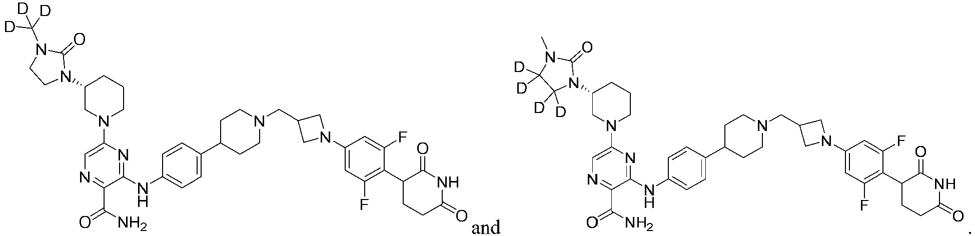

Another object of the present application is to provide use of any one of the above compounds, pharmaceutically acceptable salt thereof, deuterated compound thereof, stereoisomer thereof, tautomer thereof, or mixture thereof in the preparation of a drug for preventing and/ or treating diseases or disorders mediated by degradation of Bruton's tyrosine kinase (BTK).

Alternatively, another object of the present application is to provide any one of the above compounds, pharmaceutically acceptable salt thereof, deuterated compound thereof, stereoisomer thereof, tautomer thereof, or mixture thereof for preventing and/or treating a disease or disorder mediated by degradation of BTK. Another object is to provide a method for preventing and/or treating a disease or disorder mediated by degradation of BTK, comprising administering to a subject in need thereof a preventive and/or therapeutic effective amount of a compound, a pharmaceutically acceptable salt thereof, a deuterated compound thereof, a stereoisomer thereof, a tautomer thereof, or a mixture thereof described above. In the present application, the "preventive and/or therapeutic effective amount" refers to an amount sufficient to prevent, improve, inhibit, postpone, or slow the progression of a disease or condition mediated by degradation of Bruton's tyrosine kinase (BTK), which can be determined by a clinician based on the severity of the disease, the patient's physical condition, age, sex, weight, etc.

In the present application, the disease or disorder mediated by degradation of BTK refers to a disease or disorder or related symptoms thereof that can achieve clinically beneficial therapeutic effects such as alleviation, improvement, cessation of progression, alleviation or no longer worsening by degradation of BTK.

In some embodiments, the disease or disorder is cancer or an autoimmune disease.

In some preferred embodiments, the cancer is selected from, but is not limited to: B-cell lymphoma, myeloid leukemia (acute and chronic), acute lymphoblastic leukemia, chronic lymphocytic leukemia, myeloproliferative disease, multiple myeloma, myelodysplastic syndrome, Hodgkin disease, non-Hodgkin lymphoma (malignant lymphoma), hairy cell leukemia, mantle cell lymphoma, Waldenstrom macroglobulinemia, marginal zone lymphoma and follicular lymphoma.

In some preferred embodiments, the cancer is B-cell lymphoma, chronic lymphocytic leukemia, myeloproliferative disease, multiple myeloma, myelodysplastic syndrome, Hodgkin disease, non-Hodgkin lymphoma (malignant lymphoma), hairy cell leukemia, mantle cell lymphoma, Waldenstrom macroglobulinemia, marginal zone lymphoma or follicular lymphoma.

In some more preferred embodiments, the cancer is a B-cell lymphoma.

In some preferred embodiments, the autoimmune disease comprises inflammatory bowel disease, arthritis, lupus, rheumatoid arthritis, psoriatic arthritis, osteoarthritis, Still's disease, juvenile arthritis, diabetes mellitus, myasthenia gravis, Hashimoto's thyroiditis, Ord's thyroiditis, Graves' disease, rheumatoid arthritis syndrome, multiple sclerosis, infectious neuronitis, acute disseminated encephalomyelitis, Addison's disease, opsoclonus-myoclonus syndrome, ankylosing spondylitis, antiphospholipid antibody syndrome, aplastic anemia, autoimmune hepatitis, celiac disease, Goodpasture syndrome, immune thrombocytopenic purpura, optic neuritis, scleroderma, primary biliary cirrhosis, Reiter's syndrome, Takayasu arteritis, temporal arteritis, warm autoimmune hemolytic anemia, Wegener's granulomatosis, psoriasis, alopecia universalis, Behget's disease, chronic fatigue syndrome, familial dysautonomia, endometriosis, interstitial cystitis, neuromyotonia, scleroderma or vulvodynia, and chronic graft-versus-host disease.

It should be noted that any of the aforementioned implementation embodiments in the present application can be freely combined to form new technical solutions.

### DETAILED DESCRIPTION

In order to make the present application easier to understand, the present application will be described in detail below with reference to embodiments. These embodiments are merely illustrative and do not limit the scope of the present application.

### Terms and Definitions:

Unless defined otherwise, all technical and scientific terms used herein have the meaning commonly understood by the ordinary skill in the art to which the present application belongs. As used herein, the following terms have the meanings ascribed below unless otherwise indicated.

The term "substituted" herein refers to the replacement of one or more (e.g., 1, 2, 3, or 4) hydrogen atoms on the designated atom with a selection from the indicated group, provided that the normal valence of the designated atom in the current situation is not exceeded and the substitution forms a stable compound. Combinations of substituents and/or variables are permitted only if such combinations form stable compounds. It should be understood that other atoms, such as hydrogen atoms or substituents as described herein, may be present as needed to satisfy the valence of the atoms.

The terms "independently" or "each independently" mean that when one or more substituents (e.g., R₁, R₂) are selected from many possible substituents, those substituents (e.g., R₁, R₂) may be same or different; and when the same substituent (e.g., R₃) appears multiple times at the same time, it indicates that the substituent (e.g., R₃) may independently be selected from the same or different substituents each time it appears.

When a group is described as "optionally", it may be unsubstituted or substituted with one or more indicated substituents. In the absence of indicated substituents, it means that the group may be optionally substituted with one or more groups selected from, but not limited to deuterium, alkyl, haloalkyl, cycloalkyl, heterocycloalkyl, alkenyl, alkynyl, aryl, heteroaryl, heterocyclyl, halogen, hydroxyl, hydroxyalkyl, alkoxy, haloalkoxy, cyano, nitro, amino, and aminohydroxy; preferably substituted with deuterium, C₁-C₆ alkyl, C₁-C₆ haloalkyl, C₁-C₆ haloalkoxy, C₃-C₆ cycloalkyl, F, Cl, Br, hydroxyl, C₁-C₆ alkoxy, cyano, amino, C₆-C₁₀ aryl, 3-6 membered heterocyclyl, 5-10 membered heteroaryl, or C₁-C₆ hydroxyalkyl.

The term "alkyl" as used in the present application, alone or in combination, refers to a straight-chain or branched saturated hydrocarbon group consisting solely of carbon and hydrogen atoms (including deuterium atoms), including but not limited to C₁-C₆ alkyl, C₁-C₅ alkyl, C₁-C₄ alkyl, C₁-C₃ alkyl, C₁-C₂ alkyl, and C₁ alkyl (including deuterated C₁-C₆ alkyl, deuterated C₁-C₅ alkyl, deuterated C₁-C₄ alkyl, deuterated C₁-C₃ alkyl, deuterated C₁-C₂ alkyl, and deuterated C₁ alkyl). Non-limiting examples of alkyls include the following straight-chain or branched saturated hydrocarbon groups: methyl, ethyl, n-propyl, isopropyl, n-butyl, isobutyl, sec-butyl, tert-butyl, n-pentyl, and its seven additional isomers, n-hexyl, and its sixteen additional isomers. For instance, "C₁-C₆ alkyl" comprises methyl, CD₃, ethyl, propyl, butyl, pentyl, hexyl, and all their isomers; the C₁-C₆ alkyls may be substituted with optional substituents.

In the present application, the term "haloalkyl" refers to an alkyl in which one or more hydrogen atoms are replaced by halogen substituents, such as F or Cl. Non-limiting examples of "C₁-C₆ haloalkyl" include, but are not limited to -CF₃, -CHF₂, -CHCF₂, -CH₂CH₂F, and -CH₂CF₃.

The term "alkoxy" as used in the present application, alone or in combination, refers to "-O-alkyl" group, including deuterated alkoxy. The term alkoxy includes, but is not limited to, C₁-C₆ alkoxy, C₁-C₅ alkoxy, C₁-C₄ alkoxy, C₁-C₃ alkoxy, C₁-C₂ alkoxy, and C₁ alkoxy (including deuterated C₁-C₆ alkoxy, deuterated C₁-C₅ alkoxy, deuterated C₁-C₄ alkoxy, deuterated C₁-C₃ alkoxy, deuterated C₁-C₂ alkoxy, and deuterated C₁ alkoxy). For example, the term "C₁-C₃ alkoxy" indicates a saturated, linear, or branched hydrocarbon moiety containing at least 1 and up to 3 carbon atoms linked by an oxygen atom. Specific exemplary examples include, but are not limited to, methoxy, ethoxy, n-propoxy, isopropoxy, n-butoxy, sec-butoxy, and tert-butoxy (including deuterated methoxy, deuterated ethoxy, deuterated n-propoxy, deuterated isopropoxy, deuterated n-butoxy, deuterated sec-butoxy, and deuterated tert-butoxy). The "alkoxy" may be substituted with optional substituents, non-limiting examples of substitution with F or Cl include -OCF₃, -OCF₂, etc.

In the present application, the term "haloalkoxy" refers to an alkoxy in which one or more hydrogen atoms are replaced by halogen substituents, such as F or Cl. Non-limiting examples of "C₁-C₆ haloalkoxy" include, but are not limited to: -OCF₃, -OCHF₂, -OCHCF₂, -OCH₂CH₂F, and -OCH₂CF₃.

The term "cycloalkyl" as used in the present application, alone or in combination, refers to a saturated monocyclic or polycyclic (e.g., spiro-, fused-, or bridged-) hydrocarbon ring. For example, the term "C₃-C₁₂ cycloalkyl" represents a saturated monocyclic or polycyclic ring containing 3 to 12 carbon atoms; non-limiting examples include cyclopropyl, cyclobutyl, cyclopentyl, cyclohexyl, cycloheptyl, or cyclooctyl, which may optionally be substituted with one or more suitable substituents (e.g., 1 to 3), such as methyl-substituted cyclopropyl. The terms "monocycloalkyl" and "spirocycloalkyl" as used herein fall under the definition of cycloalkyls.

The term "heterocyclyl" as used in the present application, alone or in combination, refers to a monocyclic, bicyclic, or tricyclic group formed by carbon atoms and one or more heteroatoms, where such cyclic group may be saturated, partially unsaturated (non-aromatic), or fully unsaturated (aromatic). The heteroatom may be selected from N, S, or O as a member of the ring, or 1 or 2 -CH₂- groups in ring are substituted with 1 or 2 -C(=O)- or -C(=S)- groups. Specific non-limiting examples include: 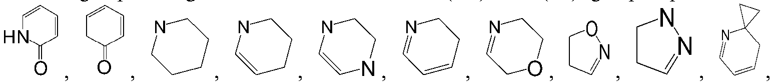 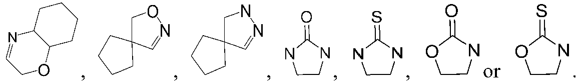 The heterocyclyl groups described herein include "spiroheterocyclyl", "bridged heterocyclyl", "fused heterocyclyl" and "monoheterocyclyl".

The term "heteroaryl" as used in the present application, alone or in combination, refers to a monovalent group with an aromatic structure of a single ring or a fused polycyclic ring, wherein at least 1 (e.g., 1, 2, 3, or 4) ring atom is a heteroatom selected from N, O, and S, and the remaining ring atoms are C; specific non-limiting examples include but are not limited to 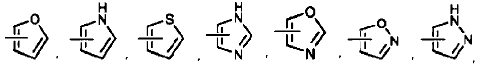 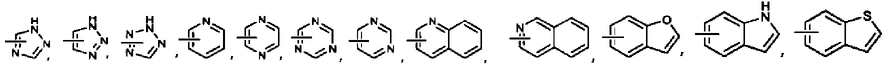 and 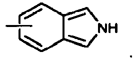 The heteroaryls described herein comprise "spiroheteroaryl", "bridged heteroaryl", "fused heteroaryl", and "monocyclic aryl".

The term "aryl" as used in the present application, alone or in combination in the present application, refers to an aromatic group containing multiple carbon atoms. An aryl is typically a monocyclic, bicyclic, or tricyclic aromatic group with multiple carbon atoms. Additionally, the term "aryl" herein represents an aromatic substituent that can be a single aromatic ring or multiple aromatic rings fused together, non-limiting examples include phenyl, naphthyl, or tetrahydronaphthyl.

In the present application, the term "halogen" refers to F, Cl, Br, or I.

In the present application, the term "hydroxyl" refers to -OH group.

In the present application, the term "amino" refers to -NH₂ group, which may be substituted or unsubstituted. When described as a substituted amino, it is represented as -N(R")ₙ, where R" represents an independent, optional, and suitable substituent (e.g., any of the substituents defined above).

In the present application, the term "cyano" refers to -CN group.

In the present application, the term "nitro" refers to -NO₂ group.

In the present application, the term "hydroxyalkyl" refers to an alkyl in which at least one hydrogen atom is replaced by a hydroxyl. For example, "C₁-C₆ hydroxyalkyl" represents a linear or branched alkyl group having 1 to 6 carbon atoms in which at least one hydrogen atom is replaced by -OH, non-limiting examples include, but are not limited to, -CH₂OH, -CH₂CH₂OH, -CH(CH₃) CH₂OH, and -C(CH₃)₃OH.

In the present application, the aforementioned alkyl, alkoxy, cycloalkyl, aryl, heteroaryl, heterocyclyl, and carbocyclyl are each optionally substituted with one or more substituents.

Unless otherwise specified, the terms "hetero" and "heteroatom" in the present application refer to atoms other than carbon or hydrogen, including, but not limited to O, S, N. In this context, heteroaryl and heterocyclyl may contain one or more heteroatoms (e.g., 1 to 3, or 1 to 2).

In the present application, the term "under a nitrogen atmosphere" refers to connecting a reaction flask to a 1 L nitrogen balloon.

Unless otherwise specified in the present application, the solution mentioned in the reaction herein refer to an aqueous solution.

In the present application, the term "room temperature" refers to a temperature range of 10°C to 25°C.

The intermediate in the present application can be purchased from commercial sources or prepared by known methods disclosed in the prior art.

The compound mentioned in the present application comprises all isomeric forms of the structure, such as stereoisomers (including enantiomers and diastereomers), geometric isomers (or conformational isomers), and tautomer. The term "stereoisomer" (or "optical isomer") refers to a stable isomer that has a perpendicular asymmetry plane due to at least one chiral factor (including a chiral center, chiral axis, chiral plane, etc.), thereby being able to rotate plane-polarized light; the term "tautomer" (or "tautomer form") refers to structural isomers with different energies that can be interconverted through a low energy barrier. For example, proton tautomers (or prototropic tautomers) include (but are not limited to) interconversions via proton migration, such as keto-enol isomerization, imine-enamine isomerization, and amide-iminoalcohol isomerization; the term "cis-trans isomer" refers to stereoisomers formed by the different positions of atoms (or groups) on either side of a double bond or ring system relative to a reference plane; in cis isomers, the atoms (or groups) are on the same side of the double bond or ring system, while in trans isomers, the atoms (or groups) are on opposite sides of the double bond or ring system.

Furthermore, the compounds of the present application may also contain non-natural proportions of atomic isotope forms at one or more atoms constituting these compounds, namely hydrogen isotopes D or T, or isotopes of any atom (e.g., all isotopes of C and N). Specifically, the compounds may be radiolabeled with radioactive isotopes such as tritium (³H), iodine-125 (¹²⁵I), or carbon-14 (¹⁴C). All isotopic variants of the compounds in any optional structural configurations described herein, whether radioactive or not, are covered by the scope of protection. In particular, the application encompasses deuterated forms of hydrogen at any position in the compounds of the general formula described herein, including hydrogen on substituent groups.

The terms "comprise" "include" and "contain" as well as their equivalents, shall be understood in an open and non-exclusive sense; that is, "including, but not limited to" implies that the scope may also encompass other unspecified elements, components, or steps in addition to those listed. Unless otherwise specified, all numerical values indicating amounts of components, measurements, or reaction conditions used herein shall be understood to be modified by the term "about" in all cases. When accompanied by a percentage, the term "about" may represent, for example, ±1%, preferably ±0.5%, more preferably ±0.1%. Unless explicitly indicated by context, singular terms herein encompass plural objects, and vice versa. Similarly, unless specifically stated otherwise, the word "or" herein is intended to include "and".

All patents, patent applications, and other publications are expressly incorporated herein by reference for the purposes of description and disclosure. These publications are provided solely for their disclosure prior to the filing date of the present application. All statements regarding the dates of these documents or the representation of the contents of these documents are based on the information available to the applicant and do not constitute any admission as to the correctness of the dates or the contents of these documents.

### Examples

The present invention is described in detail below by way of examples, but this does not necessarily limit the scope of protection of the present invention. It will be apparent to those skilled in the art that various changes and modifications will be made to the present invention without departing from the spirit and scope of the present invention. Unless otherwise indicated, the reagents, materials, etc. mentioned in the following examples are those commercially available in this area.

### Example 1:

### N-(3-(5-((5-((2S)-4-((1-(4-(2,6-dioxopiperidin-3-yl)-3,5-difluorophenyl)azetidin-3-yl)methyl)-2-methylpiperazin-1-yl)pyridin-2-yl)amino)-1-methyl-6-oxo-1,6-dihydropyridin-3-yl)-5-fluoro-2-methylphenyl)-2-fluoro-4-(2-hydroxypropan-2-yl)benzamide

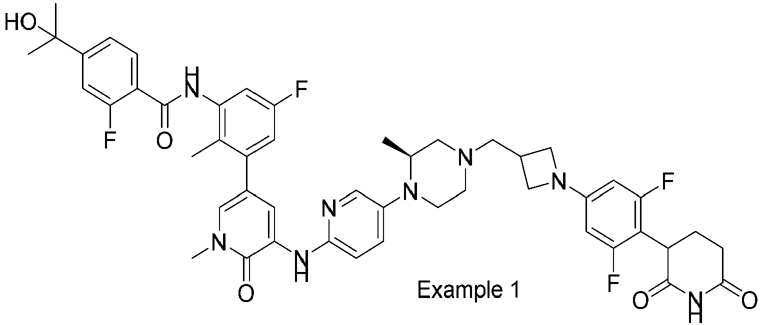

The preparation scheme is shown in the figure below:

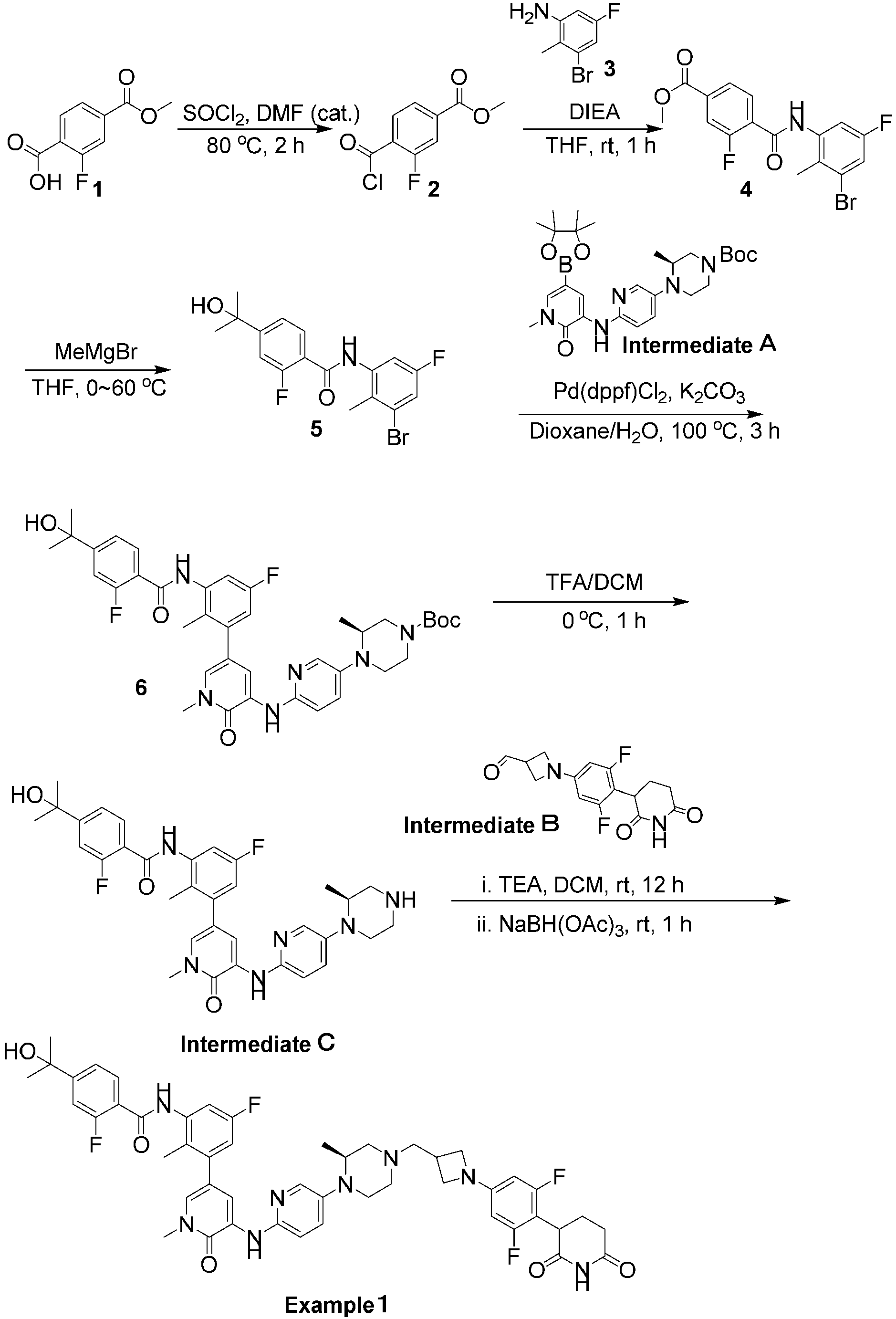

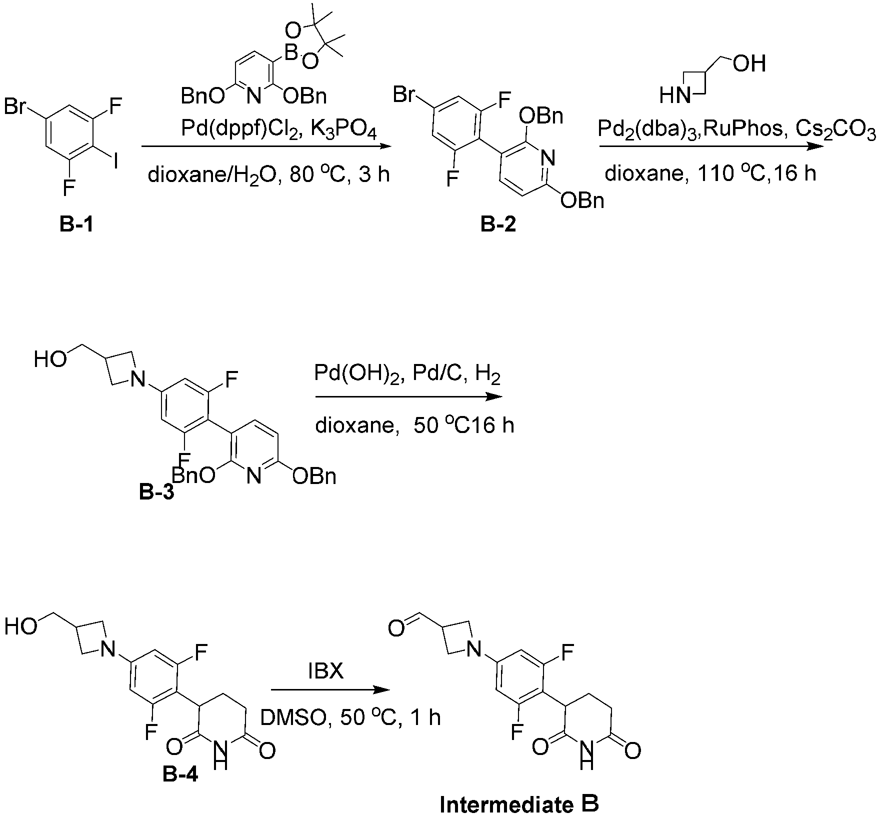

### Step 1: Preparation of methyl 4-(chlorocarbonyl)-3-fluorobenzoate (Intermediate 2)

To a solution of 2-fluoro-4-(methoxycarbonyl)benzoic acid (1100 mg, 5.55 mmol) and thionyl chloride (6.5 mL) was added dropwise N,N-dimethylformamide (0.05 mL). The reaction mixture was heated to 80°C and stirred for 2 h. The reaction solution was concentrated to dryness under reduced pressure to obtain crude Intermediate 2 (1200 mg, colorless oil), which was used directly in the next reaction.

### Step 2: Preparation of methyl 4-((3-bromo-5-fluoro-2-methylphenyl)carbamoyl)-3-fluorobenzoate (Intermediate 4)

To a solution of 3-bromo-5-fluoro-2-methylaniline (1360 mg, 6.66 mmol) in tetrahydrofuran (25 mL) were added N,N-diisopropylethylamine (3590 mg, 27.76 mmol) and Intermediate 2 (1200 mg, 5.54 mmol). The resulting mixture was stirred at 25°C for 1 h. The reaction mixture was evaporated to dryness by rotation and purified by silica gel column chromatography (eluent: ethyl acetate/petroleum ether = 30%-70%) to yield Intermediate 4 (1300 mg, white solid) in a yield rate of 60.9%.

LC-MS:m/z[M+H]⁺=385.8.

### Step 3: Preparation of N-(3-bromo-5-fluoro-2-methylphenyl)-2-fluoro-4-(2-hydroxypropan-2-yl)benzamide (Intermediate 5)

To a solution of Intermediate 4 (1300 mg, 3.38 mmol) in tetrahydrofuran (30 mL) was added methylmagnesium bromide (3.38 mL, 10.14 mmol, 3 M tetrahydrofuran solution) dropwise at 0°C. The mixture was stirred at 0°C for 0.5 h, then the reaction mixture was heated to 60°C and stirred for 16 h. The mixture was poured into a saturated aqueous ammonium chloride solution (200 mL) and extracted with ethyl acetate (100 mL × 3). The combined organic phases were washed with brine (50 mL×× 2), dried over anhydrous sodium sulfate, filtered, and concentrated to dryness under reduced pressure to obtain a crude product. The crude product was purified by silica gel column chromatography (eluent: ethyl acetate/petroleum ether = 10%-50%) to obtain Intermediate 5 (1897 mg, yellow solid) in a yield of 73.81%.

LC-MS:m/z[M+H]⁺=385.8.

### Step 4: Preparation of (S)-4-(6-((5-(5-(2-fluoro-4-(2-hydroxypropan -2-yl)benzamido)-2-methylphenyl)-1-methyl-2-oxo-1,2-dihydropyridin-3-yl)amino)pyridin-3-yl]-3-methylpiperazine-1-carboxylic acid tert-butyl ester (Intermediate 6)

To a solution of Intermediate 5 (1200 mg, 3.12 mmol), Intermediate A (2460 mg, 4.68 mmol), and potassium carbonate (1290 mg, 9.37 mmol) in dioxane (60 mL) and water (12 mL) was added 1,1-bis(diphenylphosphino)diphenylferric palladium(II) chloride (228.6 mg, 0.31 mmol). The reaction system was purged with nitrogen three times and stirred at 100°C under a nitrogen atmosphere for 3 h. The reaction solution was evaporated to dryness by rotation and purified by silica gel column chromatography (eluent: petroleum ether/ethyl acetate = 10%-50%) to obtain Intermediate 6 (1350 mg, purple solid) in a yield of 61.5%.

LC-MS:m/z[M+H]⁺=703.2.

The preparation of Intermediate A follows the method disclosed in patent WO2021091575.

### Step 5: Preparation of (S)-2-fluoro-N-(5-fluoro-2-methyl-3-(1-methyl-5-((5-(2-methylpiperazin-1-yl)pyridin-2-yl)amino)-6-oxo-1,6-dihydropyridin-3-yl)phenyl)-4-(2-hydroxypropan-2-yl)benzamide (Intermediate C)

To a solution of Intermediate 6 (700 mg, 0.99 mmol) in dichloromethane (9 mL) was added trifluoroacetic acid (3 mL), and the resulting mixture was stirred at 25°C for 1 h. The reaction solution was concentrated under reduced pressure to obtain a crude Intermediate C (600 mg, brown solid).

LC-MS:m/z[M+H]⁺=603.2.

### Step 6: Preparation of 2,6-bis(benzyloxy)-3-(4-bromo-2,6-difluorophenyl)pyridine (Compound B-2)

To a solution of 5-bromo-1,3-difluoro-2-iodobenzene (4000 mg, 12.50 mmol), 2,6-bis(benzyloxy)-3-(4,4,5,5-tetramethyl-1,3,2-dioxaborolan-2-yl)pyridine (5200 mg, 12.50 mmol), and potassium phosphate (5300 mg, 25.00 mmol) in dioxane (40 mL) and water (8 mL) was added 1,1-bis(diphenylphosphino)ferrocenepalladium(II) dichloride (917 mg, 1.25 mmol). The mixture was purged with nitrogen three times and stirred at 100°C under a nitrogen atmosphere for 16 h. The reaction mixture was evaporated to dryness by rotation and purified by silica gel column chromatography (eluent: ethyl acetate/petroleum ether = 0-5%) to obtain compound B-2 (3500 mg, colorless oil) in a yield of 58.1%.

LC-MS:m/z[M+H]⁺=483.8.

### Step 7: Preparation of (1-(4-(2,6-bis(benzyloxy)pyridin-3-yl)-3,5-difluorophenyl)azetidin-3-yl)methanol (Compound B-3)

To a solution of compound D-2 (600 mg, 1.24 mmol) and azetidin-3-ylmethanol (185 mg, 1.49 mmol) in 1,4-dioxane (20 mL) were added cesium carbonate (1620 mg, 4.97 mmol), 2-dicyclohexylphosphino-2',6'-diisopropoxy-1,1'-biphenyl (116 mg, 0.25 mmol), and tris(dibenzylideneacetone)dipalladium (114 mg, 0.12 mmol). The mixture was purged with nitrogen three times and stirred at 100°C under a nitrogen atmosphere for 16 h. The reaction mixture was evaporated to dryness by rotation and purified by silica gel column chromatography (eluent: petroleum ether/ethyl acetate = 10%-50%) to obtain compound D-3 (475 mg, yellow solid) in a yield of 78.4%.

LC-MS:m/z[M+H]⁺=489.0.

### Step 8: Preparation of 3-(2,6-difluoro-4-(3-(hydroxymethyl)azetidin-1-yl)phenyl)piperidine-2,6-dione (Compound B-4)

To a solution of compound B-3 (475 mg, 0.97 mmol) in 1,4-dioxane (15 mL) were added 10% Pd/C (150 mg) and palladium hydroxide on carbon (150 mg). The resulting mixture was purged with hydrogen three times and then stirred at 50°C under hydrogen protection for 16 h. The reaction solution was filtered through a pad of celite, the filter cake was washed with methanol (20 mL×3), and the filtrate was concentrated to dryness under reduced pressure to obtain compound B-4 (237 mg, blue solid) in a yield of 78.7%.

LC-MS:m/z[M+H]⁺=310.8.

### Step 9: Preparation of 1-(4-(2,6-dioxopiperidin-3-yl)-3,5-difluorophenyl)azetidine-3-carbaldehyde (Intermediate B)

To a solution of compound B-4 (237 mg, 0.76 mmol) in dimethyl sulfoxide (100 mL) was added 2-iodoxybenzoic acid (428 mg, 1.53 mmol), and the reaction was stirred at 50°C for 1 h. The reaction solution was added to water (20 mL) and extracted with ethyl acetate (20 mL×3). The combined organic phases were washed with brine (10 mL×2), dried over anhydrous sodium sulfate, filtered, and concentrated to dryness under reduced pressure to obtain a crude product. The crude product was purified by silica gel column chromatography (eluent: ethyl acetate/petroleum ether = 10%-50%) to obtain Intermediate B (160 mg, yellow solid) in a yield of 68.2%.

LC-MS:m/z[M+H]⁺=308.8.

### Step 10: Preparation of N-(3-(5-((5-((2S)-4-((1-(4-(2,6-dioxopiperidin-3-yl)-3,5-difluorophenyl)azetidin-3-yl)methyl)-2-methylpiperazin-1-yl)pyridin-2-yl)amino)-1-methyl-6-oxo-1,6-dihydropyridin-3-yl)-5-fluoro-2-methylphenyl)-2-fluoro-4-(2-hydroxypropane-2-yl)benzamide (Example 1)

To Intermediate C (60 mg, 0.08 mmol) in dichloromethane (1 mL) were added Intermediate B (39.7 mg, 0.13 mmol) and triethylamine (34.8 mg, 0.34 mmol), and the mixture was stirred at 25°C for 16 h. Sodium triacetoxyborohydride (45.6 mg, 0.21 mmol) was then added to the reaction system, and the mixture was stirred at 25°C for 1 h. The crude product was purified on a silica gel preparative plate (developing solvent: 100% ethyl acetate) to afford Example 1 (16.10 mg of a purple solid) in a 20.9% yield.

LC-MS:m/z[M+H]⁺=895.4.

¹HNMR(400MHz,CDCl₃) δ8.62-8.54(m,1H), 8.47-8.48(m,1H), 8.17-8.21(m,1H), 8.04-8.08(m,1H), 8.00(s,1H), 7.93-7.94(m,1H), 7.83(s,1H), 7.42(s,1H), 7.40-7.36(m,1H), 7.28-7.29(m,1H), 6.83-6.86(m,1H), 6.80-6.75(m,2H), 5.94(d,J=10.4Hz,2H), 4.01-3.90(m,3H), 3.68(s,3H), 3.54(s,2H), 3.46(s,1H), 3.02-3.04(m,2H), 2.99-2.92(m,1H),2.77-2.78(m,1H),2.69-2.58(m,5H),2.52-2.53(m,1H),2.32-2.36(m,1H),2.27(s,3H),2.17-2.06(m,1H),1.92(s,1H),1.61(s,6H),1.25(s,1H),0.96(d,J=6.4Hz,3H).

### Example 2: N-(3-(5-((5-(2S)-4-((1s,3R)-3-(2-((2-(2,6-dioxopiperidin-3-yl)-1,3-dioxoisoindol-4-yl)amino)ethoxy)cyclobutane-1-carbonyl)-2-methylpiperazin-1-yl)pyridin-2-yl)amino]-1-methyl-6-oxo-1,6-dihydropyridin-3-yl)-5-fluoro-2-methylphenyl)-2-fluoro-4-(2-hydroxypropan-2-yl)benzamide

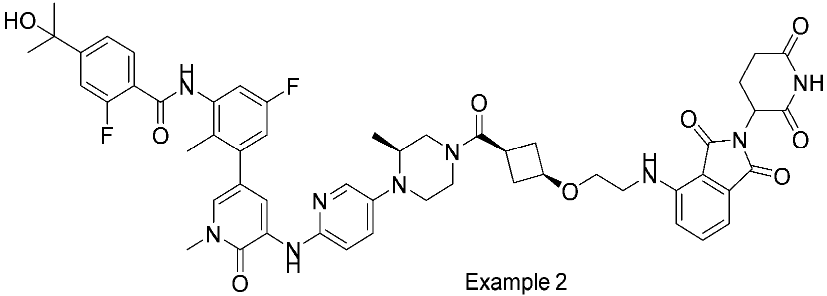

The preparation scheme is shown in the figure below:

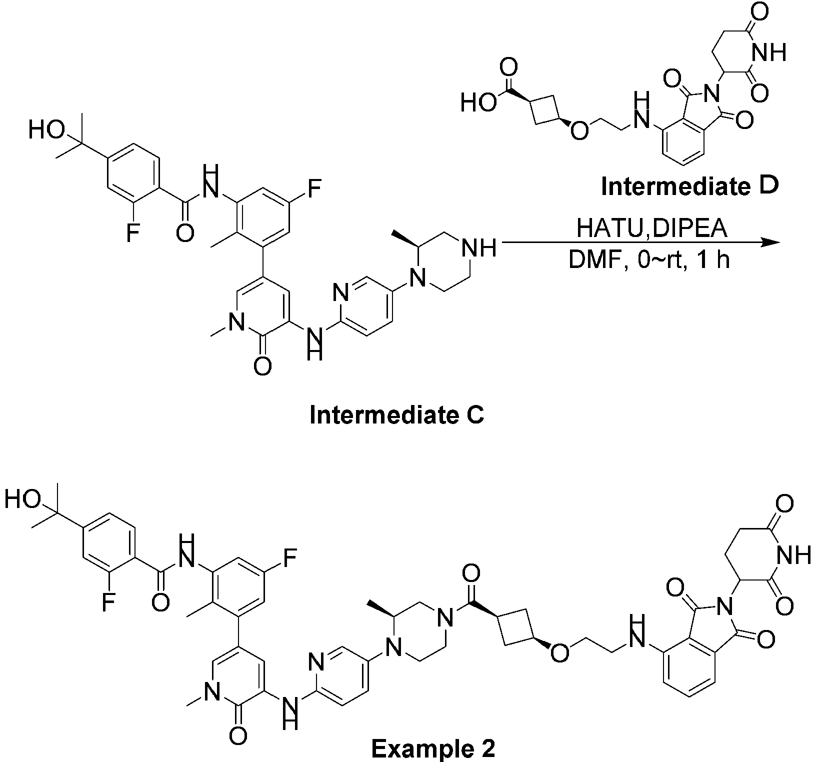

To a solution of Intermediate C (86 mg, 0.14 mmol) in N,N-dimethylformamide (3 mL) at room temperature were added Intermediate D (59 mg, 0.14 mmol) and N,N-diisopropylethylamine (54 mg, 0.42 mmol), followed by 2-(7-azobenzotriazole)-N,N,N',N'-tetramethyluronium hexafluorophosphate (80 mg, 0.21 mmol). The resulting reaction solution was stirred at room temperature for 1 h. The reaction solution was poured into water, and the precipitate was collected and dried to obtain the crude product. The crude product was purified by preparative thin layer chromatography (developing solvent: petroleum ether/EE (ethyl acetate/ethanol = 3:1)/methanol/dichloromethane = 38/12/45/5) to obtain Example 2 (29.14 mg, yellow solid) in a yield of 20.8%.

LC-MS:m/z[M+H]⁺=1000.4.

¹HNMR(400MHz,DMSO-*d*₆) δ11.12(s,1H), 9.94(s,1H), 8.66-8.35(m,2H), 7.86(s,1H), 7.73-7.62(m,1H), 7.65-7.58(m,1H), 7.45-7.42(m,4H), 7.28-7.18(m,2H), 7.16-7.10(m,1H), 7.06-7.01(m,2H), 6.62(s,1H), 5.33(s,1H), 5.10-5.08(m,1H), 3.95(m,1H), 3.62(s,4H), 3.55-3.46(m,5H), 3.09-2.87(m,7H), 2.36(s,3H), 2.23(s,3H), 2.11-2.04(m,3H), 1.49(s,6H), 0.82(m,4H).

The preparation of Intermediate D follows the method disclosed in Patent WO2021091575.

### Example 3: 3-((4-(1-(2-(6-(2,6-dioxopiperidin-3-yl)-5,7-dioxo-3,5,6,7-tetrahydropyrrolo[3,4-f]isoindol-2(1H)-yl)acetyl)piperidin-4-yl)phenyl)amino)-5-((R)-3-(3-methyl-2-oxoimidazolidin-1-yl)piperidin-1-yl)pyrazine-2-carboxamide

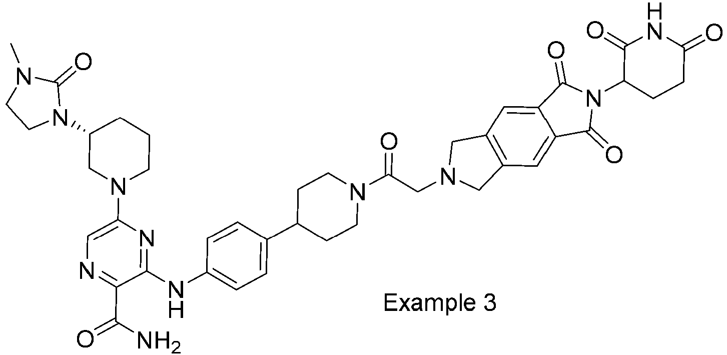

The preparation scheme is shown in the figure below:

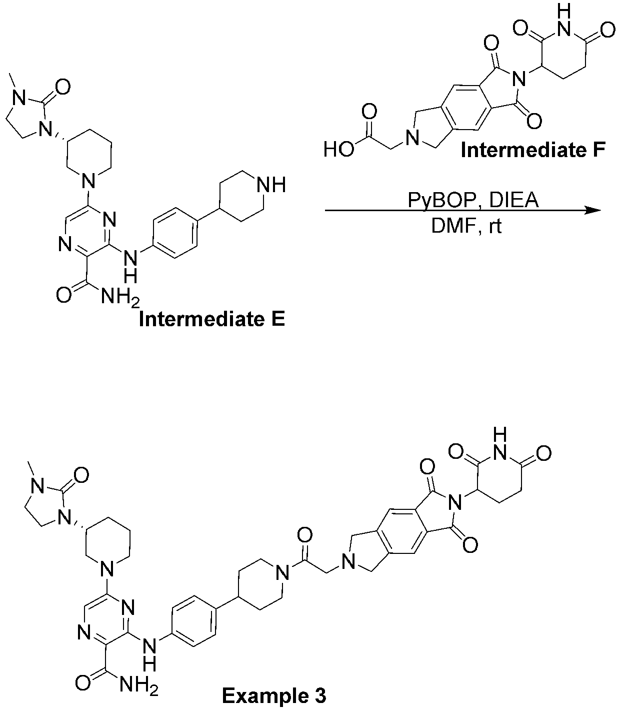

To a solution of Intermediate E (59 mg, 0.10 mmol) in N,N-dimethylformamide (2 mL) were added N,N-diisopropylethylamine (64 mg, 0.50 mmol), PyBop (104 mg, 0.20 mmol), and Intermediate F (40 mg, 0.11 mmol), and the mixture was stirred at 25°C for 1 h. The mixture was poured into water (10 mL) and extracted with ethyl acetate (10 mL×3). The combined organic phases were washed with brine (10 mL×2), dried over anhydrous sodium sulfate, filtered, and concentrated to dryness under reduced pressure. The crude product was purified on a silica gel prep plate (developing solvent: methanol/dichloromethane = 1/10) to obtain Example 3 (21.77 mg as a white solid) in a yield of 26.6%.

LC-MS:m/z[M+H]+=818.4.

¹HNMR(400MHz,DMSO-*d₆)*δ11.16-11.18(m,2H),7.79-7.77(m,3H),7.66(s,1H),7.51-7.49(m,2H),7.32(s,1H),7.16-7.14(m,2H),5.21-5.06(m,1H),4.54-4.52(m,1H),4.32-4.30(m,2H),4.13 (s,4H),3.78-3.49(m,3H),3.26-3.24(m,2H),3.15-2.81(m,5H),2.66-2.64(m,7H),2.07(s,1H),1.80(s,5H),1.51-1.49(m,4H),1.23(s,1H).

The preparation of Intermediate E follows the method disclosed in WO 2021113557.

The preparation of Intermediate F follows the method disclosed in J. Med. Chem. 2023,66,17,12559-12585.

### Example 4: 3-((4-(1-(1-(4-(2,6-dioxopiperidin-3-yl)-3,5-difluorophenyl)azetidin-3-yl)methyl)piperidin-4-yl)phenyl)amino)-5-((R)-3-(3-methyl-2-oxoimidazolidin-1-yl)piperidin-1-yl)pyrazine-2-carboxamide

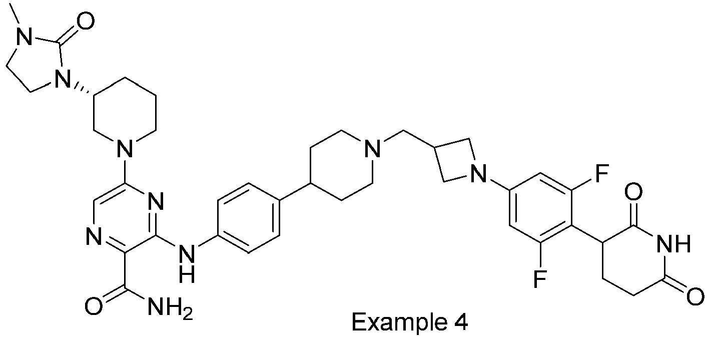

The preparation scheme is shown in the figure below:

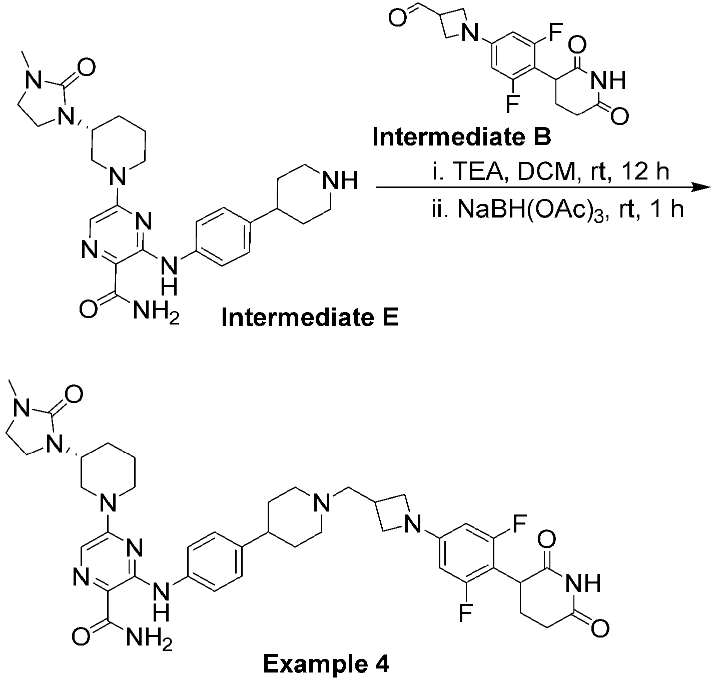

To a solution of Intermediate E (130 mg, 0.23 mmol) in dichloromethane (8 mL) were added Intermediate B (84 mg, 0.27 mmol) and triethylamine (92 mg, 0.91 mmol), and the reaction mixture was stirred at 25°C for 16 h. Sodium triacetoxyborohydride (121 mg, 0.57 mmol) was then added to the reaction system, and the mixture was stirred at 25°C for 1 h. The crude product was purified on a silica gel preparative plate (developing solvent: methanol/dichloromethane = 1/10) to afford Example 4 (11.16 mg, white solid) in a yield of 6.3%.

LC-MS:m/z[M+H]⁺=771.4.

¹HNMR(400MHz,DMSO-*d*₆) δ11.19(s,1H), 10.87(s,1H), 7.77(s,1H), 7.66(s,1H), 7.49 (d, J = 8.4 Hz, 2H), 7.34(s,1H), 7.16 (d, J = 8.4 Hz, 2H), 6.13-6.11(m,2H), 4.33-4.30(m,2H), 4.04-4.00(m,1H), 3.96-3.92(m,2H), 3.60 (m, 1H), 3.51-3.46(m, 3H), 3.27(m, 4H), 3.08-3.00((m, 1H), 3.00-2.86(m, 4H), 2.76(m,1H), 2.71 (s, 3H), 2.58(m, 2H), 2.40(m, 1H),2.06-2.02(m,3H), 1.98-1.89(m,1H), 1.80-1.76(m,5H), 1.61-1.57(m,3H).

### Example 5:

### 4-((5-((2S)-4-((1-(4-(2,6-dioxopiperidin-3-yl)-3,5-difluorophenyl)azetidin-3-yl)methyl)-2-methylpiperazin-1-yl)pyridin-2-yl)amino)-1-(5-fluoro-3-(2-fluoro-4-(2-hydroxypropan-2-yl)benzamido)-2-methylphenyl)-1H-pyrazole-3-carboxamide

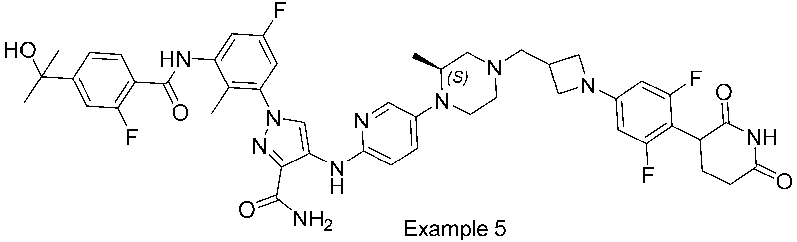

The preparation scheme is shown in the figure below:

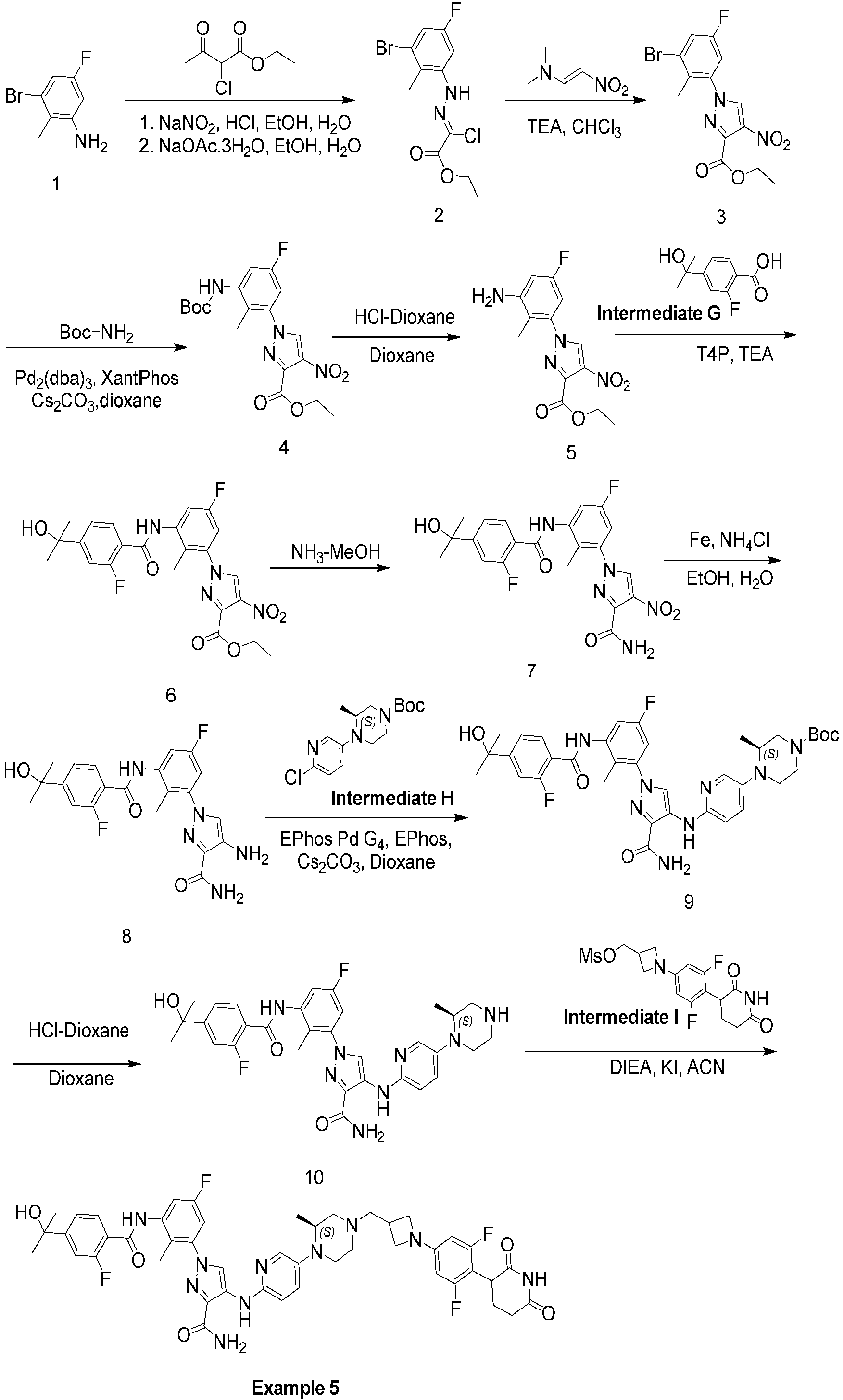

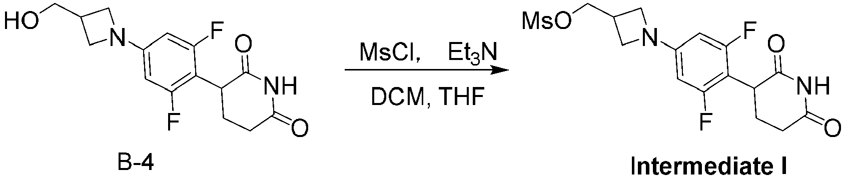

### Step 1: Preparation of ethyl 2-[(1Z)-2-(3-bromo-5-fluoro-2-methylphenyl)diazolylidene]-2-chloroacetate (Compound 2)

To a solution of compound 1 (8300 mg, 40.68 mmol) and hydrochloric acid (33.90 mL, 203.39 mmol) in ethanol (90 mL) and water (10 mL) was added dropwise sodium nitrite (3367.67 mg, 48.81 mmol) in water (10 mL) at -5°C to 5°C, and the reaction mixture was stirred at -5°C to 5°C for 0.5 h. To a solution of ethyl 2-chloro-3-oxobutanoate (6695.24 mg, 40.68 mmol) and sodium acetate trihydrate (38748.52 mg, 284.75 mmol) in ethanol (90 mL) and water (10 mL) was added dropwise the reaction mixture at -5°C to 5°C, and the mixture was stirred at 25°C for 1.5 h. The reaction mixture was added to water (1000 mL) and filtered, and the filter cake was dried under reduced pressure to obtain compound 2 (12.25 g, red solid) in a yield of 89.21%.

LC-MS:m/z[M+H]⁺=337.

### Step 2: Preparation of ethyl 1-(3-bromo-5-fluoro-2-methylphenyl)-4-nitropyrazole-3-carboxylate (Compound 3)

To a solution of compound 2 (12.25 g, 36.29 mmol) and triethylamine (10.06 mL, 72.58 mmol) in chloroform (70 mL) was added dimethyl[(1E)-2-nitrovinyl]amine (4.21 g, 36.29 mmol), the mixture was degassed and purged with nitrogen three times, and stirred at 70°C under a nitrogen atmosphere for 18 h. The reaction mixture was added to water (300 mL) and extracted with dichloromethane (150 mL×3). The combined organic phases were dried over anhydrous sodium sulfate, filtered and concentrated to dryness under reduced pressure to obtain a crude product. The crude product was purified by column chromatography (eluent: petroleum ether/ethyl acetate = 10: 1) to obtain compound 3 (2.65 g, orange solid) in a yield of 19.62%.

LC-MS:m/z[M+H]⁺=372.

### Step 3: Preparation of ethyl 1-[5-fluoro-2-methyl-3-({[(2-methylprop-2-yl)oxy]carbonyl}amino)phenyl]-4-nitropyrazole-3-carboxylate (Compound 4)

To a solution of compound 3 (2500 mg, 6.72 mmol), tert-butyl carbamate (1023.08 mg, 8.73 mmol), and cesium carbonate (4377.54 mg, 13.44 mmol) in dioxane (30 mL) were added 2-dicyclohexylphosphino-2',4',6'-triisopropylbiphenyl (480.38 mg, 1.01 mmol) and tris(dibenzylideneacetone)dipalladium (24.61 mg, 0.03 mmol) at room temperature. The reaction mixture was stirred at 100°C under a nitrogen atmosphere overnight to yield a black suspension. The reaction mixture was filtered and concentrated. The crude product was isolated and purified using a silica gel column (petroleum ether with 2%-10% ethyl acetate) to afford compound 4 (2480 mg, yellow solid) in a yield of 90.5%.

LC-MS:m/z[M+Na]⁺=431.2.

### Step 4: Preparation of 1-(3-amino-5-fluoro-2-methylphenyl)-4-nitropyrazole-3-carboxylic acid ethyl ester (Compound 5)

To a solution of compound 4 (2480 mg, 6.07 mmol) in 1,4-dioxane (15 mL) was added 1,4-dioxane hydrochloride (15 ml), and the resulting mixture was stirred at 25°C for 2 h to obtain a yellow solution. The reaction mixture was concentrated to dryness under reduced pressure to obtain crude compound 5 (1270 mg, yellow solid) in a yield of 67.84%.

LC-MS:m/z[M+H]⁺=309.2.

### Step 5: Preparation of ethyl 1-(5-fluoro-3-(2-fluoro-4-(2-hydroxypropan-2-yl)benzamido)-2-methylphenyl)-4-nitro-1H-pyrazole-3-carboxylate (Compound 6)

To a solution of compound 5 (450 mg, 1.46 mmol) and Intermediate G (318.24 mg, 1.61 mmol) in 1-butylphosphonic anhydride (8 mL) was added triethylamine (0.51 mL, 3.65 mmol) at room temperature. The reaction mixture was heated in a microwave oven at 80°C under a nitrogen atmosphere for 1 h to yield a yellow, clear liquid. The reaction mixture was diluted with ethyl acetate (60 mL) and washed sequentially with water (20 mL) and saturated sodium bicarbonate (20 mL), dried over anhydrous sodium sulfate, filtered, and concentrated. The crude product was purified using a silica gel column (petroleum ether with 20%-50% ethyl acetate) to yield compound 6 (180 mg, yellow oil) in a yield of 25.24%.

LC-MS:m/z[M+Na]⁺=511.2.

The preparation of Intermediate G follows the method published in J.Med.Chem.2020, 63, 5102-5118.

### Step 6: Preparation of 1-(5-fluoro-3-(2-fluoro-4-(2-hydroxypropyl-2-yl)benzamido)-2-methylphenyl)-4-nitro-1H-pyrazole-3-carboxamide (Compound 7)

Compound 6 (180 mg, 0.37 mmol) was dissolved in NH₃ -MeOH (7N, 4 mL) at room temperature. The reaction mixture was stirred at 60°C for 6 h to yield a yellow solution. The reaction mixture was concentrated to afford compound 7 (130 mg, white solid) in a yield of 76.79%. The crude product was used directly in the next step.

LC-MS:m/z[M+Na]⁺=482.2.

### Step 7: Preparation of 4-amino-1-(5-fluoro-3-(2-fluoro-4-(2-hydroxypropan-2-yl)benzamido)-2-methylphenyl)-1H-pyrazole-3-carboxamide (Compound 8)

Iron powder (63.20 mg, 1.13 mmol) was added to a solution of compound 7 (130 mg, 0.28 mmol) and ammonium chloride (151.36 mg, 2.83 mmol) in ethanol (4 mL) and water (2 mL). The reaction was refluxed for 1 h to yield a black suspension. After completion of the reaction, the reaction solution was filtered and concentrated. The crude product was purified by using a silica gel column (petroleum ether with 40%-100% ethyl acetate) to afford compound 8 (100 mg, yellow solid) in a yield of 82.29%.

LC-MS:m/z[M+Na]⁺=452.2.

### Step 8: Preparation of (S)-tert-butyl 4-(6-((3-carbamoyl-1-(5-fluoro-3-(2-fluoro-4-(2-hydroxypropyl-2-yl)benzamido)-2-methylphenyl)-1H-pyrazol-4-yl)amino)pyridin-3-yl)-3-methylpiperazine-1-carboxylate (Compound 9)

To a solution of compound 8 (100 mg, 0.23 mmol), Intermediate H (94.39 mg, 0.30 mmol) and cesium carbonate (151.75 mg, 0.47 mmol) in dioxane (4 mL) were added dicyclohexyl (3-ethylpropoxy-2',4',6'-triisopropyl-[1,1'-diphenyl]-2-yl) phosphine (24.91 mg, 0.05 mmol) and methanesulfonato{dicyclohexyl(3-isopropoxy-2',4',6'-triisopropyl-[1,1'-biphenyl]-2-yl)phosphane}(2'-(methylamino)-[1,1'-biphenyl]-2-yl)palladium(II) (42.78 mg, 0.05 mmol). The reaction mixture was refluxed overnight under a nitrogen atmosphere to yield a brown suspension. The reaction mixture was filtered and concentrated. The crude product was purified using a silica gel column (petroleum ether with 40-100% ethyl acetate) to afford compound 9 (90 mg, pale yellow solid) in a yield of 54.84%.

LC-MS:m/z[M+H]⁺=705.0.

The preparation of Intermediate H follows the method disclosed in patent WO2021091575.

### Step 9: Preparation of (S)-1-(5-fluoro-3-(2-fluoro-4-(2-hydroxypropyl-2-yl)benzamido)-2-methylphenyl)-4-((5-(2-methylpiperazin-1-yl)pyridin-2-yl)amino)-1H-pyrazole-3-carboxamide (Compound 10)

To a solution of compound 9 (100 mg, 0.14 mmol) in dioxane (4 mL) was added hydrochloric acid-dioxane (3 mL) at room temperature. The reaction mixture was stirred under a nitrogen atmosphere for 2 h to obtain a yellow suspension. After the reaction, the reaction mixture was concentrated to obtain crude compound 10 (90 mg, yellow solid).

LC-MS:m/z[M+H]⁺=605.4.

### Step 10: Preparation of (1-(4-(2,6-dioxopiperidin-3-yl)-3,5-difluorophenyl)azetidin-3-yl)methyl methanesulfonate (Intermediate I)

To a solution of compound B-4 (80 mg, 0.16 mmol) and triethylamine (65.41 µL, 0.47 mmol) in tetrahydrofuran (5 mL) and dichloromethane (5 mL) at 0°C to 25°C was added methanesulfonyl chloride (24.35 µL, 0.31 mmol) in portions. The reaction mixture was stirred at room temperature under a nitrogen atmosphere for 2 h to yield a white suspension. The reaction mixture was diluted with dichloromethane (100 mL), washed sequentially with saturated ammonium chloride solution (20 mL) and with brine (20 mL), dried over anhydrous sodium sulfate, and filtered and concentrated to yield Intermediate I (90 mg, white solid) in a yield of 97.53%. The crude product was used directly in the next step.

LC-MS:m/z[M+H]⁺=587.0

### Step 11: Preparation of 3-((4-(1-((1-(6-(((S)-2,6-dioxopiperidin-3-yl)carbamoyl)pyridin-3-yl)piperidin-4-yl)methyl)piperidin-4-yl)phenyl)amino)-5-((R)-3-(3-methyl-2-thionemidazolidin-1-yl)piperidin-1-yl)pyrazine-2-carboxamide (Example 5)

To a solution of compound 10 (90 mg, 0.15 mmol), potassium iodide (74.12 mg, 0.45 mmol), and N,N-diisopropylethylamine (96.19 mg, 0.74 mmol) was added Intermediate I (69.37 mg, 0.18 mmol) at room temperature. The reaction mixture was stirred at 80°C under a nitrogen atmosphere overnight to yield a yellow solution. After the reaction, the reaction mixture was filtered and the mother liquor was collected. The mother liquor was separated and purified using prep-HPLC to yield Example 5 (7 mg, white solid) in a yield of 5.24%.

LC-MS:m/z[M+H]⁺=897.3.

¹HNMR(400MHz,DMSO-*d*₆) δ10.85(s,1H), 10.10(d,J=2.0Hz,1H), 8.90(s,1H), 8.56(s,1H), 7.88(d,J=2.8Hz,1H), 7.78(s,1H), 7.71(m,1H), 7.61(d,J=9.0Hz,1H), 7.50(s,1H), 7.46-7.33(m,4H), 6.95(d,J=9.0Hz,1H), 6.16-6.07(m,2H), 5.36-5.29(m,1H), 4.04-4.01(m,1H), 3.97-3.92(m,2H), 3.51(s,4H), 3.04-2.57(m,7H), 2.43-2.35(m,3H), 2.08(s,3H), 2.06-1.87(m,2H), 1.45(s,6H), 0.90-0.86(m,3H).

### Example 6:

### (R)-3-((4-(1-(2-(4-(4-(2,4-dioxotetrahydropyrimidin-1(2H)-yl)-1H-indol-1-yl)piperidin-1-yl)acetyl)piperidin-4-yl)phenyl)amino)-5-(3-(3-methyl-2-oxoimidazolidin-1-yl)piperidin-1-yl)pyrazine-2-carboxamide

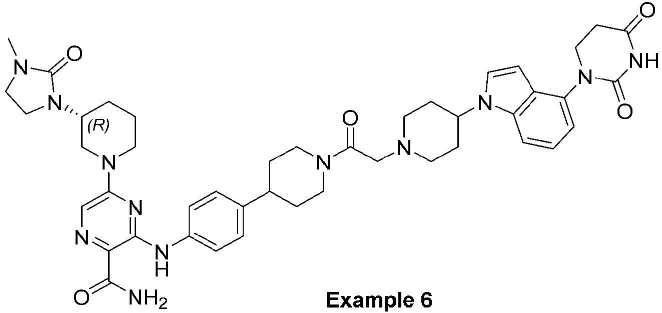

The preparation scheme is shown in the figure below:

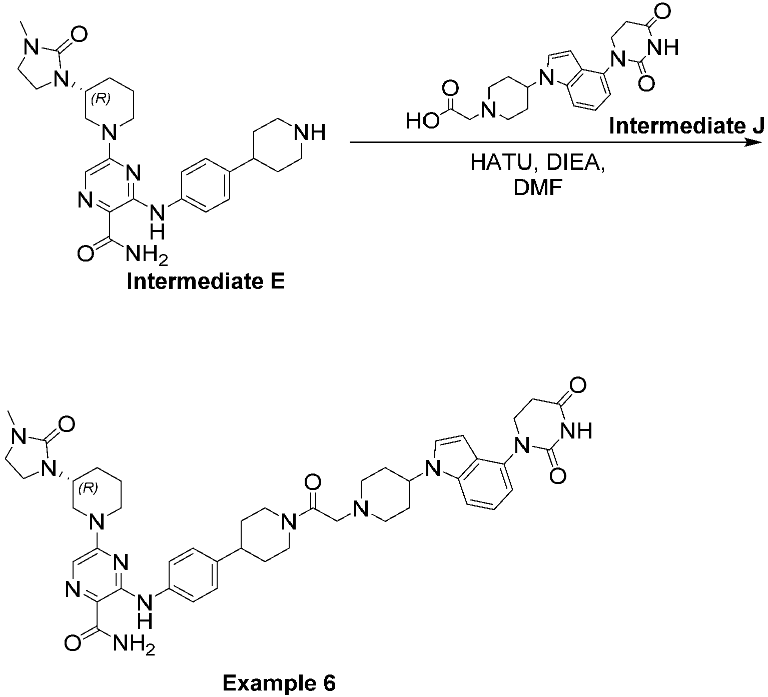

Intermediate E (180 mg, 0.25 mmol) was added to a mixture of Intermediate J (120 mg, 0.32 mmol), HATU (200 mg, 0.53 mmol), N,N-diisopropylethylamine (220 mg, 1.70 mmol), and N,N-dimethylformamide (3 mL) and allowed to react at 20°C for 2 h. The reaction mixture was filtered and purified by prep-HPLC (acetonitrile: water containing 0.1% formic acid) to afford Example 6 (65 mg, yellow solid) **in** a yield of 30.71%.

LC-MS:m/z[M+H]⁺=831.4.

¹HNMR(400MHz,DMSO-*d₆*) δ11.23(d,J=7.2Hz,1H), 10.34(s,1H), 7.77(s,1H), 7.66(s,1H), 7.56-7.49(m,4H), 7.34(s,1H),7.21-7.13(m,3H),6.97(d,J=7.4Hz,1H),6.44(d,J=2.6Hz,1H),4.53(d,J=7.2Hz,2H),4.34-4.27(m,2H),4.10(s,1H),3.78(m,2H),3.64-3.58(m,1H),3.32-2.85(m,11H),2.81-2.65(m,8H),2.29-1.97(m,4H),1.93-1.35(m,9H).

The preparation of Intermediate J follows the method disclosed **in** Patent WO2023180388.

### Example 7: 3-((4-(1-((1-(6-(((S)-2,6-dioxopiperidin-3-yl)carbamoyl)pyridin-3-yl)piperidin-4-yl)methyl)piperidin-4-yl)phenyl)amino)-5-((R)-3-(3-methyl-2-thioneimidazolidin-1-yl)piperidin-1-yl)pyrazine-2-carboxamide

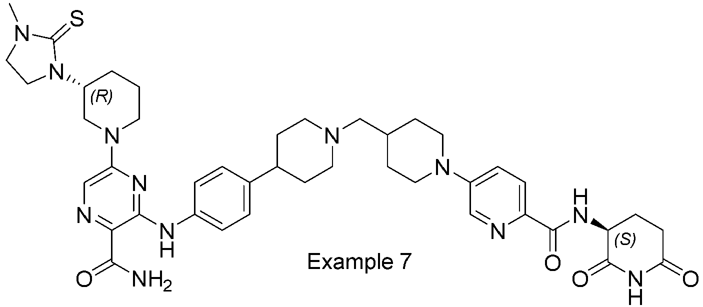

The preparation scheme is shown in the figure below:

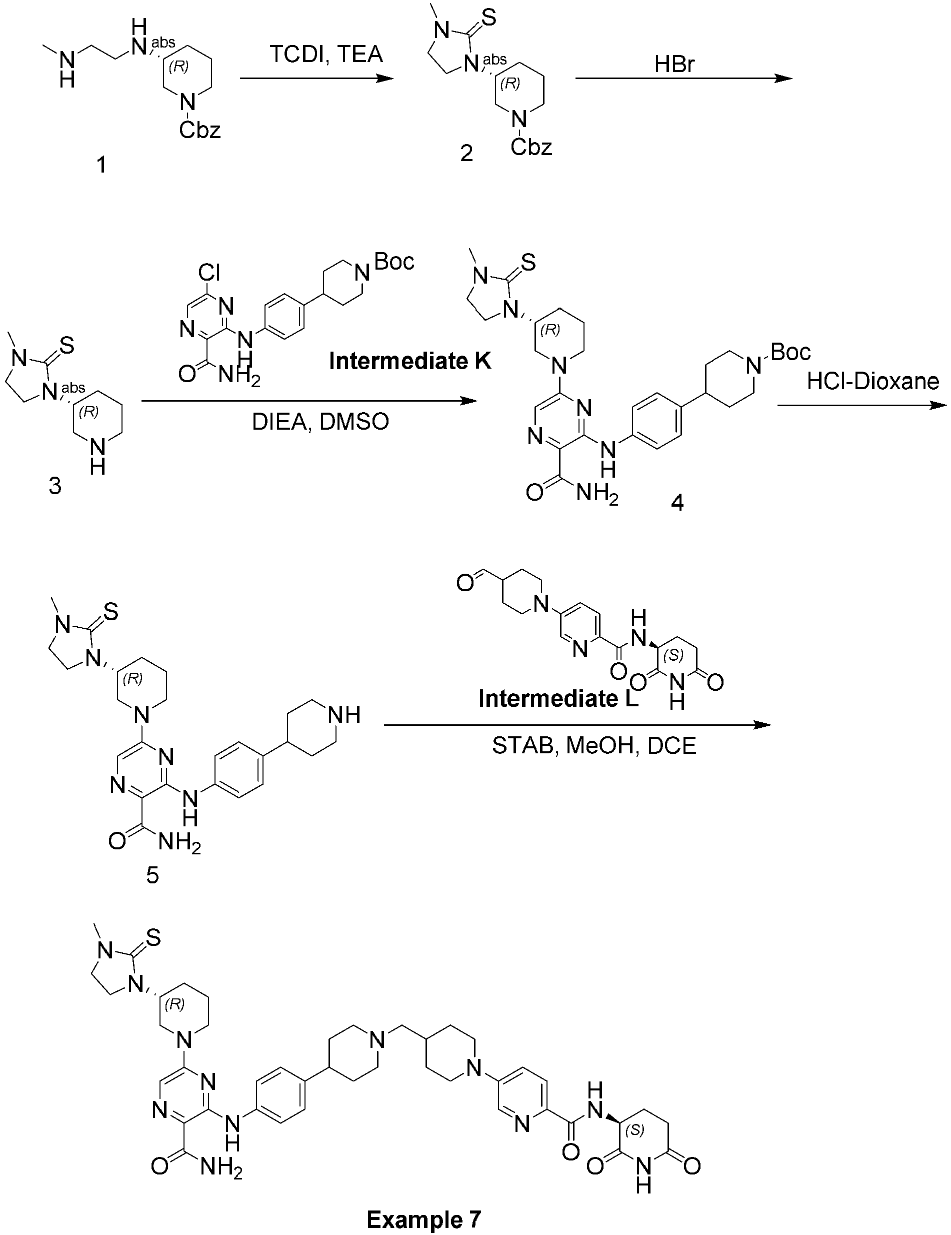

### Step 1: Preparation of (R)-3-(3-methyl-2-thionemidazolidin-1-yl)piperidine-1-carboxylic acid benzyl ester (Compound 2)

At room temperature, benzyl (R)-3-((2-(methylamino)ethyl)amino)piperidine-1-carboxylate (2.5 g, 8.56 mmol) was dissolved in dichloromethane (30 mL), and triethylamine (8.47 g, 85.6 mmol) and N,N'-thiocarbonyldiimidazole (2.28 g, 12.86 mmol) were added. The reaction was stirred at 25°C for 2 h. After the reaction was complete, the reaction solution was added with water (50 ml) and dichloromethane (50 ml) and extracted three times, washed once with saturated brine, and the organic phase was collected, concentrated, and purified by silica gel column chromatography (dichloromethane/methanol = 20/1) to obtain compound 2 (1.8 g, yellow solid) in a yield of 63%.

LC-MS:m/z[M+H]⁺=334.

### Step 2: Synthesis of (R)-1-methyl-3-(piperidin-3-yl)imidazolidine-2-thione (Compound 3)

Compound 2 (500 mg, 1.49 mmol) was dissolved in 33% hydrobromic acid in acetic acid solution (5 ml) at room temperature. The reaction solution was stirred at 25°C for 2 h. After the reaction was complete, the reaction solution was concentrated to obtain a crude product of compound 3 (300 mg, yellow oil).

LC-MS:m/z[M+H]⁺=200.

### Step 3: Synthesis of (R)-tert-butyl 4-(4-((3-carbamoyl-6-(3-(3-methyl-2-thionemidazolidin-1-yl)piperidin-1-yl)pyrazin-2-yl)amino)phenyl)piperidine-1-carboxylate (Compound 4)

Intermediate K (300 mg, 0.69 mmol) and compound 3 (151 mg, 0.76 mmol) were dissolved in dimethyl sulfoxide (5 ml), and then N,N-diisopropylethylamine (196 mg, 1.52 mmol) was added. The mixture was stirred at 100°C overnight. After the reaction was complete, water (20 ml) and ethyl acetate (10 ml) were added, and extracted three times. The mixture was washed twice with saturated sodium chloride solution. The organic phase was collected and concentrated to dryness. The crude product was separated and purified using a silica gel column (0-80% ethyl acetate in petroleum ether) to obtain compound 4 (250 mg, yellow solid) in a yield of 61%.

LC-MS:m/z[M+H]⁺=595.

The synthesis of Intermediate K follows the method disclosed in WO2021113557.

### Step 4: Synthesis of (R)-5-(3-(3-methyl-2-thionemidazolidin-1-yl)piperidin-1-yl)-3-((4-(piperidin-4-yl)phenyl)amino)pyrazine-2-carboxamide (Compound 5)

Compound 4 (250 mg, 0.42 mmol) was dissolved in a hydrogen chloride-dioxane solution (4 mol/L) (4 ml). The reaction was stirred at 25°C for 2 h. After the reaction was complete, the reaction solution was concentrated to obtain a crude compound 5 (120 mg, yellow oil), which was used directly in the next step.

LC-MS:m/z[M+H]⁺=495.

### Step 5: Preparation of 3-((4-(1-((1-(6-(((S)-2,6-dioxopiperidin-3-yl)carbamoyl)pyridin-3-yl)piperidin-4-yl)methyl)piperidin-4-yl)phenyl)amino)-5-((R)-3-(3-methyl-2-thionemidazolidin-1-yl)piperidin-1-yl)pyrazine-2-carboxamide (Example 7)

Compound 5 (77 mg, 0.23 mmol) and Intermediate L (120 mg, 0.21 mmol) were dissolved in a mixed solution of 1,2-dichloroethane (2 ml) and methanol (0.2 ml). After stirring for 30 min, sodium triacetoxyborohydride (74 mg, 0.35 mmol) was added followed by stirring at room temperature for 2 h. After the reaction was complete, the mixture was concentrated and purified by prep-HPLC to obtain Example 7 (12 mg, yellow solid) in a yield of 6%.

LC-MS:m/z[M+H]⁺=823.

^{¡}HNMR(500MHz,DMSO-d₆) δ11.20(s,1H),10.84(s,1H), 8.69(d,J=8.0Hz,1H), 8.31(d,J=3.0Hz,1H), 7.87-7.76(m,2H), 7.70(s,1H), 7.50(d,J=8.5Hz,2H), 7.40(m,1H),7.33(s,1H), 7.18(d,J=8.5Hz,2H), 4.80-4.68(m,1H), 4.41-4.29(m,3H),3.97-3.91(m,2H),3.68-3.51(m,4H), 3.07(s,3H), 3.05-2.72(m,7H), 2.21-2.15(m,3H), 2.04-1.95(m,3H), 1.94-1.71(m,9H), 2.21-2.15(m,3H), 1.27-1.14(m,2H).

The synthesis of Intermediate L follows the method disclosed in WO2021113557.

### Example 8:

### N-(3-(5-((5-(2S)-4-((1-(4-(2,6-dioxopiperidin-3-yl)-3,5-difluorophenyl)azetidin-3-yl)methyl)-2-methylpiperazin-1-yl)pyridin-2-yl)amino)-1-methyl-6-oxo-1,6-dihydropyridin-3-yl)-5-fluoro-2-methylphenyl)-2,3-difluoro-4-(2-hydroxypropan-2-yl)benzamide

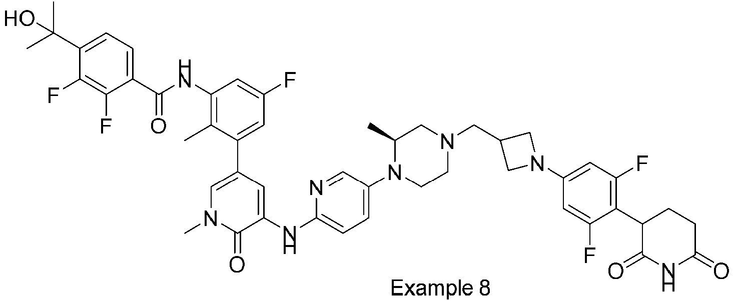

The preparation scheme is shown in the figure below:

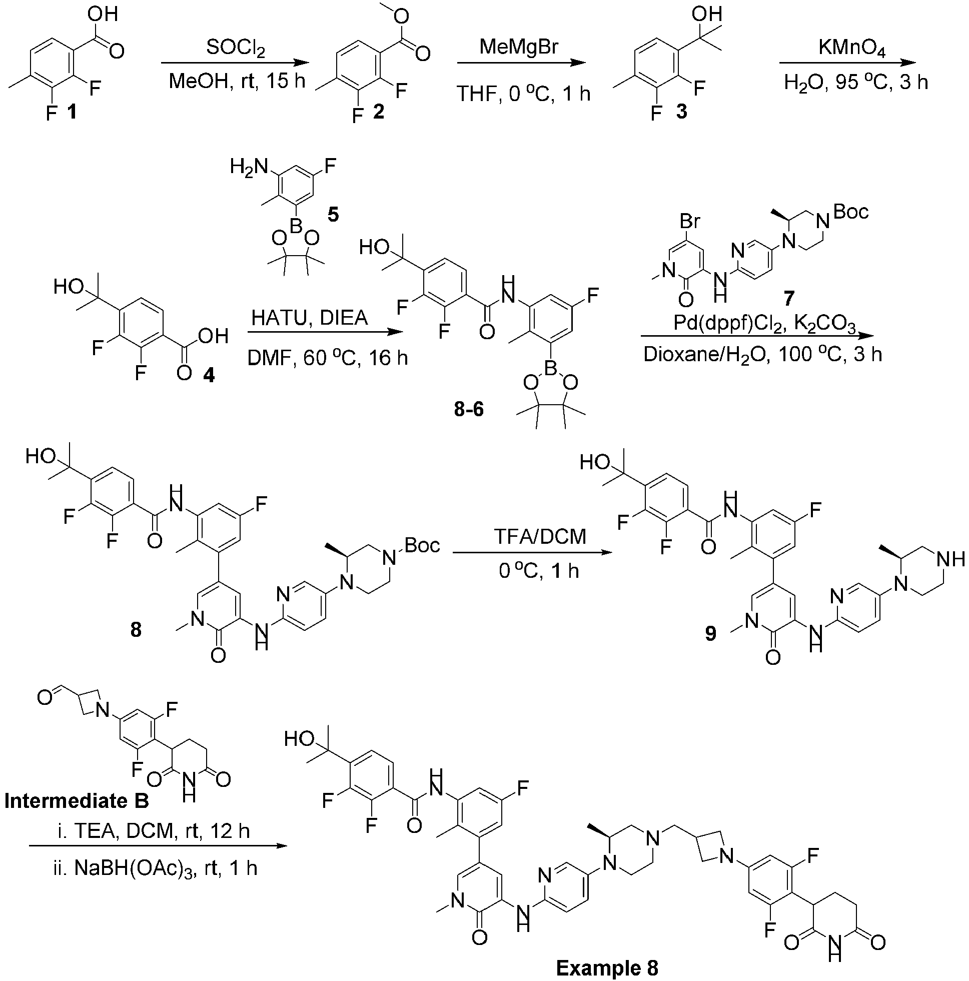

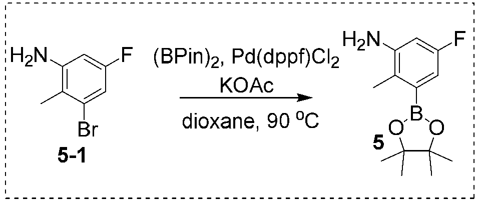

### Step 1: Preparation of 5-fluoro-2-methyl-3-(4,4,5,5-tetramethyl-1,3,2-dioxaborolan-2-yl)aniline (Compound 5)

To a solution of 3-bromo-5-fluoro-2-methylaniline (1.00 g, 4.60 mmol) in 1,4-dioxane (20 mL) were added bis (pinacolato) diboron (1.53 g, 6.00 mmol), potassium acetate (1.35 g, 13.80 mmol), and 1,1-bis(diphenylphosphino)ferrocenepalladium(II) dichloride (336 mg, 0.46 mmol). After nitrogen substitution, the reaction mixture was heated to 100°C and stirred for 3 h. The reaction mixture was evaporated to dryness by rotation, mixed with silica gel, and purified by silica gel column chromatography (ethyl acetate:petroleum ether = 0-10%) to afford compound 5 (1.14 g, yellow solid) in a yield of 99.00%.

LC-MS:m/z[M+H]⁺=251.9.

### Step 2: Preparation of methyl 2,3-difluoro-4-methylbenzoate (Compound 2)

To a solution of 2,3-difluoro-4-methylbenzoic acid (5.00 g, 29.05 mmol) in methanol (50 mL) was added thionyl chloride (4.15 g, 34.86 mmol), and the reaction mixture was stirred at 25°C for 3 h. The reaction solution was evaporated to dryness by rotation, and the mixture was added to water (100 mL) and extracted with methyl tert-butyl ether (100 mL×3). The combined organic phase was washed with saturated aqueous sodium carbonate solution (50 mL×2), then with brine (50 mL×2), dried over anhydrous sodium sulfate, filtered, and concentrated to dryness under reduced pressure to obtain compound 2 (5.1 g, colorless oil) in a yield of 94.4%.

LC-MS:m/z[M+H]⁺=186.8.

### Step 3: Preparation of 2-(2,3-difluoro-4-methylphenyl)propan-2-ol (Compound 3)

To a solution of compound 2 (2.50 g, 13.43 mmol) in tetrahydrofuran (25 mL) was added methylmagnesium bromide (40.29 mL, 40.29 mmol, 1 M in tetrahydrofuran) at 0°C. The reaction mixture was heated to 25°C and stirred for 16 h. The mixture was poured into a saturated aqueous ammonium chloride solution (200 mL) and extracted with ethyl acetate (100 mL×3). The combined organic phase was washed with brine (50 mL×2), dried over anhydrous sodium sulfate, filtered, and concentrated to dryness under reduced pressure to give a crude product of compound 3 (2.50 g, colorless oil).

LC-MS:m/z[M-H₂O+H]⁺=168.8.

### Step 4: Preparation of 2,3-difluoro-4-(2-hydroxyprop-2-yl)benzoic acid (Compound 4)

Potassium permanganate (7.98 g, 50.5 mmol) was added to a solution of compound 3 (2.70 g, 14.4 mmol) in water (33 mL). The reaction mixture was heated to 95°C and stirred for 3 h. The mixture was filtered, and the filtrate was washed with methyl tert-butyl ether (100 mL). The resulting aqueous phase was adjusted to a pH of 2-3 with 2N hydrochloric acid and extracted with ethyl acetate (100 mL×3). The combined organic phases were washed with brine (50 mL×2), dried over anhydrous sodium sulfate, filtered, and concentrated to dryness under reduced pressure to obtain compound 4 (1.1 g, white solid) in a yield of 35.3%.

LC-MS:m/z[M+H]⁺=216.8.

### Step 5: Preparation of 2,3-difluoro-N-(5-fluoro-2-methyl-3-(4,4,5,5-tetramethyl-1,3,2-dioxaborolan-2-yl)phenyl)-4-(2-hydroxypropan-2-yl)benzamide (Compound 8-6)

To a solution of compound 4 (794 mg, 3.67 mmol)/HATU (2090 mg, 5.51 mmol) in N,N-dimethylformamide (10 mL) was added N,N-diisopropylethylamine (1420 mg, 11.01 mmol). The resulting mixture was stirred at 25°C for 1 h, followed by the addition of compound 5 (1110 mg, 1.20 mmol). The resulting mixture was stirred at 60°C for 16 h. The mixture was poured into water (20 mL) and extracted with ethyl acetate (20 mL×3). The combined organic phases were washed with brine (20 mL×2), dried over anhydrous sodium sulfate, filtered, and concentrated to dryness under reduced pressure to obtain a crude product. The crude product was purified by silica gel column chromatography (ethyl acetate:petroleum ether = 0-50%) to obtain compound 8-6 (340 mg, white solid) in a yield of 20.6%.

LC-MS:m/z[M+H]⁺=450.0.

### Step 6: Preparation of (S)-4-(6-((5-(3-(2,3-difluoro-4-( 2-hydroxypropan-2-yl)benzamido)-5-fluoro-2-methylphenyl)-1-methyl-2-oxo-1,2-dihydropyridin-3-yl)amino)pyridin-3-yl-3-methylpiperazine-1-carboxylic acid tert-butyl ester) (Compound 8)

To a solution of compound 8-6 (400 mg, 0.89 mmol), compound 7 (427 mg, 0.89 mmol), and potassium carbonate (369 mg, 2.67 mmol) in dioxane (10 mL) and water (2 mL) was added 1,1-bis(diphenylphosphino)ferrocenepalladium(II) dichloride (66 mg, 0.09 mmol). The mixture was purged with nitrogen three times and stirred at 100°C under a nitrogen atmosphere for 3 h. The reaction mixture was evaporated to dryness by rotation and purified by silica gel column chromatography (petroleum ether:ethyl acetate = 0-50%) to obtain compound 8 (300 mg, white solid) in a yield of 41.6%.

LC-MS:m/z[M+H]⁺=721.4.

The preparation of compound 7 followed the method published in J. Med. Chem. 2020, 63, 5102-5118.

### Step 7: Preparation of (S)-2,3-difluoro-N-(5-fluoro-2-methyl-3-(1-methyl-5-((5-(2-methylpiperazin-1-yl)pyridin-2-yl)amino)-6-oxo-1,6-dihydropyridin-3-yl)phenyl)-4-(2-hydroxypropan-2-yl)benzamide (Compound 9)

To a solution of compound 8 (300 mg, 0.42 mmol) in dichloromethane (3 mL) was added trifluoroacetic acid (1 mL), and the resulting mixture was stirred at 25°C. for 1 h. The reaction mixture was concentrated to dryness under reduced pressure to give crude compound 9 (258 mg, green solid), which was used directly in the next reaction.

LC-MS:m/z[M+H]⁺=621.0.

### Step 8:

### Preparation of N-(3-(5-((5-(2S)-4-((1-(4-(2,6-dioxopiperidin-3-yl)-3,5-difluorophenyl)azetidin-3-yl)methyl)-2-methylpiperazin-1-yl)pyridin-2-yl)amino)-1-methyl-6-oxo-1,6-dihydropyridin-3-yl)-5-fluoro-2-methylphenyl)-2,3-difluoro-4-(2-hydroxypropan-2-yl)benzamide (Example 8)

To compound 9 (60 mg, 0.14 mmol) in dichloromethane (8 mL) were added Intermediate B (54 mg, 0.17 mmol) and triethylamine (72 mg, 0.72 mmol), and the mixture was stirred at 25°C for 16 h. Sodium triacetoxyborohydride (76 mg, 0.36 mmol) was then added to the reaction system, and the mixture was stirred at 25°C for 1 h. The crude product was purified by using a silica gel preparative plate (developing solvent: methanol/dichloromethane = 1/10) to afford Example 8 (27.82 mg, pink solid) in a yield of 21.8%.

LC-MS:m/z[M+H]⁺=913.4.

¹HNMR(400MHz,DMSO-*d*₆) δ10.84(s,1H), 10.07(s,1H), 8.50-8.48(m,1H), 8.43(s,1H), 7.83-7.81(m,1H), 7.58-7.50(m,2H),7.47-7.43(m,1H),7.38-7.34(m,1H),7.26-7.22(m,2H),7.05-7.03(m,1H),6.13-6.07(m,2H),5.56(s,1H),4.00-3.98(m,1H),3.95-3.93 (m,2H),3.63-3.59(m,4H),3.50-3.48(m,2H),3.03(s,1H),2.94-2.90(m,2H),2.77(s,1H),2.69-2.61(m,1H),2.58-2.56(m,2H),2.45(s,2H),2.34-2.30(m,1H),2.20(s,3H),2.05(s,1H),1.97-1.87(m,1H),1.53(s,6H),1.26-1.22(m,1H),0.88-0.92(m,3H).

### Example 9:

### (R)-3-((4-(1-(2-(4-(4-(2,4-dioxytetrahydropyrimidin-1(2H)-yl)-1H-indazol-1-yl)piperidin-1-yl)acetyl)piperidin-4-yl)phenyl)amino)-5-(3-(3-methyl-2-oxoimidazolidin-1-yl)pyridin-1-yl)pyrazine-2-carboxamide

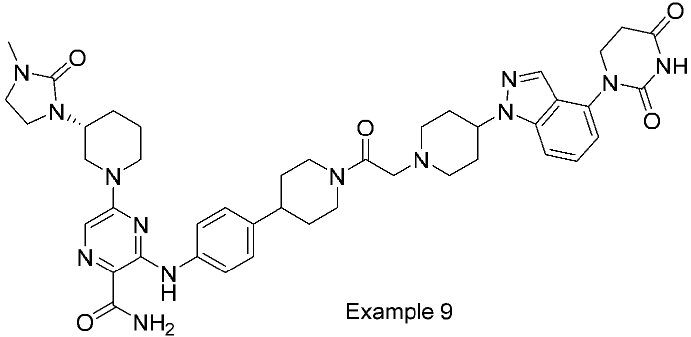

The preparation scheme is shown in the figure below:

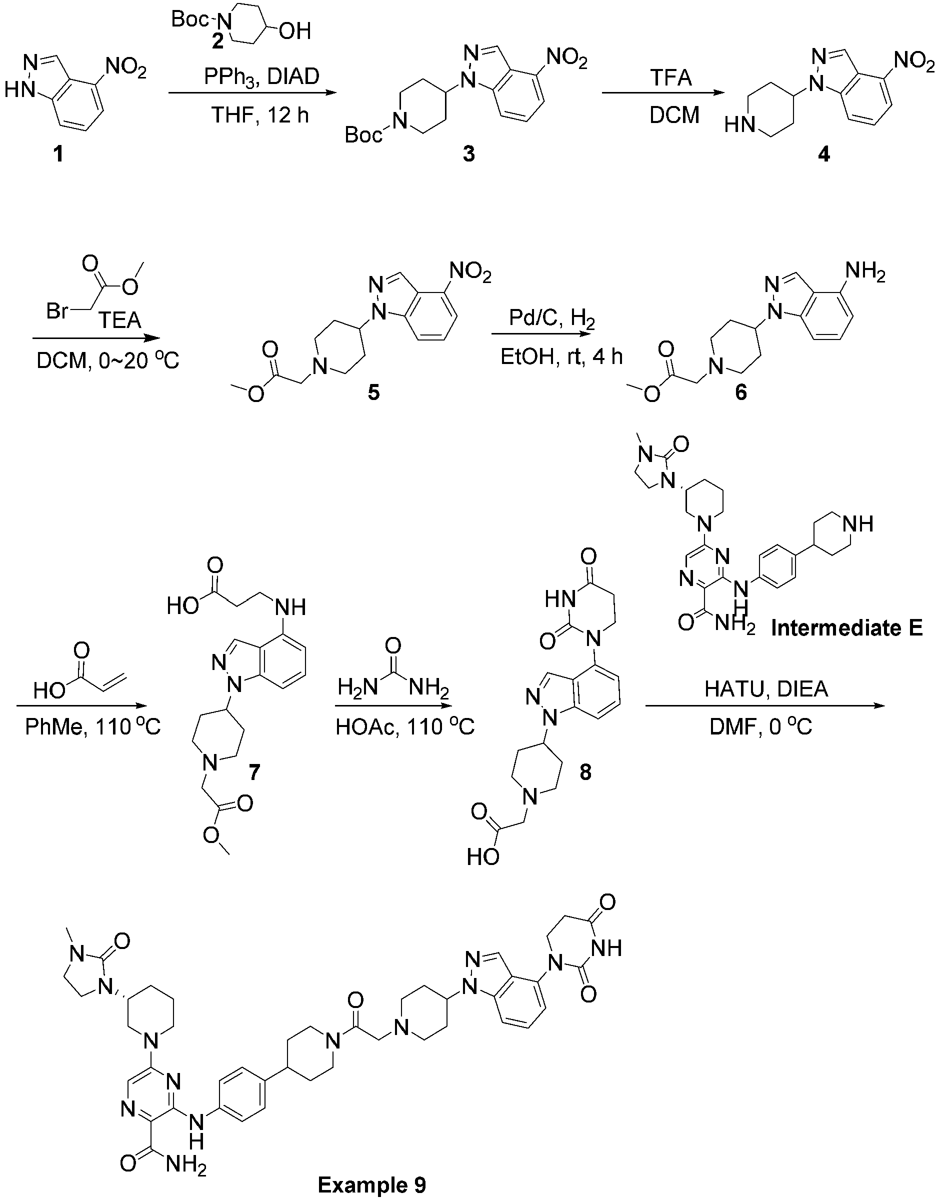

### Step 1: Preparation of tert-butyl 4-(4-nitro-1H-indazol-1-yl)piperidine-1-carboxylate (Compound 3)

To a solution of 4-nitro-1H-indazole (1.5 g, 9.195 mmol) and tert-butyl 4-(methylsulfonyl)oxy)piperidine-1-carboxylate (5137 mg, 18.389 mmol) in N,N-dimethylformamide (15 mL) was added cesium carbonate (8987 mg, 27.584 mmol). The resulting reaction mixture was stirred at 80°C overnight. The reaction mixture was poured into water and extracted with ethyl acetate (60 mL×3). The combined organic phases were washed with saturated brine (60 mL×3), dried over anhydrous sodium sulfate, filtered, and concentrated to dryness under reduced pressure to obtain the crude product. The crude product was purified by silica gel column chromatography (eluent:ethyl acetate/petroleum ether=8%-20%) to afford compound 3 (2700 mg, yellow solid) in an 84.87% yield.

LC-MS:m/z[M-56+H]⁺=290.4

### Step 2: Preparation of 4-nitro-1-(piperidin-4-yl)-1H-indazole (Compound 4)

To a solution of compound 3 (3000 mg, 8.67 mmol) in dichloromethane (27 mL) was added trifluoroacetic acid (10 mL), and the resulting reaction mixture was stirred at room temperature for 1 h. The reaction mixture was concentrated under reduced pressure to dryness to obtain crude compound 4 (2133 mg, yellow oil), which was used directly in the next reaction.

LC-MS:m/z[M+H]⁺=246.8.

### Step 3: Preparation of methyl 2-(4-(4-nitro-1H-indazol-1-yl)piperidin-1-yl)acetate (Compound 5)

To a solution of compound 4 (2133 mg, 8.66 mmol) and triethylamine (2632 mg, 26.01 mmol) in dichloromethane (20 mL) was added methyl 2-bromoacetate (1990 mg, 13.01 mmol) at 0°C, and the resulting reaction mixture was stirred at room temperature overnight. The reaction solution was concentrated to dryness under reduced pressure. The crude product was purified by silica gel column chromatography (eluent: ethyl acetate/petroleum ether=15%-75%) to afford compound 5 (2200 mg, brown oil) in a yield of 79.80%.

LC-MS:m/z[M+H]⁺=351.8.

### Step 4: Preparation of methyl 2-(4-(4-amino-1H-indazol-1-yl)piperidin-1-yl)acetate (Compound 6)

To a solution of compound 5 (700 mg, 2.201 mmol) in ethanol/water (8 mL/2 mL) were added reduced iron powder (1233 mg, 22.013 mmol) and ammonium chloride (1178 mg, 22.013 mmol). The resulting reaction mixture was heated to 80°C and stirred for 1 h. The reaction mixture was filtered, and the filtrate was concentrated to dryness under reduced pressure. The crude product was purified by silica gel column chromatography (eluent: ethyl acetate/petroleum ether=50%-85%) to afford compound 6 (340 mg, green solid) in a yield of 53.57%.

LC-MS:m/z[M+H]⁺=289.0.

### Step 5: Preparation of 3-((1-(1-(2-methoxy-2-oxoethyl)piperidin-4-yl)-1H-indazol-4-yl)amino)propionic acid (Compound 7)

Acrylic acid (255.21 mg, 3.542 mmol) was added to a solution of compound 6 (340 mg, 1.18 mmol) in toluene (6 mL). The resulting reaction mixture was stirred at 110°C under a nitrogen atmosphere for 4 h. The reaction mixture was concentrated to dryness under reduced pressure to obtain crude compound 7 (424.8 mg, yellow oil), which was used directly in the next step.

LC-MS:m/z[M+H]⁺=361.0

### Step 6: Preparation of (4-(4-(2,4-dioxytetrahydropyrimidin-1(2H)-yl)-1H-indazol-1-yl)piperidin-1-yl)acetic acid (Compound 8)

To a solution of compound 7 (424.8 mg, 1.18 mmol) in glacial acetic acid (5 mL) was added urea (212.6 mg, 3.54 mmol). The resulting reaction mixture was stirred at 110°C under a nitrogen atmosphere overnight. Hydrochloric acid (4N, 2 mL) was then added to the reaction mixture, followed by stirring at 100°C for 4 h. The reaction mixture was concentrated under reduced pressure to yield crude compound 8 (438.2 mg, yellow solid) which was used directly in the next reaction.

LC-MS:m/z[M+H]⁺=372.0

### Step 7: Preparation of (R)-3-((4-(1-(2-(4-(4-(2,4-dioxytetrahydropyrimidin-1(2H)-yl)-1H-indazol-1-yl)piperidin-1-yl)acetyl)piperidin-4-yl)phenyl)amino)-5-(3-(3-methyl-2-oxoimidazolidin-1-yl)pyridin-1-yl)pyrazine-2-carboxamide (Example 9)

To a solution of compound 7 (64.68 mg, 0.1743 mmol) and Intermediate E (100 mg, 0.2092 mmol) in N,N-dimethylformamide (1.5 mL) were added N,N-diisopropylethylamine (90.133 mg, 0.6974 mmol) and 2-(7-azobenzotriazole)-N,N,N',N'-tetramethyluronium hexafluorophosphate (99.43 mg, 0.2615 mmol), and the mixture was stirred at room temperature for 3 **h,** and poured into ice water (10 mL). The suspended mixture was filtered and the filter cake was washed with water (2 mL×2). The solid was collected and purified by reverse phase preparative HPLC (acetonitrile:water = 10%-40%) to give Example 9 (15.15 mg, yellow solid) in a yield of 10.45%.

LC-MS:m/z[M+H]⁺=832.4

¹HNMR(400MHz,DMSO-*d₆*) δ 11.20 (d,J=9.6Hz,1H), 10.44(s,1H), 8.01(s,1H), 7.75(s,1H), 7.66(s,2H), 7.51(d,J=7.2Hz,2H), 7.39(m,1H),7.32(s,1H), 7.16(d,J=9.6Hz,2H), 7.04(d,J=7.2Hz,1H), 4.66-4.64(m,1H), 4.52-4.49(m,1H), 4.28-4.23(m,3H), 3.89-3.83(m,2H), 3.59-3.57(m,2H), 3.15-3.11(m,3H), 3.01-2.92(m,5H),2.82-2.73(m,3H),2.72-2.68(m,4H),2.64-2.61(m,1H),2.35-2.28(m,2H),2.17-2.15(m,2H),1.93-1.44(m,11H).

### Example 10: (R)-3-((4-(1-(2-(4-(6-chloro-4-(2,4-dioxytetrahydropyrimidin-1(2H)-yl)-1H-indol-1-yl)piperidin-1-yl)acetyl)piperidin-4-yl)phenyl)amino)-5-(3-(3-methyl-2-oxoimidazolidin-1-yl)pyridin-1-yl)pyrazine-2-carboxamide

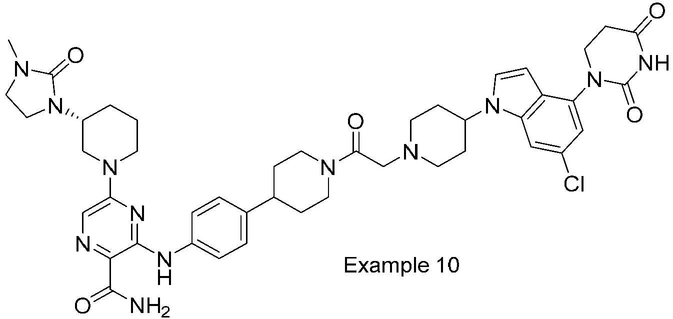

The preparation scheme is shown in the figure below:

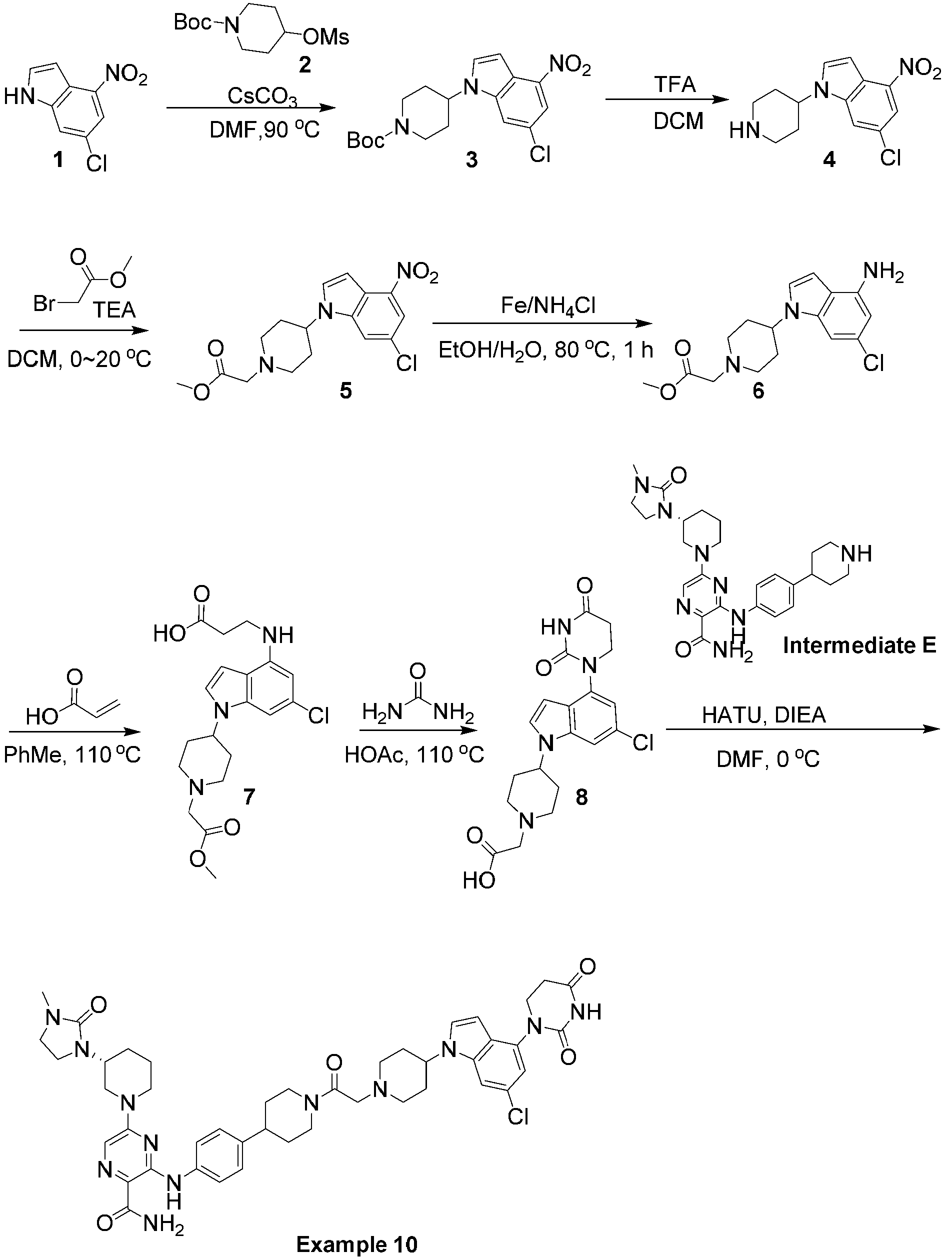

### Step 1: Preparation of tert-butyl 4-(6-chloro-4-nitro-1H-indol-1-yl)piperidine-1-carboxylate (Compound 3)

To a solution of 6-chloro-4-nitro-1H-indole (1.00 g, 2.54 mmol) and tert-butyl 4-(methylsulfonyl)oxy)piperidine-1-carboxylate (1.42 g, 5.08 mmol) in N,N-dimethylformamide (5 mL) was added cesium carbonate (2.48 g, 7.63 mmol), and the resulting reaction mixture was stirred at 90 °C for 16 h. The reaction mixture was poured into water (50 mL) and extracted with ethyl acetate (30 mL×3). The combined organic phase was washed with saturated brine (30 mL×3), dried over anhydrous sodium sulfate, filtered, and concentrated to dryness under reduced pressure. The crude product was purified by silica gel column chromatography (eluent: ethyl acetate/petroleum ether=0-33%) to obtain compound 3 (1.93 g, yellow oil) in a yield of 100%.

LC-MS: m/z [M-56+H]⁺ = 323.8.

### Step 2: Preparation of 6-chloro-4-nitro-1-(piperidin-4-yl)-1H-indole (Compound 4)

Trifluoroacetic acid (7 mL) was added to a solution of compound 3 (1.93 g, 5.08 mmol) in dichloromethane (20 mL), and the resulting reaction solution was stirred at 25°C for 0.5 h. The reaction solution was concentrated to dryness under reduced pressure to obtain crude compound 4 (1.42 g, yellow oil).

LC-MS: m/z[M+H]⁺ =279.8.

### Step 3: Preparation of methyl 2-(4-(6-chloro-4-nitro-1H-indol-1-yl)piperidin-1-yl)acetate (Compound 5)

To a solution of compound 4 (1.42 g, 5.09 mmol) and triethylamine (1.54 g, 15.26 mmol) in dichloromethane (20 mL) was added methyl 2-bromoacetate (1.16 g, 7.63 mmol), and the resulting mixture was stirred at 25°C for 16 h. The reaction solution was concentrated to dryness under reduced pressure, and the crude product was purified by silica gel column chromatography (eluent: ethyl acetate/petroleum ether=0-50% ) to afford compound 5 (1.79 g, red oil) in a yield of 100%.

LC-MS: m/z[M+H]⁺ =351.8.

### Step 4: Preparation of methyl 2-(4-(4-amino-6-chloro-1H-indol-1-yl)piperidin-1-yl)acetate (Compound 6)

Iron powder (2.81 g, 50.31 mmol) and ammonium chloride (2.69 g, 50.31 mmol) were added to a mixture of ethanol/water (16 mL/4 mL) of compound 5 (1.77 g, 5.03 mmol), and the resulting reaction mixture was stirred at 80°C for 1 h under a nitrogen atmosphere. The suspension was filtered through diatomaceous earth and washed with ethanol (20 mL×3). The ethanol was removed by concentration under reduced pressure, and the concentrate was poured into water (50 mL) and extracted with ethyl acetate (30 mL×3). The combined organic phase was washed with saturated brine (30 mL), dried over anhydrous sodium sulfate, filtered, and concentrated to dryness under reduced pressure. The crude product was purified by silica gel column chromatography (eluent: ethyl acetate/petroleum ether=0-50%) to obtain compound 6 (1.40 g, yellow oil) in a yield of 87%.

LC-MS: m/z[M+H]⁺ =321.9.

### Step 5: Preparation of 3-((6-chloro-1-(1-(2-methoxy-2-oxoethyl)piperidin-4-yl)-1H-indol-4-yl)amino)propionic acid (Compound 7)

Acrylic acid (116 mg, 1.61 mmol) was added to a solution of compound 6 (400 mg, 1.24 mmol) in toluene (5 mL) , and the resulting mixture was stirred at 110°C for 8 h. The reaction mixture was concentrated to dryness under reduced pressure to obtain crude compound 7 (489 mg, yellow oil), which was used directly in the next reaction.

LC-MS: m/z[M+H]⁺ =394.0.

### Step 6: Preparation of 2-(4-(6-chloro-4-(2,4-dioxytetrahydropyrimidin-1(2H)-yl)-1H-indol-1-yl)piperidin-1-yl)acetic acid (Compound 8)

To a solution of compound 7 (489 mg, 1.243 mmol) in acetic acid (5 mL) was added urea (224 mg, 3.72 mmol), and the resulting reaction mixture was stirred at 110°C for 16 h. Dilute hydrochloric acid (4N, 4 mL) was added to the reaction solution, and the resulting reaction mixture was further stirred at 100°C for 6 h. The reaction mixture was concentrated to dryness under reduced pressure, and water/ethyl acetate (15 mL/10 mL) was added, stirred, and filtered. The filter cake was dried under reduced pressure to obtain compound 8 (210 mg, gray solid) in a yield of 42%.

LC-MS: m/z[M+H]⁺ =405.0.

### Step 7: Preparation of (R)-3-((4-(1-(2-(4-(6-chloro-4-(2,4-dioxytetrahydropyrimidin-1(2H)-yl)-1H-indol-1-yl)piperidin-1-yl)acetyl)piperidin-4-yl)phenyl)amino)-5-(3-(3-methyl-2-oxoimidazolidin-1-yl)pyridin-1-yl)pyrazine-2-carboxamide (Example 10)

To a solution of compound 8 (60 mg, 0.14 mmol) in N,N-dimethylformamide (2 mL) were added Intermediate E (79 mg, 0.13 mmol), N,N-diisopropylethylamine (51 mg, 0.44 mmol), and HATU (113 mg, 0.29 mmol). The resulting reaction mixture was stirred at 25°C for 1 h. The reaction solution was purified by preparative HPLC to afford Example 10 (45.78 mg, light yellow solid) in a yield of 21%.

LC-MS:m/z[M+H]⁺=865.4.

¹HNMR(400MHz,DMSO-*d₆*) δ11.22(d,J=7.2Hz,1H), 10.40(s,1H),7.76(s,1H), 7.72(s,1H), 7.67(s,1H), 7.59(d,J=2.8Hz,1H), 7.53(d,J=7.6Hz,2H), 7.33(s,1H), 7.19(d,J=8.4Hz,2H), 7.06(d,J=1.6Hz,1H), 6.47(d,J=3.2Hz,1H),4.55-4.23(m,5H),3.82-3.79(m,2H),3.62-3.61(m,1H),3.29-2.88(m,11H),2.78-2.75(m,3H),2.73-2.59(m,4H),2.36-2.34(m,2H),2.07-1.70(m,9H),1.59-1.55(m,2H),1.50-1.36(m,1H).

### Example 11:

### 3-((4-(4-((6-(2,6-dioxopiperidin-3-yl)-5,7-dioxo-3,5,6,7-tetrahydropyrrolo[3,4-f]isoindol-2(1H)-yl)methyl)piperidin-1-yl)phenyl)amino)-5-((R)-3-(3-methyl-2-oxoimidazolidin-1-yl)piperidin-1-yl)pyrazine-2-carboxamide

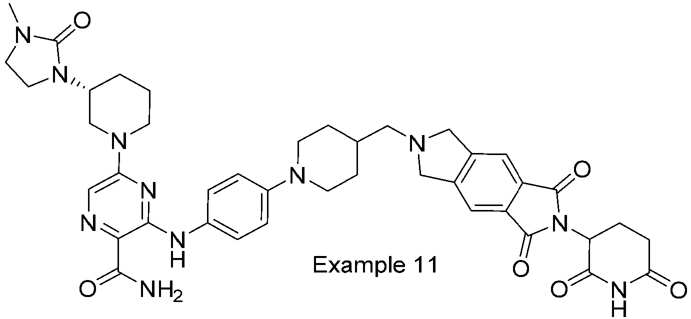

The preparation scheme is shown in the figure below:

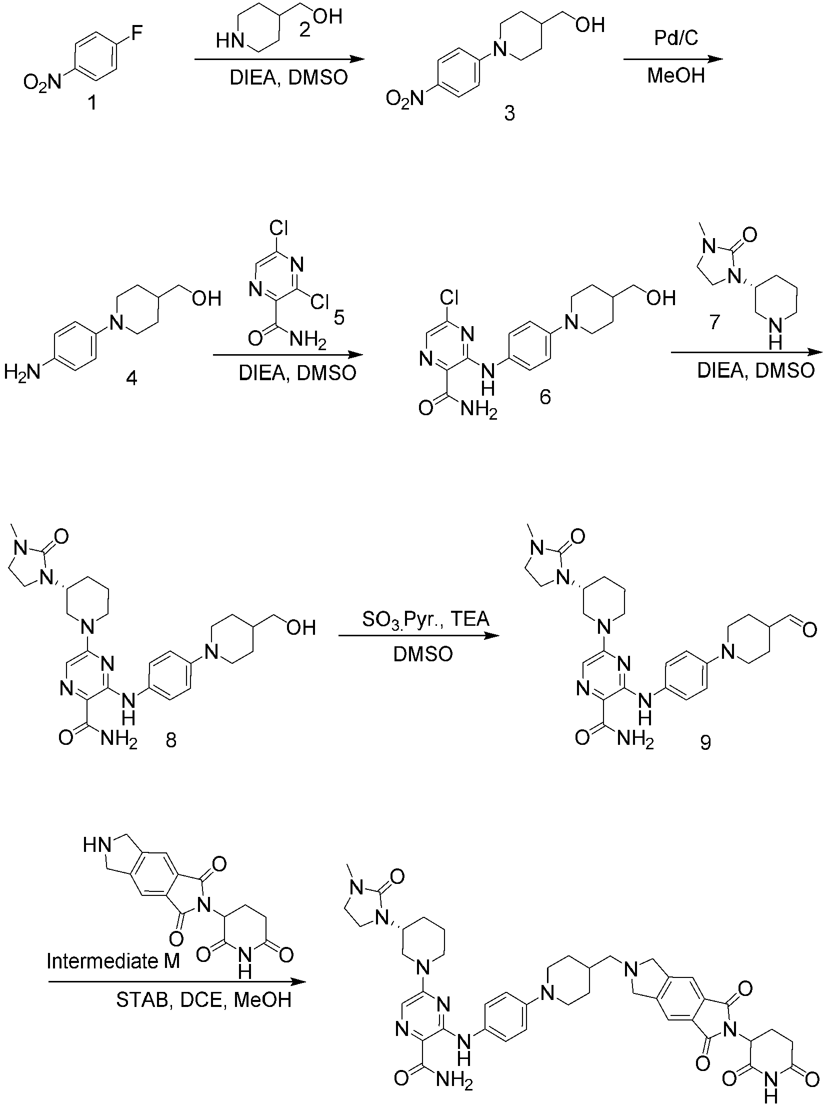

### Example 11

### Step 1: Preparation of [1-(4-nitrophenyl)hexahydropyridin-4-yl]methanol (Compound 3)

To a solution of 4-fluoro-1-nitrobenzene (2000 mg, 14.17 mmol) and hexahydropyridin-4-ylmethanol (1959.12 mg, 17.01 mmol) in dimethyl sulfoxide (20 L) was added N,N-diisopropylethylamine (3.52 mL, 21.26 mmol), and the resulting mixture was stirred at 120°C for 1 h. The mixture was poured into water (300 mL) and filtered, and the filter cake was washed with water (50 mL) and dried under reduced pressure to obtain compound 3 (3345 mg, yellow solid) in a yield of 99.88%.

LC-MS: m/z [M+H]⁺ =237.2

### Step 2: Preparation of [1-(4-aminophenyl)piperidin-4-yl]methanol (Compound 4)

To a suspension of compound 3 (2000 mg, 8.46 mmol) in methanol (30 mL) was added 10% Pd/C (540.5 mg, 5.08 mmol), the resulting mixture was degassed and purged with hydrogen, and stirred at 25°C under hydrogen for 2 h. The suspension was filtered through celite and washed with methanol (30 mL), and the filtrate was concentrated to dryness under reduced pressure to give compound 4 (1730 mg, gray solid) in a yield of 99.07%.

LC-MS: m/z [M+H]⁺ =207.2

### Step 3: Preparation of 5-chloro-3-({4-[4-(hydroxymethyl)hexahydropyridin-1-yl]phenyl}amino)pyrazine-2-carboxamide (Compound 6)

To a solution of 3,5-dichloropyrazine-2-carboxamide (1000 mg, 5.21 mmol) and compound 4 (1074.43 mg, 5.21 mmol) in dimethyl sulfoxide (20 mL) was added N,N-diisopropylethylamine (1.36 mL, 7.81 mmol). The mixture was degassed and purged with nitrogen three times, and stirred at 130°C under a nitrogen atmosphere for 18 h. The mixture was poured into water (300 mL) and extracted with ethyl acetate (200 mL×3). The combined organic phases were washed with brine (50 ml), dried over anhydrous sodium sulfate, filtered, and concentrated to dryness under reduced pressure to obtain the crude product, which was purified by silica gel column chromatography to afford compound 6 (531 mg, red solid) in a yield of 28%.

LC-MS: m/z[M+H]⁺ =362.2.

### Step 4: Preparation of 3-({4-[4-(hydroxymethyl)hexahydropyridin-1-yl]phenyl}amino)-5-[(3R)-3-(3-methyl-2-oxotetrahydro-1H-imidazol-1-yl)hexahydropyrazine-2-carboxamide (Compound 8)

To a solution of compound 6 (531 mg, 0.77 mol) and compound 7 (141.00 mg, 0.77 mmol) in dimethyl sulfoxide (10 ml) was added N,N-diisopropylethylamine (0.32 mL, 1.55 mmol). The mixture was poured into water (80 mL) and filtered, and the filter cake was washed with water (20 mL) and dried under reduced pressure to obtain compound 8 (170 mg, yellow solid) in a yield of 27.10%.

LC-MS: m/z [M+H]⁺ =509.

Compound 7 was prepared following the method disclosed in patent WO2021113557.

### Step 5: Preparation of 3-{[4-(4-formylpiperidin-1-yl)phenyl]amino}-5-[(3R)-3-(3-methyl-2-oxotetrahydro-1H-imidazol-1-yl)piperidin-1-yl]pyrazine-2-carboxamide (Compound 9)

To a solution of compound 8 (80 mg, 0.16 mmol) and triethylamine (436.05 µL, 3.15 mmol) in dimethyl sulfoxide (3 mL) was added sulfur trioxide pyridine (250.34 mg, 1.57 mmol), and the reaction mixture was heated to 25 °C and stirred for 2 h. The reaction mixture was quenched with water (30 mL) and extracted with ethyl acetate (30 mL × 3). The combined organic phases were washed with brine (10 mL), dried over anhydrous sodium sulfate, filtered and concentrated to dryness under reduced pressure to give a crude product of compound 9 (79 mg, yellow solid), which was used directly in the next step of synthesis.

LC-MS: m/z[M+H]⁺ =507.

### Step 6: Preparation of 3-((4-(4-((6-(2,6-dioxopiperidin-3-yl)-5,7-dioxo-3,5,6,7-tetrahydropyrrolo[3,4-f]isoindol-2(1H)-yl)methyl)piperidin-1-yl)phenyl)amino)-5-((R)-3-(3-methyl-2-oxoimidazolidin-1-yl)piperidin-1-yl)pyrazine-2-carboxamide (Example 11)

A mixture of compound 9 (58 mg, 0.11 mmol) and Intermediate M (30.84 mg, 0.10 mmol) in 1,2-dichloroethane (5 mL) and MeOH (0.5 mL) was stirred at 25°C for 0.5 h, and then sodium triacetoxyborohydride (48.53 mg, 0.23 mmol) was added and stirred at 25°C for 2 h. Saturated sodium bicarbonate (30 mL) was added to the reaction mixture and extracted with dichloromethane: methanol = 10: 1 (50 mL×3). The combined organic phase was washed with brine (20 mL) and dried over anhydrous sodium sulfate to give a crude product. The crude product was purified by preparative HPLC to give Example 11 (31.6 mg, yellow solid) in a yield of 34.94%.

LC-MS:m/z[M+H]⁺=790.5.

¹HNMR(400MHz,DMSO-*d₆*)δ11.11(s,1H),10.87-10.78(m,1H), 7.80(s,2H), 7.67(s,1H), 7.56(s,1H), 7.36(d,J=8.8Hz,2H), 7.23(s,1H), 6.48(d,J=8.8Hz,2H),5.13(m,1H),4.42-4.18(m,2H), 3.99(s,4H), 3.60(s,1H), 3.43(d,J=8.2Hz,1H), 3.21(d,J=8.2Hz,2H),3.04-2.83(m,5H),2.81-2.60(m,6H),2.22-1.94(m,5H), 1.83-1.41(m,9H).

The preparation of Intermediate M follows the method published in J. Med. Chem. 2023, 66, 17, 12559-12585.

### Example 12:

### 3-((4-(1-(1-(4-(2,6-dioxopiperidin-3-yl)-3,5-difluorophenyl)azetidin-3-yl)piperidin-4-yl)phenyl)amino)-5-((R)-3-(3-methyl-2-oxoimidazolidin-1-yl)piperidin-1-yl)pyrazine-2-carboxamide

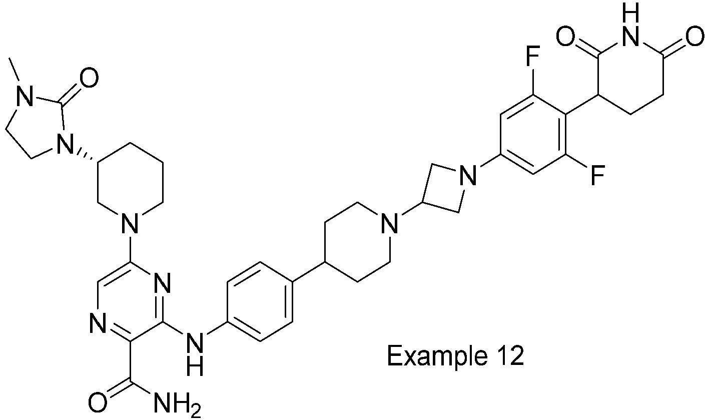

The preparation scheme is shown in the figure below:

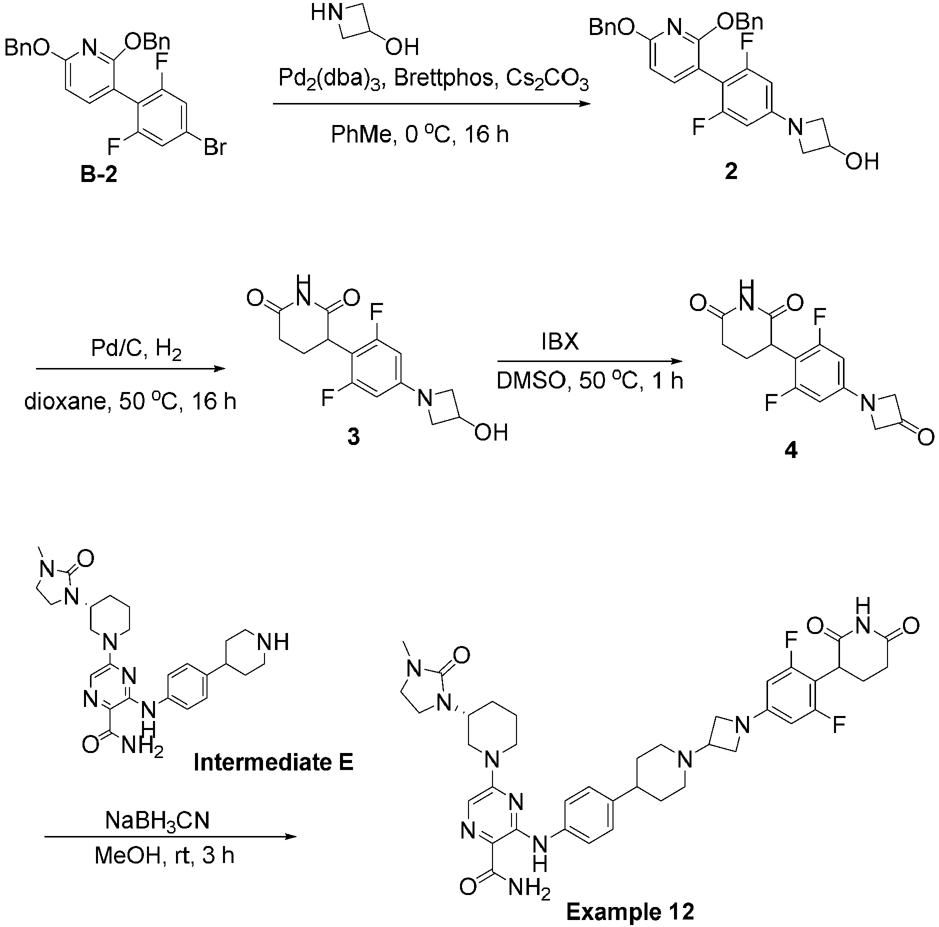

### Step 1: Preparation of 1-(4-(2,6-bis(benzyloxy)pyridin-3-yl)-3,5-difluorophenyl)azetidin-3-ol (Compound 2)

To a solution of compound B-2 (420 mg, 0.873 mmol) and azetidine-3-ol (143.4 mg, 1.31 mmol) in dioxane (10 mL) was added -(dicyclohexylphosphine)-3,6-dimethoxy-2'-4'-6'-tri-I-propyl-11'-biphenyl (111.6 mg, 0.208 mmol), tris(dibenzylidene-acetone)dipalladium (79.8 mg, 0.087 mmol) and cesium carbonate (567.5 mg, 1.75 mmol). The resulting reaction mixture was purged with nitrogen three times and stirred at 100°C under a nitrogen atmosphere for 16 h. The mixture was poured into an aqueous solution (20 mL) and extracted with ethyl acetate (30 mLx3). The combined organic phase was washed with saturated brine (30 mL), dried over anhydrous sodium sulfate, filtered, and concentrated to dryness under reduced pressure to give a crude product. The crude product was purified by silica gel column chromatography (eluent: ethyl acetate/petroleum ether= 0-8%) to give compound 2 (230 mg, gray solid) in a yield of 55.5%.

LC-MS: m/z[M+1]⁺ =475.0.

### Step 2: Preparation of 3-(2,6-difluoro-4-(3-hydroxyazetidin-1-yl)phenyl)piperidine-2,6-dione (Compound 3)

To a solution of compound 2 (200 mg, 0.422 mmol) in dioxane (5 mL) were added 10% Pd(OH)₂ /C (70 mg) and 10% Pd/C (70 mg). The reaction mixture was stirred at 50°C under a hydrogen atmosphere for 16 h. The mixture was filtered, and the filtrate was concentrated to dryness under reduced pressure to afford crude compound 3 (120 mg, gray solid), which was used directly in the next reaction.

LC-MS: m/z[M+H]⁺ =296.8.

### Step 3: Preparation of 3-(2,6-difluoro-4-(3-oxoazetidin-1-yl)phenyl)piperidine-2,6-dione (Compound 4)

To a solution of compound 3 (120 mg, 0.405 mmol) in dimethyl sulfoxide (4 mL) was added 2-iodoxybenzoic acid (170 mg, 0.607 mmol), and the reaction mixture was stirred at 50°C for 2 h. The mixture was poured into an aqueous solution (20 mL) and extracted with ethyl acetate (30 mL×3). The combined organic phase was washed with saturated brine (30 mL), dried over anhydrous sodium sulfate, filtered, and concentrated to dryness under reduced pressure to obtain a crude product. The crude product was purified by silica gel column chromatography (eluent: ethyl acetate/petroleum ether=0-50%) to obtain compound 3 (83 mg, brown oil) in a yield of 71.31%.

LC-MS: m/z[M+H]⁺ =294.8.

### Step 4: Preparation of 3-((4-(1-(1-(4-(2,6-dioxopiperidin-3-yl)-3,5-difluorophenyl)azetidin-3-yl)piperidin-4-yl)phenyl)amino)-5-((R)-3-(3-methyl-2-oxoimidazolidin-1-yl)piperidin-1-yl)pyrazine-2-carboxamide (Example 12)

To a solution of compound 3 (83 mg, 0.289 mmol) and Intermediate E (133.3 mg, 0.289 mmol) in dichloromethane (1 mL) and methanol (3 mL) was added triethylamine (36.62 mg, 0.375 mmol) at 0°C and stirred for 5 min. Then sodium cyanoborohydride (35.1 mg, 0.578 mmol) was added and the resulting mixture was stirred at room temperature for 16 h. The reaction mixture was poured into water (20 mL) and extracted with ethyl acetate (30 mL×3). The combined organic phase was washed with saturated brine (30 mL), dried over anhydrous sodium sulfate, filtered, and concentrated to dryness under reduced pressure to give a crude product. The crude product was purified by reverse preparative HPLC to give Example 12 (7.01 mg, white solid) in a yield of 3.28%.

LC-MS:m/z[M+H]⁺=757.4.

¹HNMR(400MHz,DMSO-*d*₆) δ11.19(s,1H), 10.85(s,1H), 7.75(s,1H), 7.66(s,1H), 7.49(d,J=8.8Hz,2H), 7.32(s,1H), 7.16(d,J=8.4Hz,2H), 6.13(d,J=11.2Hz,2H), 4.43-4.20(m,2H), 4.09-3.35(m,1H), 3.98-3.88(m,2H), 3.65-3.55(m,3H), 3.28-3.15(m,6H), 3.09-2.94(m,2H), 2.94-2.81(m,2H), 2.79-2.67(m,1H), 2.70(s,3H), 2.45-2.38(m,1H), 2.14-2.02(m,1H), 1.98-1.88(m,3H), 1.79-1.45(m,5H), 1.66-1.55(m,3H).

### Example 13:

### N-(3-(5-(5-(4-(1-(4-(2,6-dioxopiperidin-3-yl)-3,5-difluorophenyl)azetidin-3-yl)methyl)piperazin-1-yl)pyridin-2-yl)amino)-1-methyl-6-oxo-1,6-dihydropyridin-3-yl)-5-fluoro-2-methylphenyl)-2,3-difluoro-4-(2-hydroxypropan-2-yl)benzamide

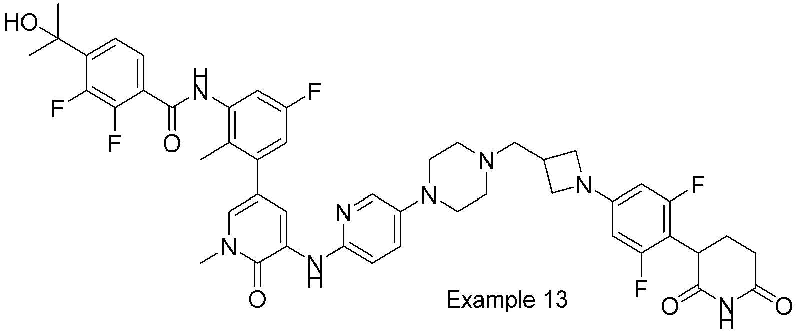

The preparation scheme is shown in the figure below:

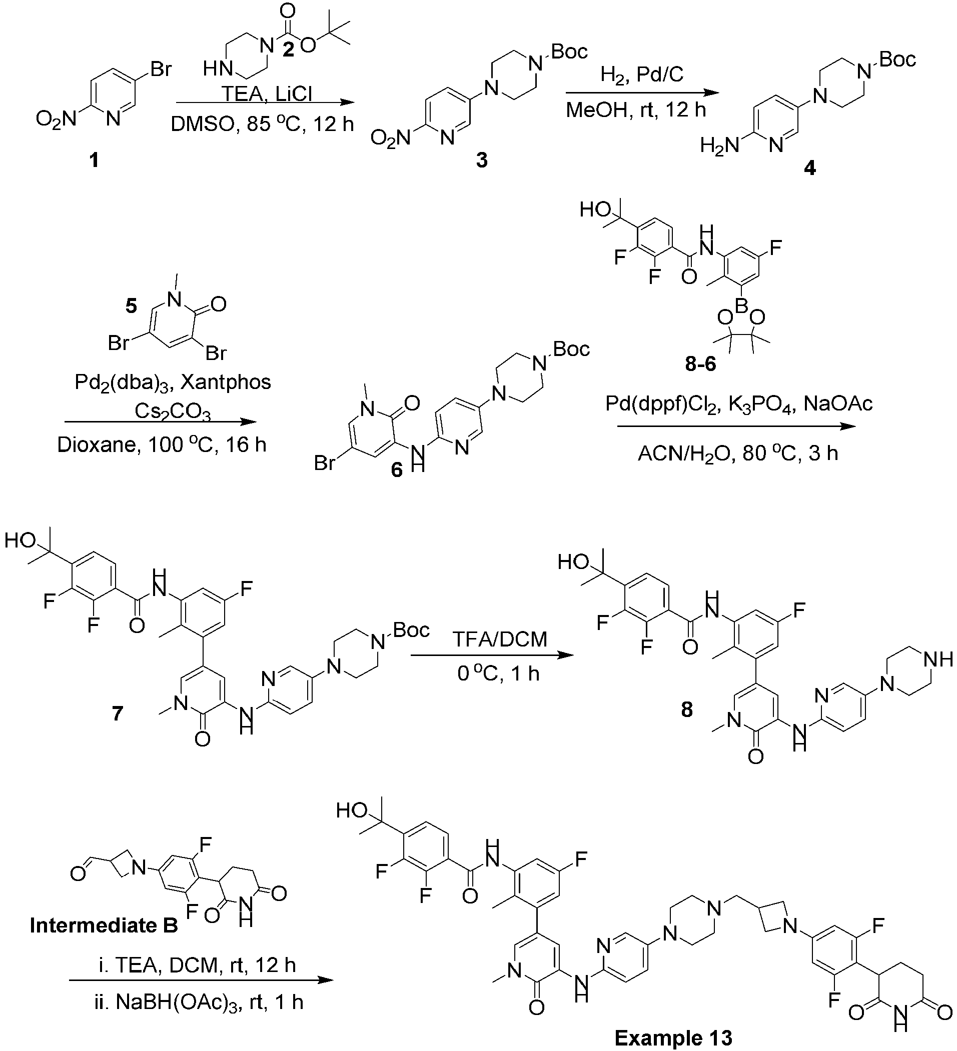

### Step 1: Preparation of tert-butyl 4-(6-nitropyridin-3-yl)piperazine-1-carboxylate (Compound 3)

To a solution of 5-bromo-2-nitropyridine (1.00 g, 4.93 mmol) in dimethyl sulfoxide (10 mL) were added lithium chloride (0.21 g, 4.93 mmol), tert-butyl piperazine-1-carboxylate (1.38 g, 7.39 mmol), and triethylamine (0.75 g, 7.39 mmol). The resulting reaction mixture was stirred at 85°C for 16 h. The reaction solution was poured into water (50 mL) and extracted with ethyl acetate (50 mL×3). The combined organic phases were washed with saturated brine (50 mL×2), dried over anhydrous sodium sulfate, filtered, and the filtrate was concentrated to dryness under reduced pressure. The crude product was purified by silica gel column chromatography (eluent: ethyl acetate/petroleum ether =30%-50%) to obtain compound 3 (1.39 g, yellow solid) in a yield of 91.5%.

LC-MS: m/z[M+H]⁺ =309.0

### Step 2: Preparation of tert-butyl 4-(6-aminopyridin-3-yl)piperazine-1-carboxylate (Compound 4)

To a solution of compound 3 (1.39 g, 4.51 mmol) in tetrahydrofuran (20 mL) and methanol (20 mL) was added 10% Pd/C (139 mg). The resulting mixture was degassed and purged with hydrogen three times, then stirred at 25°C. under hydrogen protection for 16 h. The suspension was filtered, the filter cake was washed with tetrahydrofuran (40 mL×3), and the filtrate was concentrated to dryness under reduced pressure to obtain crude compound 4 (1.2 g, gray solid), which was used directly in the next reaction.

LC-MS: m/z[M+H]⁺ =279.0.

### Step 3: Preparation of tert-butyl 4-(6-(5-bromo-1-methyl-2-oxo-1,2-dihydropyridin-3-yl)amino)pyridin-3-ylpiperazine-1-carboxylate (Compound 6)

To a solution of compound 4 (1.20 g, 4.31 mmol) and 3,5-dibromo-1-methylpyridin-2(1H)-one (1.15 g, 4.31 mmol) in 1,4-dioxane (40 mL) were added cesium carbonate (4.21 g, 12.93 mmol), 4,5-bis(diphenylphosphino)-9,9-dimethylxanthene (0.50 g, 0.86 mmol), and tris(dibenzylideneacetone)dipalladium (0.40 g, 0.43 mmol). The resulting reaction mixture was purged with nitrogen three times and stirred at 100°C under a nitrogen atmosphere for 16 h. The reaction mixture was concentrated to dryness under reduced pressure, and the crude product was purified by silica gel column chromatography (eluent: ethyl acetate/petroleum ether=50%-75%) to afford compound 6 (1.39 g, green solid) in a yield of 69.5%.

LC-MS: m/z[M+H]⁺ =466.0.

### Step 4: Preparation of tert-butyl 4-(6-((5-(3-(2,3-difluoro-4-(2-hydroxypropan-2-yl)benzamido)-5-fluoro-2-methylphenyl)-1-methyl-2-oxo-1,2-dihydropyridin-3-yl)amino)pyridin-3-yl]piperazine-1-carboxylate (Compound 7)

To a suspension of compound 8-6 (230 mg, 0.51 mmol), compound 6 (183 mg, 0.39 mmol), potassium phosphate (167 mg, 0.79 mmol), and sodium acetate (65 mg, 0.79 mmol) in acetonitrile (8 mL) and water (2 mL) was added 1,1'-bis(diphenylphosphino)ferrocenepalladium(II) dichloride (29 mg, 0.04 mmol). The resulting reaction mixture was purged with nitrogen three times and stirred at 100°C under a nitrogen atmosphere for 3 h. The reaction mixture was concentrated to dryness under reduced pressure, and the crude product was purified by silica gel column chromatography (eluent: ethyl acetate/petroleum ether=50%-80%) to afford compound 7 (144 mg, brown solid) in a yield of 51.8%.

LC-MS: m/z[M+H]⁺ = 707.2.

### Step 5: Preparation of 2,3-difluoro-N-(5-fluoro-2-methyl-3-(1-methyl-6-oxo-5-((5-(piperazin-1-yl)pyridin-2-yl)amino)-1,6-dihydropyridin-3-yl)phenyl)-4-(2-hydroxypropan-2-yl)benzamide (Compound 8)

Trifluoroacetic acid (2 mL) was added to a solution of compound 7 (144 mg, 0.20 mmol) in dichloromethane (6 mL), and the resulting mixture was stirred at 25°C for 1 h. The reaction mixture was concentrated under reduced pressure to dryness to obtain crude compound 8 (124 mg, green solid), which was used directly in the next reaction.

LC-MS: m/z[M+H]⁺ = 607.2.

### Step 6: Preparation of N-(3-(5-(5-(4-(1-(4-(2,6-dioxopiperidin-3-yl)-3,5-difluorophenyl)azetidin-3-yl)methyl)piperazin-1-yl)pyridin-2-yl)amino)-1-methyl-6-oxo-1,6-dihydropyridin-3-yl)-5-fluoro-2-methylphenyl)-2,3-difluoro-4-(2-hydroxypropan-2-yl)benzamide (Example 13)

To compound 8 (124 mg, 0.20 mmol) in dichloromethane (10 mL) were added Intermediate B (90 mg, 0.26 mmol) and triethylamine (113 mg, 1.12 mmol). The resulting reaction mixture was stirred at 25°C for 16 h. Sodium triacetoxyborohydride (149 mg, 0.71 mmol) was then added to the reaction system, followed by stirring for 1 h. The reaction mixture was concentrated to dryness under reduced pressure, and the crude product was purified by preparative thin-layer plate (developing solvent: methanol/dichloromethane = 1:10) and then by reverse-phase preparative HPLC to obtain Example 13 (66.76 mg, white solid) in a yield of 37.2%.

LC-MS:m/z[M+H]⁺=899.4.

¹HNMR(400MHz,DMSO-*d*₆) δ10.81(s,1H), 10.04(s,1H), 8.46(d,J=2.4Hz,1H), 8.39(s,1H), 8.10(s,1H), 7.81-7.80(m,1H),7.52-7.47(m,2H),7.41(d,J=8.0Hz,1H),7.35-7.32(m,1H),7.21-7.18(m,2H),7.02-6.99(m,1H),6.09-6.06(m,2H),5.53(s,1H),4.01-3.96(m,1H),3.93-3.89(m,2H),3.56(s,3H),3.47-3.44(m,2H),3.00(m,4H),2.97-2.85(m,1H),2.80-2.68(m,1H),2.63-2.59(m,2H),2.49-2.48(m,2H),2.46-2.44(m,2H),2.16(s,3H),2.05-2.01(m,1H),1.94-1.91(m,1H),1.50(s,6H).

### Example 14:

### 5-(((R)-1-(cyclopropanecarbonyl)piperidin-3-yl)(methyl)amino)-3-((4-(1-((1-(4-(2,6-dioxymethylpiperidin-3-yl)-3,5-difluorophenyl)azetidin-3-yl)methyl)piperidin-4-yl)phenyl)amino)pyrazine-2-carboxamide

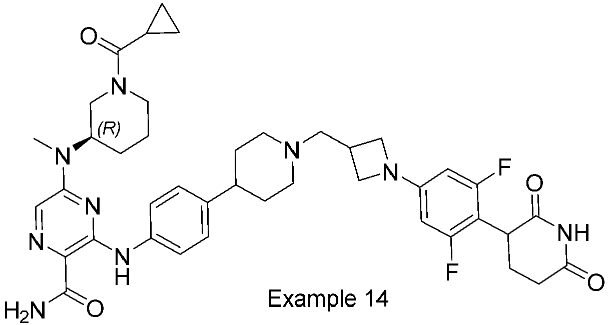

The preparation scheme is shown in the figure below:

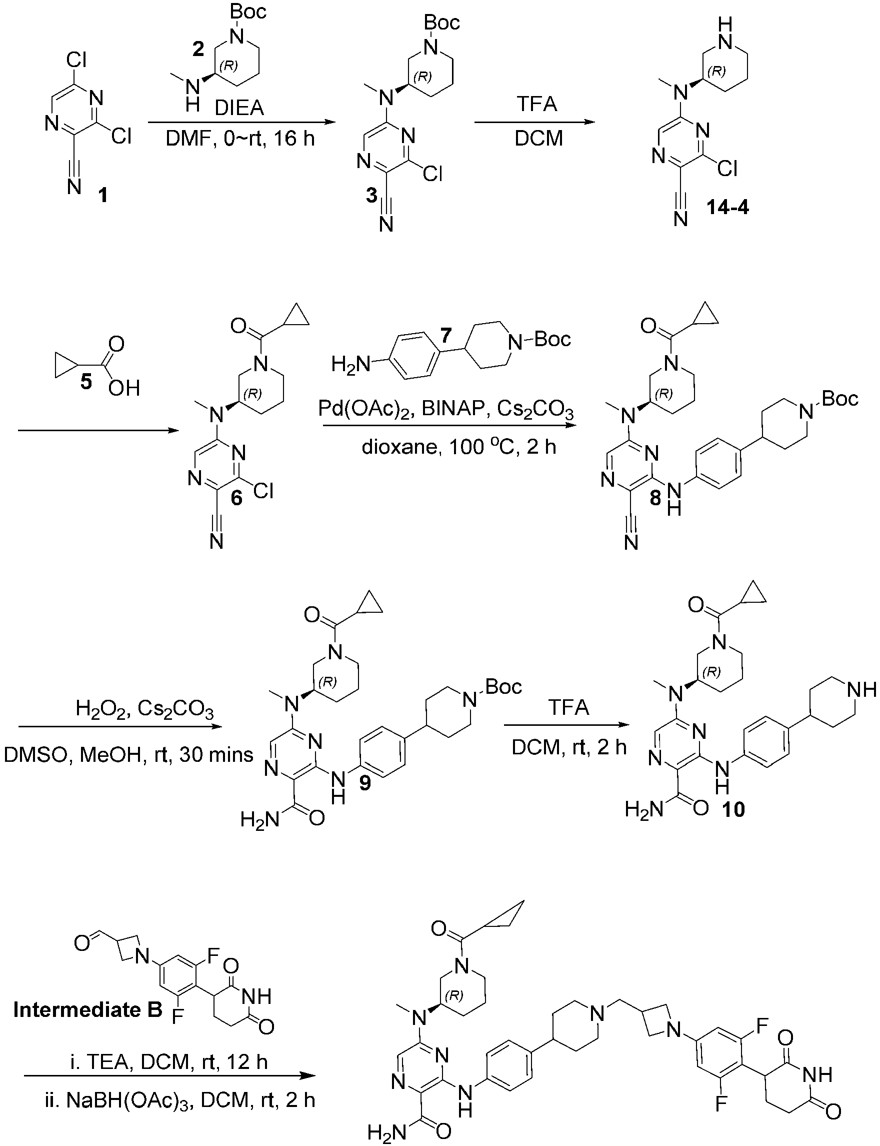

### Example 14

### Step 1: Preparation of tert-butyl (R)-3-((6-chloro-5-cyanopyrazin-2-yl)(methyl)amino)piperidine-1-carboxylate (Compound 3)

To a solution of 3,5-dichloropyrazine-2-carbonitrile (300 mg, 1.72 mmol) and N,N-diisopropylethylamine (445 mg, 3.44 mmol) in N,N-dimethylformamide (3 mL) was added tert-butyl (R)-3-(methylamino)piperidine-1-carboxylate (407 mg, 1.90 mmol). The resulting reaction mixture was stirred at 25°C for 1 h. The reaction mixture was poured into water (30 mL) and extracted with ethyl acetate (30 mL×3). The combined organic phases were washed with saturated brine (10 mL×3), dried over anhydrous sodium sulfate, filtered, and concentrated to dryness under reduced pressure. The crude product was purified by silica gel column chromatography (eluent: ethyl acetate/petroleum ether =12%-50%) to afford compound 3 (600 mg, brown oil).

LC-MS: m/z[M-56+H]⁺ =295.8.

### Step 2: Preparation of (R)-3-chloro-5-(methyl(piperidin-3-yl)amino)pyrazine-2-carbonitrile (Compound 14-4)

To a solution of compound 3 (600 mg, 1.70 mmol) in dichloromethane (12 mL) was added trifluoroacetic acid (4 mL), and the resulting reaction mixture was stirred at 25°C for 1 h. The reaction solution was concentrated to dryness under reduced pressure to obtain crude compound 14-4 (429 mg, brown oil), which was used directly in the next reaction.

LC-MS: m/z[M+H]⁺ =251.8.

### Step 3: Preparation of (R)-3-chloro-5-((1-(cyclopropanecarbonyl)piperidin-3-yl)(methyl)amino)pyrazine-2-carbonitrile (Compound 6)

To a solution of compound 14-4 (376 mg, 1.49 mmol) in N,N-dimethylformamide (5 mL) were added cyclopropylcarboxylic acid (154 mg, 1.79 mmol), N,N,N',N'-tetramethylchloroformamidine hexafluorophosphate (836 mg, 2.98 mmol), and N-methylimidazole (367 mg, 4.47 mmol). The reaction mixture was stirred at 25°C for 1 h. The reaction mixture was poured into water (20 mL) and extracted with ethyl acetate (50 mL×3). The combined organic phases were dried over anhydrous sodium sulfate, filtered, and concentrated to dryness under reduced pressure to obtain a crude product. The crude product was purified by silica gel column chromatography (eluent: ethyl acetate/petroleum ether=25%-50%) to obtain compound 6 (400 mg, colorless oil) in a yield of 83.9%.

LC-MS: m/z[M+H^{]+} =320.0.

### Step 4: Preparation of (R)-tert-butyl 4-(4-(3-cyano-6-((1-(cyclopropanecarbonyl)piperidin-3-yl)(methyl)amino)pyrazin-2-yl)amino)phenyl)piperidine-1-carboxylate (Compound 8)

To a solution of compound 6 (200 mg, 0.63 mmol), compound 7 (190 mg, 0.69 mmol), and cesium carbonate (611 mg, 1.88 mmol) in 1,4-dioxane (10 ml) were added palladium acetate (14 mg, 0.06 mmol) and 1,1'-binaphthyl-2,2'-bisdiphenylphosphine (78 mg, 0.13 mmol). The resulting reaction mixture was purged with nitrogen three times and stirred at 100°C under a nitrogen atmosphere for 16 h. The reaction mixture was concentrated to dryness under reduced pressure, and the crude product was purified by silica gel column chromatography (eluent: ethyl acetate/petroleum ether=25%-80%) to obtain compound 8 (200 mg, yellow solid) in a yield of 57.1%.

LC-MS: m/z[M-Boc+H]⁺ =460.0.

Compound 7 was prepared following the method disclosed in WO2021113557.

### Step 5: Preparation of (R)-tert-butyl 4-(4-(3-carbonyl-6-((1-(cyclopropanecarbonyl)piperidin-3-yl)(methyl)amino)pyrazin-2-yl)amino)phenyl)piperidine-1-carboxylate (Compound 9)

To a solution of compound 8 (200 mg, 0.36 mmol) and cesium carbonate (116 mg, 0.36 mmol) in methanol (4 mL) was added hydrogen peroxide (243 mg, 7.14 mmol), and the resulting mixture was stirred at 25°C for 0.5h. The mixture was poured into water (50 mL) and extracted with ethyl acetate (50 mL×3). The combined organic phase was dried over anhydrous sodium sulfate, filtered, and concentrated to dryness under reduced pressure to obtain a crude product of compound 9 (190 mg, yellow solid), which was used directly in the next reaction.

LC-MS: m/z[M-boc+H]⁺ =478.2.

### Step 6: Preparation of (R)-5-((1-(cyclopropanecarbonyl)piperidin-3-yl)(methyl)amino)-3-((4-(piperidin-4-yl)phenyl)amino)pyrazine-2-carboxamide (Compound 10)

Trifluoroacetic acid (2 mL) was added to a solution of compound 9 (190 mg, 0.33 mmol) in dichloromethane (6 mL), and the resulting reaction solution was stirred at 25°C. for 30 min. The reaction solution was concentrated under reduced pressure to dryness to obtain crude compound 10 (157 mg, brown oil), which was used directly in the next reaction.

LC-MS: m/z[M+H]⁺ =478.2.

### Step 7: Preparation of 5-(((R)-1-(cyclopropanecarbonyl)piperidin-3-yl)(methyl)amino)-3-((4-(1-((1-(4-(2,6-dioxymethylpiperidin-3-yl)-3,5-difluorophenyl)azetidin-3-yl)methyl)piperidin-4-yl)phenyl)amino)pyrazine-2-carboxamide (Example 14)

To a solution of compound 10 (132 mg, 0.23 mmol) in dichloromethane (10 mL) were added Intermediate B (90 mg, 0.29 mmol) and triethylamine (90 mg, 0.90 mmol). The resulting reaction mixture was stirred at room temperature for 16 h. Sodium acetate borohydride (120 mg, 0.56 mmol) was then added to the reaction mixture, followed by stirring for 1 h. The reaction mixture was concentrated under reduced pressure to dryness to obtain a crude product, which was purified by reverse-phase HPLC to afford Example 14 (18.78 mg, white solid) in a yield of 10.7%.

LC-MS:m/z[M+H]⁺=770.4.

¹HNMR(400MHz,DMSO-*d*₆) δ 11.24(s,1H),10.85(s,1H),7.76(s,1H),7.53-7.49(m,3H), 7.33(s,1H), 7.11(d,J=8.4Hz,2H), 6.11(d,J=8.4Hz,2H), 4.58-4.12(m,3H), 4.05-4.00(m,1H), 3.96-3.93(m,2H), 3.51-3.48(m,2H), 3.05(s,3H), 2.98-2.89(m,3H), 2.85-2.70(m,2H), 2.59-2.57(m,2H), 2.42-2.39(m,2H), 2.14-1.99(m,4H), 1.97-1.77(m,5H), 1.74-1.71(m,2H), 1.61(s,3H), 0.77-0.62(m,4H).

### Example 15:

### 5-((((R)-1-(cyclopropanecarbonyl)piperidin-3-yl)amino)-3-((4-((1-(4-(2,6-dioxopiperidin-3-alkyl)-3,5-difluorophenyl)azetidin-3-yl)methyl)piperidin-4-yl)phenyl)amino)pyrazine-2-carboxamide

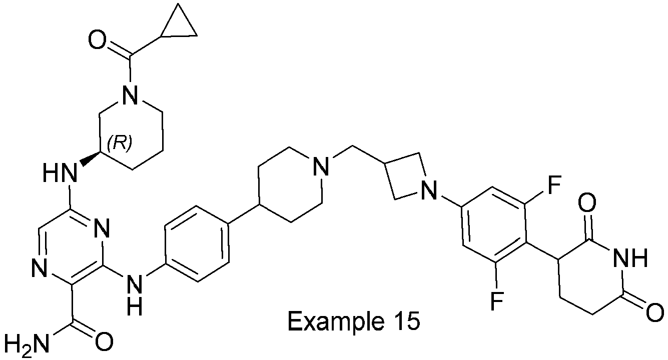

The preparation scheme is shown in the figure below:

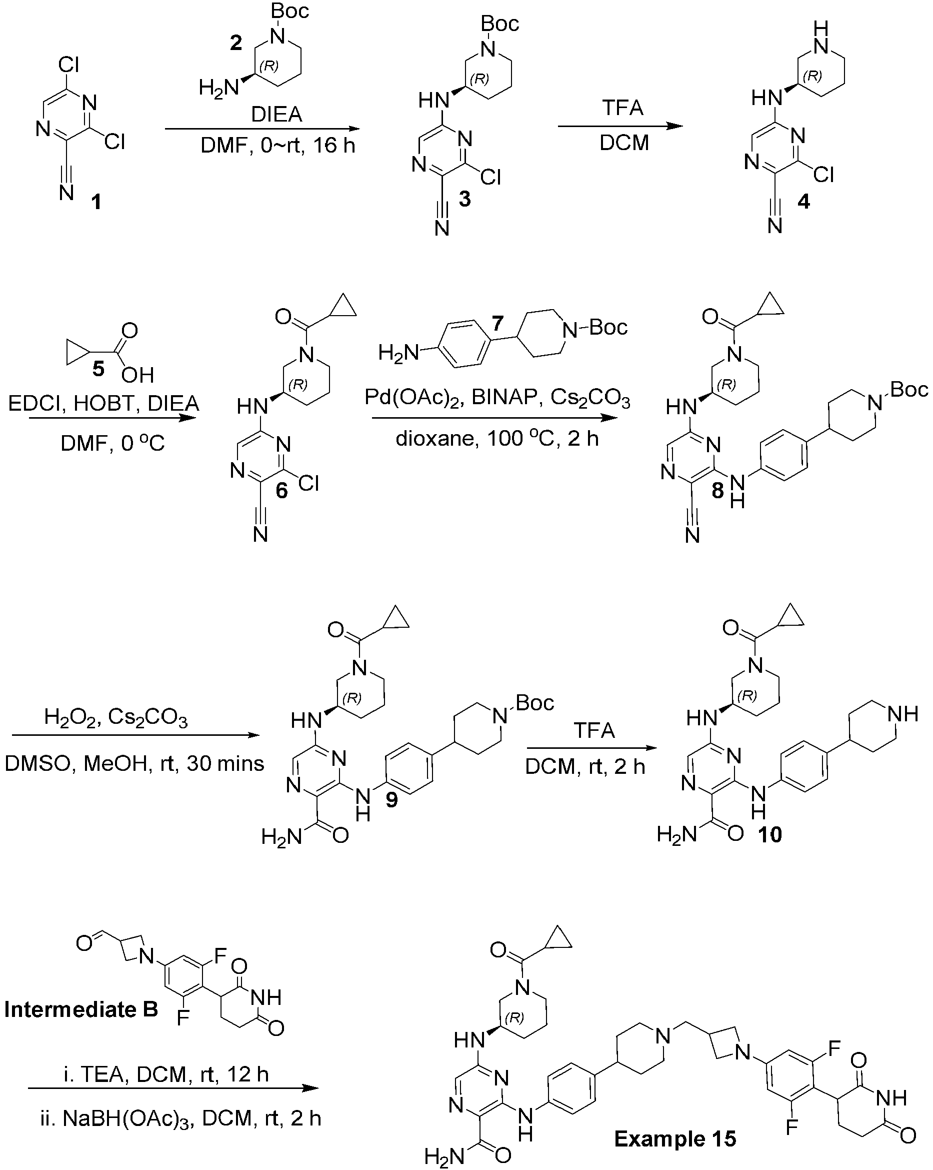

### Step 1: Preparation of tert-butyl (R)-3-((6-chloro-5-cyanopyrazin-2-yl)amino)piperidine-1-carboxylate (Compound 3)

To a solution of 3,5-dichloropyrazine-2-carbonitrile (1.00 g, 5.74 mmol) and N,N-diisopropylethylamine (1.32 g, 11.49 mmol) in N,N-dimethylformamide (10 mL) was added tert-butyl (R)-3-aminopiperidine-1-carboxylate (1.38 g, 6.89 mmol). The resulting reaction mixture was stirred at 0°C for 1 h. After the reaction for completion, the reaction solution was poured into water (50 mL) and extracted with ethyl acetate (30 mL×3). The combined organic phases were washed with saturated brine (20 mL×3), dried over anhydrous sodium sulfate, filtered, and concentrated to dryness under reduced pressure. The crude product was purified by silica gel column chromatography (eluent: ethyl acetate/petroleum ether=15%-33%) to afford compound 3 (1.94 g, yellow oil) in a yield of 100%.

LC-MS: m/z[M-Boc+H]⁺ =237.8.

### Step 2: Preparation of (R)-3-chloro-5-(piperidin-3-ylamino)pyrazine-2-carbonitrile (Compound 4)

Trifluoroacetic acid (4 mL) was added to a solution of compound 3 (1.0 g, 2.96 mmol) in dichloromethane (10 mL), and the resulting reaction solution was stirred at 25°C for 1 h. The reaction solution was concentrated under reduced pressure to dryness to obtain crude compound 4 (704 mg, yellow oil), which was used directly in the next reaction.

LC-MS: m/z[M+H]⁺ =237.8.

### Step 3: Preparation of (R)-3-chloro-5-((1-(cyclopropanecarbonyl)piperidin-3-yl)amino)pyrazine-2-carbonitrile (Compound 6)

To a solution of compound 4 (700 mg, 2.95 mmol) in dichloromethane (20 mL) were added cyclopropylcarboxylic acid (253 mg, 2.94 mmol), N,N-diisopropylethylamine (1.14 g, 8.83 mmol), EDCI (847 mg, 4.42 mmol) and HOBt (596 mg, 4.41 mmol), and the reaction mixture was stirred at 0°C for 0.5 h. The reaction mixture was poured into water (100 mL) and extracted with dichloromethane (60 mL×3). The combined organic phase was dried over anhydrous sodium sulfate, filtered, and concentrated to dryness under reduced pressure to obtain a crude product. The crude product was purified by silica gel column chromatography (eluent: 33%-50%, ethyl acetate/petroleum ether) to obtain compound 6 (940 mg, colorless oily oil) in a yield of 100%.

LC-MS: m/z[M+H]⁺ =305.8.

### Step 4: Preparation of (R)-tert-butyl 4-(4-(3-cyano-6-((1-(cyclopropanecarbonyl)piperidin-3-yl)amino)pyrazin-2-yl)aminophenyl)piperidine-1-carboxylate (Compound 8)

To a mixture of compound 6 (420 mg, 1.37 mmol), compound 7 (418 mg, 1.51 mmol), and cesium carbonate (1.34 g, 4.12 mmol) in 1,4-dioxane (10 ml) were added palladium acetate (31 mg, 0.13 mmol) and BINAP (171 mg, 0.27 mmol). The reaction mixture was purged with nitrogen three times and stirred at 100°C under a nitrogen atmosphere for 2 h. The reaction mixture was concentrated to dryness under reduced pressure, and the crude product was purified by silica gel column chromatography (eluent: ethyl acetate/petroleum ether =33%-100%) to obtain compound 8 (441 mg, yellow oil) in a yield of 59%.

LC-MS: m/z[M-boc+H]⁺ =446.0.

### Step 5: Preparation of (R)-tert-butyl 4-(4-(3-carbamoyl-6-((1-(cyclopropanecarbonyl)piperidin-3-yl)amino)pyrazin-2-yl)amino)phenyl)piperidine-1-carboxylate (Compound 9)

To a solution of compound 8 (441 mg, 0.80 mmol) and cesium carbonate (263 mg, 0.80 mmol) in dimethyl sulfoxide and methanol (2 mL/4 mL) was added hydrogen peroxide (0.3 mL), and the resulting reaction mixture was stirred at 25 °C for 0.5 h. The reaction mixture was poured into water (50 mL) and extracted with ethyl acetate (60 mL×3). The combined organic phase was dried over anhydrous sodium sulfate, filtered, and concentrated to dryness under reduced pressure to obtain a crude product of compound 9 (455 mg, yellow oil), which was used directly in the next reaction.

LC-MS: m/z[M+H]⁺ =564.2.

### Step 6: Preparation of (R)-5-((1-(cyclopropanecarbonyl)piperidin-3-yl)amino)-3-((4-(piperidin-4-yl)phenyl)amino)pyrazine-2-carboxamide (Compound 10)

Trifluoroacetic acid (1.6 mL) was added to a solution of compound 9 (455 mg, 0.80 mmol) in dichloromethane (5 mL), and the resulting reaction solution was stirred at 25°C for 30 min. The reaction solution was concentrated under reduced pressure to dryness to obtain crude compound 10 (370 mg, yellow oil), which was used directly in the next reaction.

LC-MS: m/z[M+H]⁺ =464.2.

### Step 7: Preparation of 5-((((R)-1-(cyclopropanecarbonyl)piperidin-3-yl)amino)-3-((4-((1-(4-(2,6-dioxopiperidine-3-alkyl)-3,5-difluorophenyl)azetidin-3-yl)methyl)piperidin-4-yl)phenyl)amino)pyrazine-2-carboxamide (Example 15)

To a solution of compound 10 (120 mg, 0.251 mmol) in dichloromethane (7 mL) were added Intermediate B (100 mg, 0.325 mmol) and triethylamine (64 mg, 0.627 mmol). The resulting reaction mixture was stirred at room temperature under a nitrogen atmosphere for 16 h. Sodium acetate borohydride (80 mg, 0.373 mmol) was then added to the reaction solution, followed by stirring at 25°C for 2 h. The reaction solution was concentrated to dryness under reduced pressure, and the crude product was purified by preparative HPLC to afford Example 15 (20 mg, white solid) in a yield of 10%.

LC-MS: m/z[M+H]⁺ =756.4.

¹HNMR(400MHz,DMSO-*d₆*)δ11.34(m,1H),10.86(s,1H),7.71-7.54(m,4H), 7.39-7.33(m,1H), 7.24(s,1H), 7.14(s,2H), 6.18-6.10(m,2H), 4.28-4.14(m,1H), 4.09-4.01(m,4H), 3.97-3.86(m,1H), 3.75-3.48(m,6H), 2.96-2.93(m,2H), 2.84-2.69(m,2H), 2.61-2.59(m,2H), 2.43(s,2H), 2.06-2.03(m,4H), 1.99-1.91(m,1H), 1.74-1.72(m,2H), 1.66-1.45(m,4H), 0.78-0.56(m,3H), 0.39(m,1H).

### Example 16:

### 3-((4-(1-(1-(4-(2,6-dioxopiperidin-3-yl)-3,5-difluorophenyl)azetidin-3-yl)methyl)piperidin-4-yl)phenyl)amino)-5-(methyl((R)-1-(methylsulfonyl)piperidin-3-ylamino)pyrazine-2-carboxamide

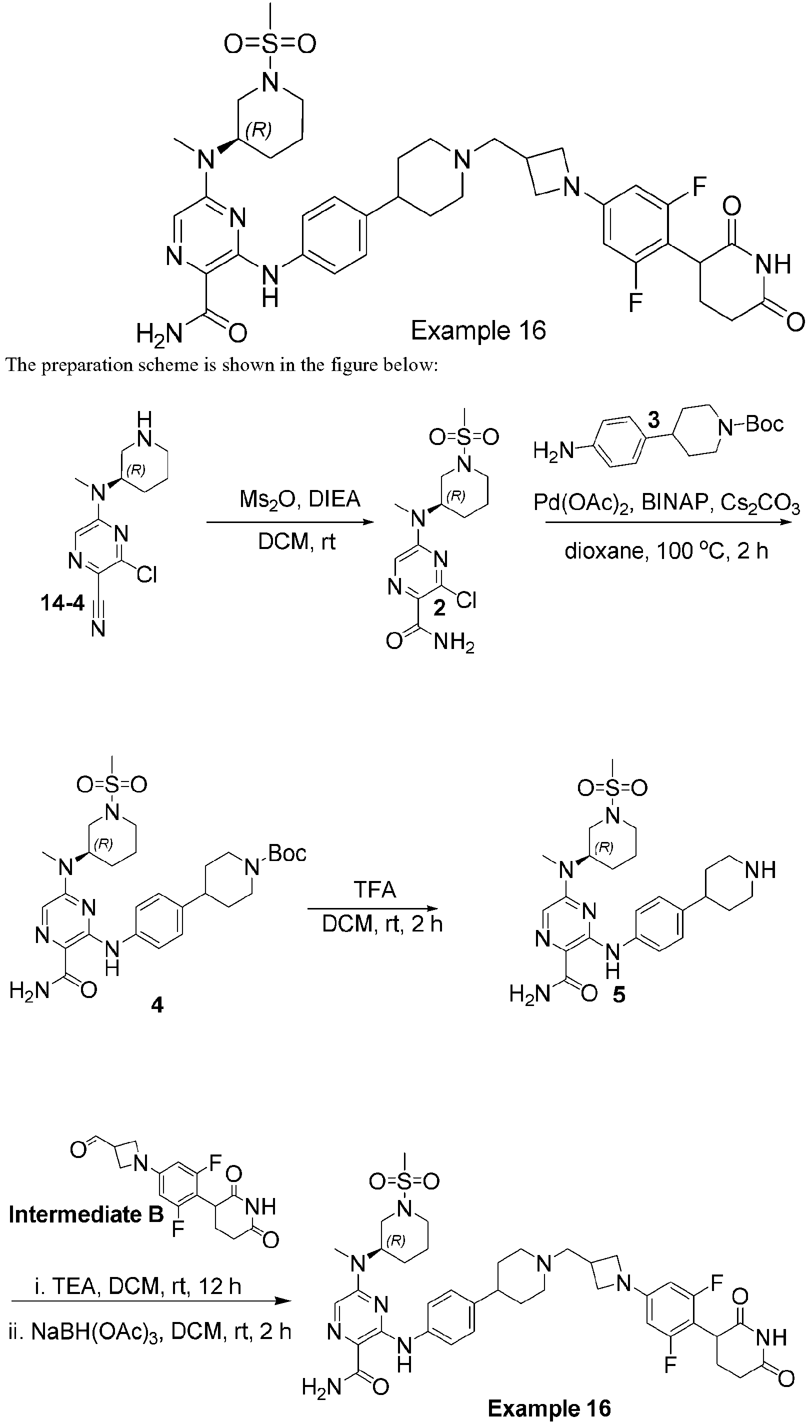

### Step 1: Preparation of (R)-3-chloro-5-(methyl(1-(methylsulfonyl)piperidin-3-yl)amino)pyrazine-2-carbonitrile (Compound 2)

To a solution of compound 14-4 (400 mg, 1.58 mmol) and methanesulfonic anhydride (359 mg, 2.06 mmol) in dichloromethane (10 mL) was added dropwise N,N-diisopropylethylamine (411 mg, 3.17 mmol) at 0°C, and the resulting reaction solution was stirred at 25°C for 1 h. The reaction solution was poured into water (30 mL) and extracted with dichloromethane (20 mL×3). The combined organic phases were dried over anhydrous sodium sulfate, filtered, and concentrated to dryness under reduced pressure to obtain a crude product. The crude product was purified by column chromatography (eluent: ethyl acetate/petroleum ether=20%-50%) to obtain compound 2 (157 mg, yellow oil) in a yield of 28%.

LC-MS: m/z[M+H]⁺ =347.8.

### Step 2: Preparation of (R)-tert-butyl 4-(4-((3-carbamoyl-6-(methyl(1-(methylsulfonyl)piperidin-3-yl)amino)pyrazin-2-yl)amino)phenyl)piperidine-1-carboxylate (Compound 4)

To a solution of compound 2 (157 mg, 0.45 mmol) and compound 3 (137 mg, 0.49 mmol) in 1,4-dioxane (10 mL) were added cesium carbonate (441 mg, 1.35 mmol), 1,1'-binaphthyl-2,2'-bisdiphenylphosphine (56 mg, 0.09 mmol), and palladium acetate (10 mg, 0.04 mmol). The reaction mixture was degassed and purged with nitrogen three times, then stirred at 100°C under a nitrogen atmosphere for 2 h. The reaction mixture was concentrated to dryness under reduced pressure, and the crude product was purified by silica gel column chromatography (eluent: ethyl acetate/petroleum ether=20%-50%) to obtain compound 4 (124 mg, yellow oil) in a yield of 46%.

LC-MS: m/z[M-Boc+H]⁺ =488.0.

### Step 3: Preparation of (R)-5-(methyl(1-(methylsulfonyl)piperidin-3-yl)amino)-3-((4-(piperidin-4-yl)phenyl)amino)pyrazine-2-carboxamide (Compound 5)

Trifluoroacetic acid (1 mL) was added to a solution of compound 4 (124 mg, 0.21 mmol) in dichloromethane (3 mL), and the resulting mixture was stirred at 25°C for 1 h. The reaction solution was concentrated to dryness under reduced pressure to obtain crude compound 5 (102 mg, yellow oil), which was used directly in the next reaction.

LC-MS: m/z[M+H]⁺ =488.0.

### Step 4: Preparation of 3-((4-(1-(1-(4-(2,6-dioxopiperidin-3-yl)-3,5-difluorophenyl)azetidin-3-yl)methyl)piperidin-4-yl)phenyl)amino)-5-(methyl((R)-1-(methylsulfonyl)piperidin-3-ylamino)pyrazine-2-carboxamide (Example 16)

To a solution of compound 5 (85 mg, 0.14 mmol) and Intermediate B (57 mg, 0.18 mmol) in dichloromethane (5 mL) was added triethylamine (36 mg, 0.35 mmol), and the resulting reaction mixture was stirred at 25°C for 12 h. Sodium acetate borohydride (45 mg, 0.21 mmol) was then added, followed by stirring at 25°C for 1 h. The reaction solution was concentrated to dryness under reduced pressure, and the crude product was purified by preparative HPLC to afford Example 16 (32.58 mg, white solid) in a yield of 29%.

LC-MS:m/z[M+H]⁺=780.3.

¹HNMR(400MHz,DMSO-*d*₆) δ10.86(s,1H),8.40 (s, 1H), 8.99(s,1H),7.70(s,1H), 7.41(d,J=8.4Hz,2H), 7.07(d,J=8.4Hz,2H),6.23-5.96(m,2H),4.44(s,1H),4.32-4.17(m,1H),4.05-4.01(m,1H),3.97-3.93(m,2H),3.85-3.83(m,1H),3.51-3.48(m,3H),3.25-3.07(m,2H),3.03-2.92(m,7H),2.83-2.73(m,2H),2.61-2.59(m,2H),2.41-2.28(m,2H),2.07-2.02(m,3H),1.99-1.92(m,3H),1.72-1.69(m,3H),1.60-1.57(m,3H),1.39-1.23(m,1H).

### Example 17: Preparation of 3-{[4-(4-{4-[4-(2,4-dioxohexahydropyrimidin-1-yl)indazol-1-yl]hexahydropyridin-1-yl}hexahydropyridin-1-yl)phenyl]amino}-5-{[(3R)-1-(cyclopropylcarbonyl)hexahydropyridin-3-yl]amino}pyrazine-2-carboxamide

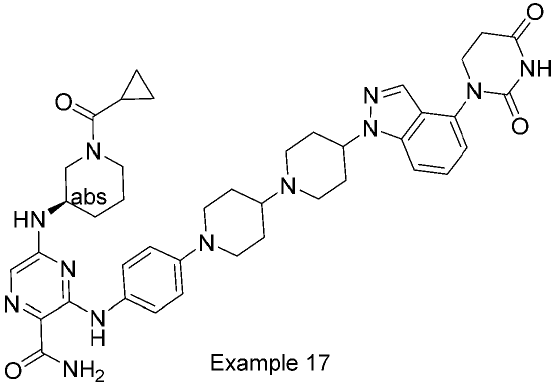

The preparation scheme is shown in the figure below:

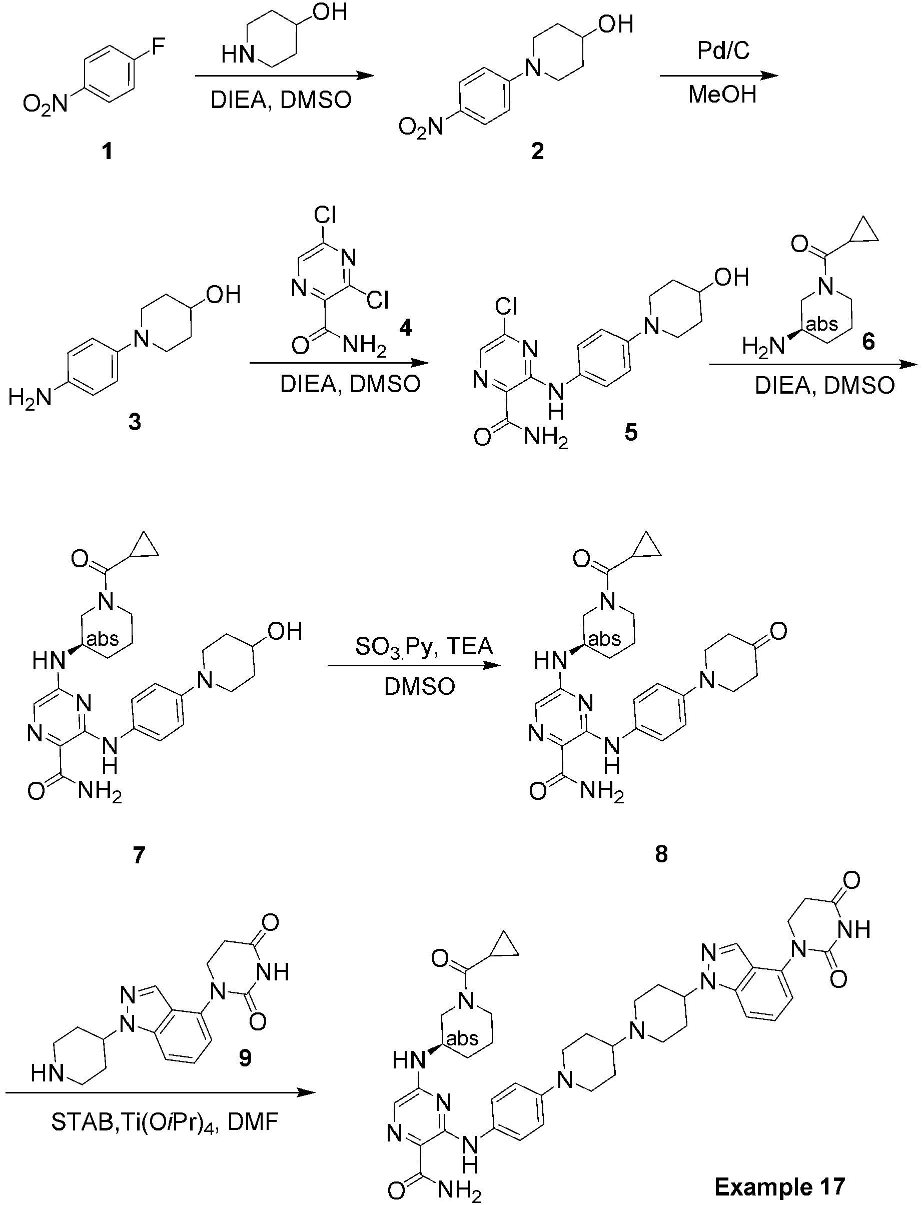

### Step 1: Preparation of 1-(4-aminophenyl)piperidin-4-ol (Compound 2)

To a solution of 4-fluoro-1-nitrobenzene (2000 mg, 14.17 mmol) and hexahydropyridin-4-ol (1719.95 mg, 17.00 mmol) in dimethyl sulfoxide (20 mL) was added N,N-diisopropylethylamine (3.52 mL, 21.26 mmol), and the resulting mixture was stirred at 120°C for 1 h. The mixture was poured into water (300 mL) and filtered, and the filter cake was washed with water (50 mL) and dried under reduced pressure to obtain compound 2 (3080 mg, yellow solid) in a yield of 97.80%.
LC-MS: m/z[M+H]⁺ =223.2

### Step 2: Preparation of 1-(4-aminophenyl)piperidin-4-ol (Compound 3)

To a suspension/mixture of compound 2 (1080 mg, 4.86 mmol) in methanol (30 mL) was added 10% Pd/C (310.30 mg, 2.92 mmol), and the resulting mixture was degassed and purged with hydrogen three times, then stirred at 25°C under hydrogen protection for 2 h. The suspension was filtered through celite and washed with methanol (30 mL), and the filtrate was concentrated to dryness under reduced pressure to give compound 3 (930 mg, gray solid) in a yield of 99.54%.
LC-MS: m/z[M+H]⁺ =193.2

### Step 3: Preparation of 5-chloro-3-{[4-(4-hydroxyhexahydropyridin-1-yl)phenyl]amino}pyrazine-2-carboxamide (Compound 5)

To a solution of compound 4 (1800 mg, 9.38 mmol) and compound 3 (1802.44 mg, 9.38 mmol) in dimethyl sulfoxide (20 mL) was added N,N-diisopropylethylamine (2.46 mL, 14.06 mmol). The mixture was degassed and purged with nitrogen three times, and stirred at 130°C under a nitrogen atmosphere for 18 h. The mixture was poured into water (300 mL) and extracted with ethyl acetate (300 mL×3). The combined organic phases were concentrated to dryness under reduced pressure to obtain a crude product. The crude product was purified by preparative purification to obtain compound 5 (650 mg, red solid) in a yield of 19.93%.
LC-MS: m/z[M+H]⁺ =348.1

### Step 4: Preparation of 3-{[4-(4-hydroxyhexahydropyridin-1-yl)phenyl]amino}-5-{[(3R)-1-(cyclopropylcarbonyl)hexahydropyridin-3-yl]amino}pyrazine-2-carboxamide (Compound 7)

To a solution of compound 5 (1.00 g, 2.88 mmol) and compound 6 in dimethyl sulfoxide (15 mL) was added N,N-diisopropylethylamine (3.01 mL, 17.25 mmol). The mixture was degassed and purged with nitrogen three times, and stirred at 130°C under a nitrogen atmosphere for 18 h. The mixture was poured into water (200 mL) and extracted with ethyl acetate (150 mL×3). The combined organic phases were concentrated to dryness under reduced pressure to obtain a crude product. The crude product was purified by preparative purification to obtain compound 7 (200 mg, yellow solid) in a yield of 14.50%.

LC-MS: m/z [M+H] ⁺ = 480.1.

### Step 5: Preparation of 5-{[(3R)-1-(cyclopropylcarbonyl)hexahydropyridin-3-yl]amino}-3-{[4-(4-oxohexahydropyridin-1-yl)phenyl]amino}pyrazine-2-carboxamide (Compound 8)

To compound 7 (200 mg, 0.42 mmol) in dimethyl sulfoxide (5 mL) were added triethylamine (1.16 mL, 8.34 mmol) and pyridine sulfur trioxide (663.75 mg, 4.17 mmol), and the reaction mixture was stirred at 25°C for 2 h. The reaction mixture was quenched with water (50 mL) and then extracted with ethyl acetate (50 mL×2). The combined organic phase was washed with brine (50 mL×3), dried over anhydrous sodium sulfate, filtered, and concentrated to dryness under reduced pressure to give a crude product of compound 8 (159 mg, yellow solid) in a yield of 79.84%.

LC-MS: m/z[M+H]⁺ =478.2.

### Step 6: Preparation of 3-{[4-(4-{4-[4-(2,4-dioxohexahydropyrimidin-1-yl)indazol-1-yl]hexahydropyridin-1-yl}hexahydropyridin-1-yl)phenyl]amino}-5-{[(3R)-1-(cyclopropylcarbonyl)hexahydropyridin-3-yl]amino}pyrazine-2-carboxamide (Example 17)

To a solution of compound 8 (139 mg, 0.29 mmol) and compound 9 (91.21 mg, 0.29 mmol) in N,N-dimethylformamide (5 mL) was added tetraisopropyl titanate (165.45 mg, 0.58 mmol), and the mixture was stirred at 25°C for 2 h. Then, sodium triacetoxyborohydride (185.06 mg, 0.87 mmol) was added, and the mixture was stirred at 25°C for 1 h.

Water (50 mL) was added to the reaction mixture, followed by extraction with dichloromethane:methanol = 10:1 (50 mL×3). The organic phases were combined and concentrated to dryness under reduced pressure to obtain a crude product. The crude product was purified by preparative purification to obtain Example 17 (28 mg, yellow solid) in a yield of 12.41%.

LC-MS:m/z[M+H]⁺=775.6.

¹HNMR(400MHz,DMSO-*d₆*)δ11.10(s,1H),10.44(s,1H),8.01(s,1H),7.68-7.54(m,3H),7.51-7.23(m,4H),7.15(s,1H),7.04(d,J=7.2Hz,1H),6.91-6.83(m,2H),4.64-4.57(m,1H),4.43-4.33(m,1H),4.12-4.05(m,1H),3.87(t,J=6.8Hz,2H),3.68-3.61(m,6H),3.07(m,2H),2.80-2.58(m,4H),2.47-2.39(m,3H),2.20-1.73(m,8H),1.67-1.34(m,4H),0.87-0.28(m,4H).

### Example 18: (3R)-3-[(5-carbamoyl-6-{[4-(4-{4-[4-(2,4-dioxohexahydropyrimidin-1-yl)indazol-1-yl]hexahydropyridin-1-yl}hexahydropyridin-1-yl)phenyl]amino}pyrazin-2-yl)amino]piperidin-1-carboxylic acid methyl ester

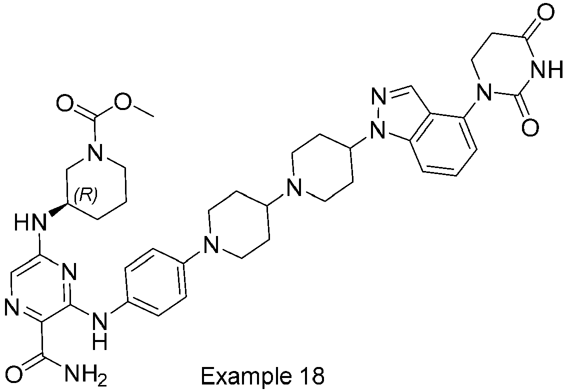

The preparation scheme is shown in the figure below:

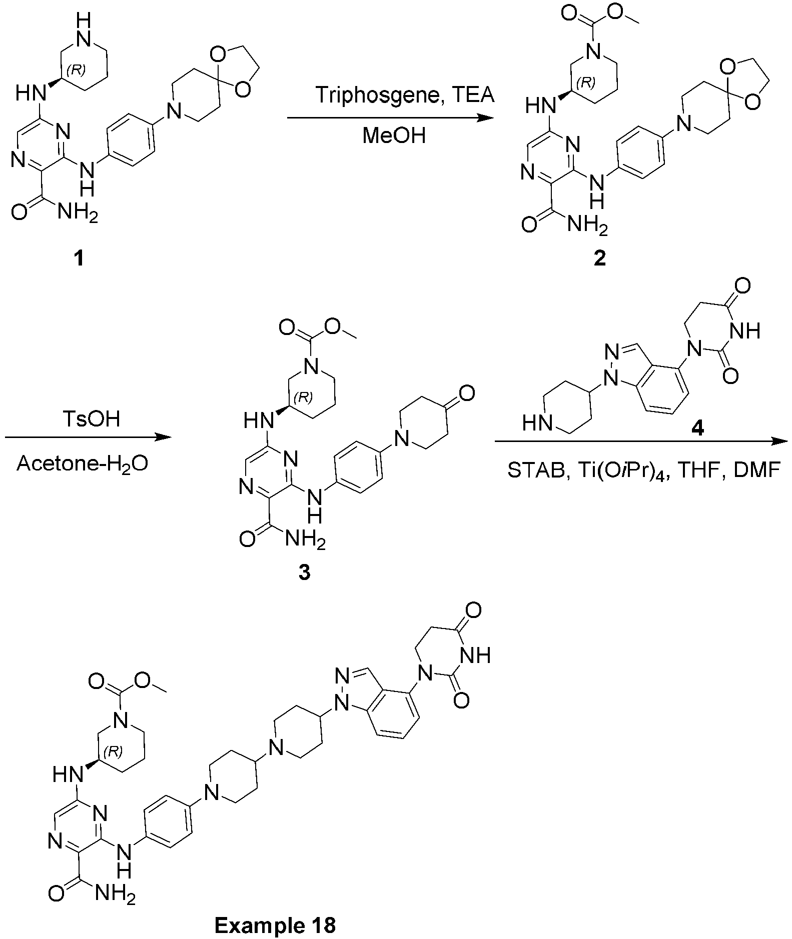

### Step 1: Preparation of (3R)-3-[(6-{[4-(8-aza-1,4-dioxaspiro[4.5]decane-8-yl)phenyl]amino}-5-carbamoylpyrazin-2-yl)amino]piperidine-1-carboxylic acid methyl ester (Compound 2)

To a solution of methanol (268 µL, 6.61 mmol) and triethylamine (1.52 mL, 11.0 mmol) in tetrahydrofuran (30 mL) was added dropwise a solution of triphosgene (654 mg, 2.20 mmol) in tetrahydrofuran (30 mL) at 0°C. The reaction system was stirred at 0°C for 1 h, followed by the addition of a solution of compound 1 (1.0 g, 2.20 mmol) in tetrahydrofuran (30 mL), then the reaction mixture was heated to 25°C and stirred for 2 h. The mixture was purified by silica gel column chromatography (ethyl acetate:dichloromethane = 1:1 to 4:1) to afford compound 2 (780 mg, yellow solid) in a yield of 69.1%.
LC-MS: m/z[M+H]⁺ =512.2

### Step 2: Preparation of methyl (3R)-3-[(5-carbamoyl-6-{[4-(4-oxopiperidin-1-yl)phenyl]amino} pyrazin-2-yl)amino] piperidine-1-carboxylate (Compound 3)

To a mixture of compound 2 (730 mg, 1.43 mmol) in acetone (10 mL) and water (20 mL) was added 4-methylbenzenesulfonic acid (492 mg, 2.86 mmol), and the resulting mixture was stirred at 70°C for 18 h. The reaction mixture was concentrated under reduced pressure to remove acetone. The reaction mixture was poured into water (20 mL) and extracted with dichloromethane: methanol = 10: 1 (50 mL×3). The combined organic phase was washed with brine (20 mL), dried over anhydrous sodium sulfate, filtered, and concentrated to dryness under reduced pressure to obtain a crude product. The crude product was purified by preparative purification to obtain compound 3 (415 mg, yellow solid) in a yield of 62.2%.
LC-MS: m/z[M+H]⁺ =468.7

### Step 3: Preparation of (3R)-3-[(5-carbamoyl-6-{[4-(4-{4-[4-(2,4-dioxohexahydropyrimidin-1-yl)indazol-1-yl]hexahydropyridin-1-yl}hexahydropyridin-1-yl)phenyl]amino}pyrazin-2-yl)amino]piperidin-1-carboxylic acid methyl ester (Example 18)

To a solution of compound 3 (150 mg, 0.32 mmol) and compound 4 (150 mg, 0.48 mmol) in DMF (2 mL) and tetrahydrofuran (10 mL) was added tetraisopropyl titanate (182 mg, 0.64 mmol). After stirring at 70°C for 2 h, sodium triacetoxyborohydride (203 mg, 0.96 mmol) was added, and the resulting mixture was stirred at 25°C for 1 h. The reaction mixture was concentrated under reduced pressure to remove tetrahydrofuran, then poured into water (30 mL) and filtered. The filter cake was slurried with dichloromethane:methanol = 10:1 (100 mL), and the suspension was separated by filtration. The filtrate was concentrated to dryness under reduced pressure to obtain the crude product. The crude product was subjected to preparative purification to obtain Example 18 (18.5 mg, brown solid) in a yield of 7.58%.

LC-MS:m/z[M+H]⁺=765.4
¹HNMR(400MHz,DMSO-*d₆*)δ11.18(s,1H),10.45(s,1H),8.02(s,1H),7.59-7.66(m,3H),7.48(m,2H),7.38(d,J=8.0Hz,1H),7.30(s,1H),7.19(s,1H),7.04(d,J=7.2Hz,1H),6.87(d,J=8.4Hz,2H),4.62(m,1H ),3.81-3.89(m,4H),3.63-3.66(m,5H),3.04-3.07(m,5H),2.78(t,J=6.8Hz,2H),2.63(m,2H),2.41-2.47(m,3H),2.07-2.15(m,2H),1.77-1.95(m,6H),1.48-1.65(m,4H).

### Example 19: (R)-5-((1-(cyclopropylcarbonyl)piperidin-3-yl)(methyl)amino)-3-((4-(4-(2,4-dioxotetrahydropyrimidin-1(2H)-yl)-1H-indazol-1-yl)-[1,4'-bipiperidinyl]-1'-yl)phenyl)amino)pyrazine-2-carboxamide

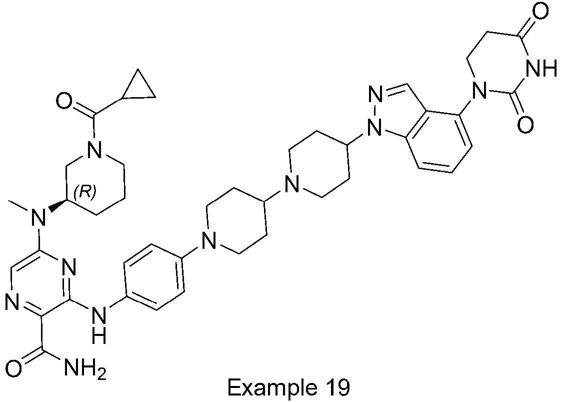

The preparation scheme is shown in the figure below:

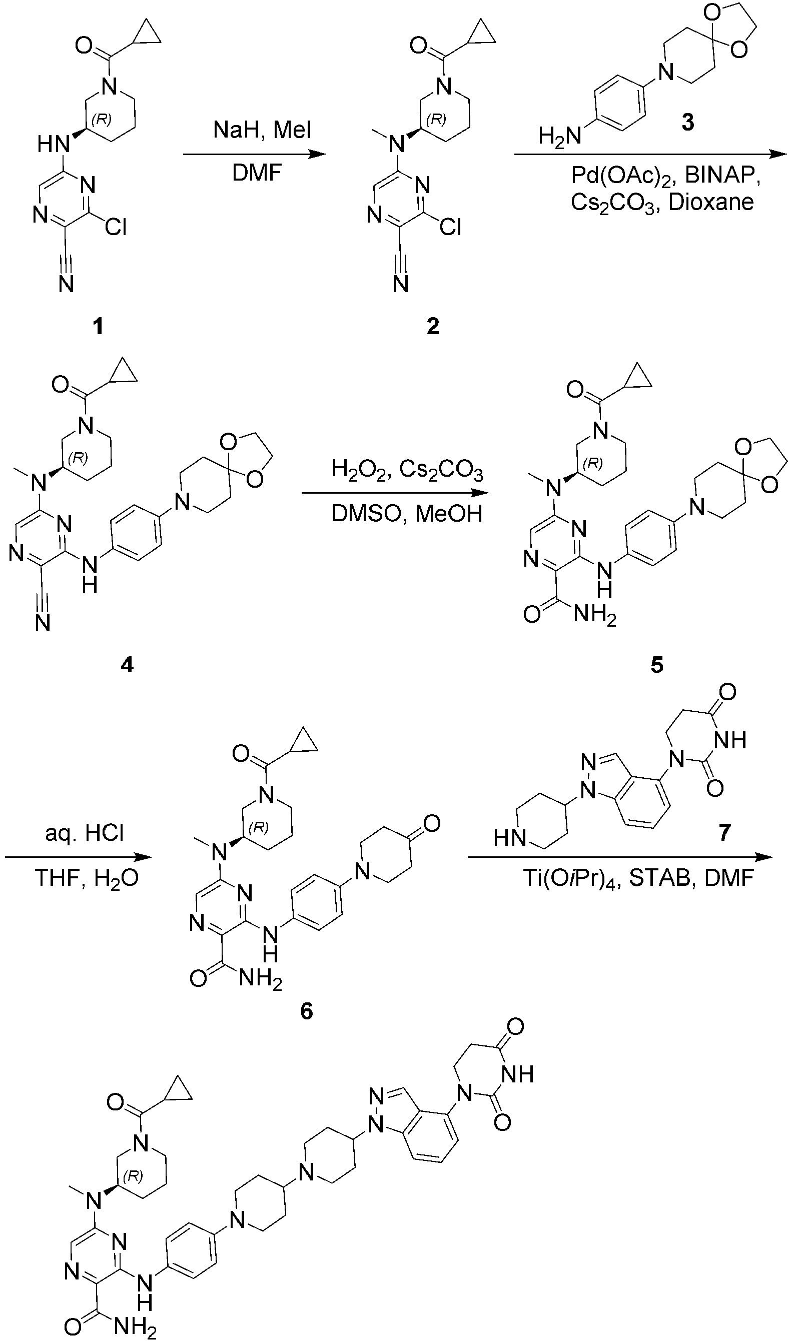

### Example 19

### Step 1: Preparation of (R)-3-chloro-5-((1-(cyclopropylcarbonyl)piperidin-3-yl)(methyl)amino)pyrazine-2-carbonitrile (Compound 2)

Compound 1 (1.2 g, 3.93 mmol) was dissolved in N,N-dimethylformamide (12 mL), and sodium hydride (452 mg, 19.6 mmol) was added at 0°C. After stirring for 30 min, iodomethane (1.5 mL) was added, followed by stirring at room temperature for 1 h. After the reaction was complete, the mixture was poured into water (30 mL) and extracted with ethyl acetate (60 mL). The organic phase was collected, dried over anhydrous sodium sulfate, filtered, concentrated to dryness and purified by silica gel column chromatography (petroleum ether/ethyl acetate = 2:1) to obtain compound 2 (1.2 g, yellow solid) in a yield of 95.6%.
LC-MS: m/z[M+H]⁺ =320.1

### Step 2: Preparation of (R)-3-((4-(1,4-dioxa-8-azaspiro[4.5]decane-8-yl)phenyl)amino)-5-((1-(cyclopropylcarbonyl)piperidin-3-yl)(methyl)amino)pyrazine-2-carbonitrile (Compound 4)

Compound 2 (1.2 g, 3.76 mmol), compound 3 (880 mg, 3.76 mmol), 1,1'-binaphthyl-2,2'-bisdiphenylphosphine (700 mg, 1.13 mmol) and cesium carbonate (2451 mg, 7.52 mmol) were dissolved in 1,4-dioxane (15 mL). Palladium acetate (86 mg, 0.38 mmol) was added to the above mixture, and the atmosphere was purged with nitrogen three times. The mixture was stirred at 100°C overnight. After the reaction was complete, the reaction solution was filtered and concentrated to dryness under reduced pressure. The product was purified by silica gel column chromatography to obtain compound 4 (1200 mg, yellow solid) in a yield of 61.7%.
LC-MS: m/z[M+H]⁺ =518.3

### Step 3: Preparation of (R)-3-((4-(1,4-dioxa-8-azaspiro[4.5]decane-8-yl)phenyl)amino)-5-((1-(cyclopropylcarbonyl)piperidin-3-yl)(methyl)amino)pyrazine-2-carboxamide (Compound 5)

To a solution of compound 4 (1200 mg, 2.32 mmol) and cesium carbonate (756 mg, 2.32 mmol) in dimethyl sulfoxide (2 mL) and methanol (10 mL) was added hydrogen peroxide (394 mg, 11.6 mmol), and the mixture was stirred at 25°C overnight. After the reaction was complete, water (30 mL) was added, and the mixture was filtered. The filter cake was collected, washed with water, and dried by distillation under reduced pressure to obtain crude compound 5 (980 mg, yellow solid) in a yield of 78.8%.
LC-MS: m/z[M+H]⁺ =536.3

### Step 4: Preparation of (R)-5-((1-(cyclopropylcarbonyl)piperidin-3-yl)(methyl)amino)-3-((4-(4-oxopiperidin-1-yl)phenyl)amino)pyrazine-2-carboxamide (Compound 6)

Compound 5 (980 mg, 1.83 mmol) was dissolved in a mixed solution of water (5 mL) and acetonitrile (5 mL). To the above solution was added p-toluenesulfonic acid (944 mg, 5.49 mmol), and the mixture was stirred at 70°C for 5 h. After the reaction was complete, the mixture was concentrated to dryness and purified by reverse phase chromatography to obtain compound 6 (500 mg, yellow solid) in a yield of 55.5%.
LC-MS: m/z [M+H]⁺ = 492.2

### Step 5: Preparation of (R)-5-((1-(cyclopropylcarbonyl)piperidin-3-yl)(methyl)amino)-3-((4-(4-(2,4-dioxotetrahydropyrimidin-1(2H)-yl)-1H-indazol-1-yl)-[1,4'-bipiperidinyl]-1'-yl)phenyl)amino)pyrazine-2-carboxamide (Example 19)

Compound 6 (500 mg, 1.01 mmol) and compound 7 (1.01 mmol, 318 mg) were dissolved in a mixed solution of N,N-dimethylformamide (2 mL) and tetrahydrofuran (10 mL), followed by the addition of tetraisopropyl titanate (456 mg, 2.02 mmol), and the mixture was stirred at 70°C for 1 h. Then, sodium triacetoxyborohydride (426 mg, 2.02 mmol) was added and the mixture was returned to room temperature and stirred for a further 1 h. After the reaction was complete, the reaction solution was poured into water (20 mL) and extracted three times with ethyl acetate (20 mL). The combined organic phases were added with anhydrous sodium sulfate and dried, filtered, and concentrated to dryness to obtain a crude product. The crude product was purified by preparative purification to obtain Example 19 (26 mg, yellow solid) with a yield of 3.1%.

LC-MS:m/z[M+H]⁺=789.4
¹HNMR(400MHz,DMSO-*d₆*) δ11.03(s,1H), 10.45(s,1H), 8.02(s,1H), 7.72(s,1H), 7.66(d,J=8.4Hz,1H), 7.36-7.47(m,4H), 7.28(s,1H), 7.04(m,1H), 6.85(d,J=8.4Hz,2H), 4.62(s,1H), 4.36(s,2H), 3.87(t,J=6.4Hz,3H), 3.61-3.63(m,2H), 3.03(m,5H), 2.78(t,J=6.4Hz,3H), 2.59-2.68(m,4H), 2.40-2.44(m,2H), 2.10-2.13(m,3H), 1.75-1.88(m,6H), 1.45-1.63(m,4H), 0.75-0.80(m,4H).

### Example 20:

### 3-[(4-{1-[(1-{4-[(3S*)-2,6-dioxohexahydropyridin-3-yl]-3,5-difluorophenyl}azetidin-3-yl)methyl]hexahydropyridin-4-yl}phenyl)amino]-5-[(3R)-3-(3-methyl-2-oxotetrahydro-1H-imidazol-1-yl)hexahydropyridin-1-yl]pyrazine-2-carboxamide

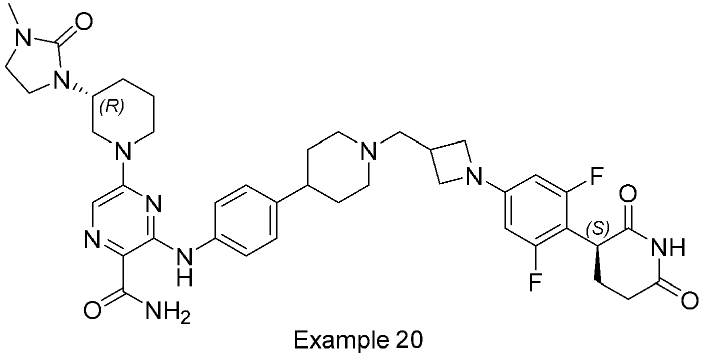

### Example 21:

### 3-[(4-{1-[(1-{4-[(3R*)-2,6-dioxohexahydropyridin-3-yl]-3,5-difluorophenyl}azetidin-3-yl)methyl]hexahydropyridin-4-yl}phenyl)amino]-5-[(3R)-3-(3-methyl-2-oxo-tetrahydro-1H-imidazol-1-yl)hexahydropyridin-1-yl]pyrazine-2-carboxamide

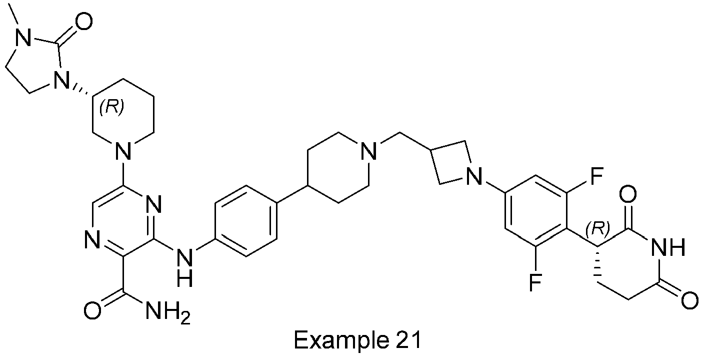

Example 4 (20 mg) was separated by SFC using a ChiralPak IE column (250 × 30 mm I.D., 10 µm) with mobile phase A (methanol + 0.1% ammonia) and B (acetonitrile) at a flow rate of 120 mL/min and room temperature. Two single-configuration compounds with different retention times were collected in sequence. After lyophilization, Example 20 (9 mg, yellow solid, retention time: 4.6 min) and Example 21 (10 mg, yellow solid, retention time: 6.7 min) were afforded, respectively.

### Example 22:

### 3-((4-(1-((1-(4-((S*)-2,6-dioxopiperidin-3-yl)-3,5-difluorophenyl)azetidin-3-yl)methyl)piperidin-4-yl)phenyl)amino)-5-((R)-3-(2-oxooxazolidin-3-yl)piperidin-1-yl)pyrazine-2-carboxamide

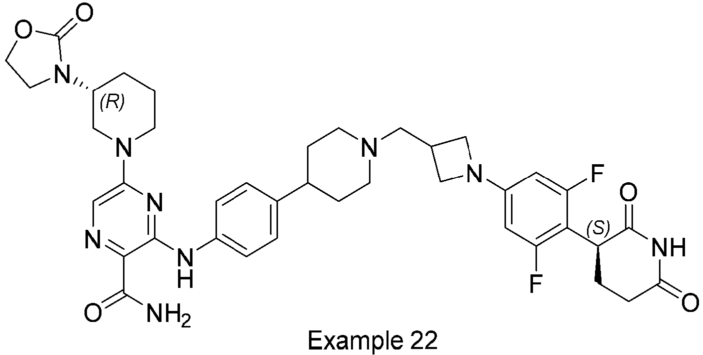

### Example 23:

### 3-((4-(1-((1-(4-((R*)-2,6-dioxopiperidin-3-yl)-3,5-difluorophenyl)azetidin-3-yl)methyl)piperidin-4-yl)phenyl)amino)-5-((R)-3-(2-oxooxazolidin-3-yl)piperidin-1-yl)pyrazine-2-carboxamide

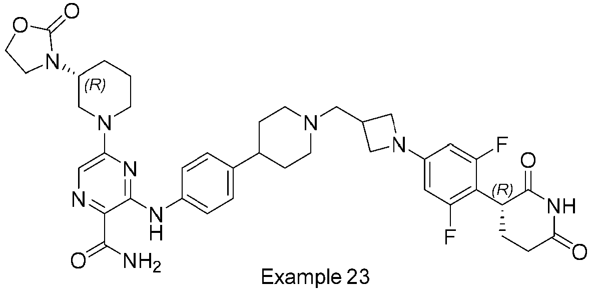

Examples 22 and 23 were synthesized using the same method as Examples 4, 20, and 21. Finally, SFC separation was carried out using a ChiralPak IE column (250 × 30 mm I.D., 10 µm) with mobile phase A (methanol + 0.1% ammonia) and B (acetonitrile) at a flow rate of 120 mL/min under room temperature. Two single-configuration compounds with different retention times were collected in sequence. After lyophilization, Example 22 (10 mg, yellow solid, retention time: 3.0 min) and Example 23 (8 mg, yellow solid, retention time: 4.2 min) were afforded, respectively.

LC-MS:m/z[M+H]⁺=758.4
¹HNMR(400MHz,DMSO-*d₆*) δ11.24(s,1H), 10.85(s,1H), 8.31(s,2H), 7.78(s,1H), 7.67(s,1H), 7.50(d,J=8.2Hz,2H),7.34(s,1H),7.16(d,J=8.2Hz,2H),6.11(d,J=11.4Hz,2H),4.40(d,J=12.8Hz,1H),4.29(q,J=7.6Hz,3H),3.9 8(m,4H),3.13(t,J=11.8Hz,2H),3.02(d,J=12.4Hz,1H),2.95(t,J=9.7Hz,4H),2.78(m,2H),2.60(d,J=7.4Hz,2H),2.45-2.37(m,1H),2.10-2.00(m,3H),1.99-1.89(m,2H),1.87-1.78(m,2H), 1.77-1.69(m,2H),1.61(m,3H).

### Example 24:

### (R)-methyl 3-((5-carbamoyl-6-((4-(4-(2,4-dioxotetrahydropyrimidin-1(2H)-yl)-1H-indazol-1-yl)-[1,4'-bipiperidinyl]-1'-yl)phenyl)amino)pyrazin-2-yl)(methyl)amino)piperidine-1-carboxylate

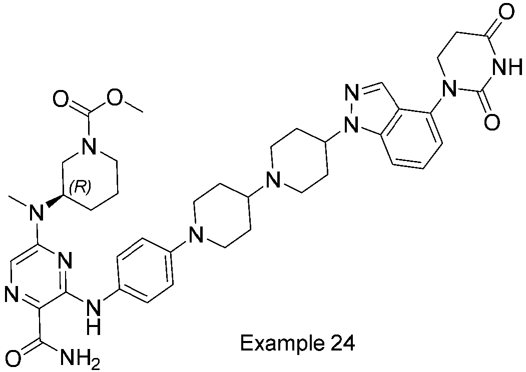

The synthesis of Example 24 was carried out by referring to Examples 18 and 19. Finally, Example 24 (44 mg, yellow solid) was obtained by reverse preparative column chromatography.

LC-MS:m/z[M+H]⁺=779.4
¹HNMR(400MHz,DMSO-*d₆*) δ 11.04(s,1H), 10.45(s,1H), 8.21(s,1H), 8.02(s,1H), 7.72(s,1H), 7.66(d,J=8.8Hz,1H), 7.48(s,1H),7.45-7.36(m,3H), 7.29(s,1H), 7.04(d,J=7.4Hz,1H), 6.85(d,J=8.9Hz,2H), 4.62(s,1H), 4.48-4.33(m,1H), 3.99(s,2H), 3.88(t,J=6.7Hz,3H), 3.64(d,J=9.5Hz,2H), 3.07(s,2H), 3.02(s,3H), 2.78(t,J=6.5Hz,3H),2.66(d,J=12.0Hz,4H),2.5(s,3H),2.11(d,J=10.0Hz,2H),1.82(m,7H),1.61(d,J=10.6Hz,2H),1.47(s,1H).

### Example 25:

### 5-((R(-3-(3-cyclopropyl-2-oxoimidazolidin-1-yl)piperidin-1-yl)-3-(4-(1-(4-((S*)-2,6-dioxopiperidin-3-yl)-3,5-difluorophenyl)azetidin-3-yl)methyl)piperidin-4-yl)phenyl)amino)pyrazine-2-carboxamide

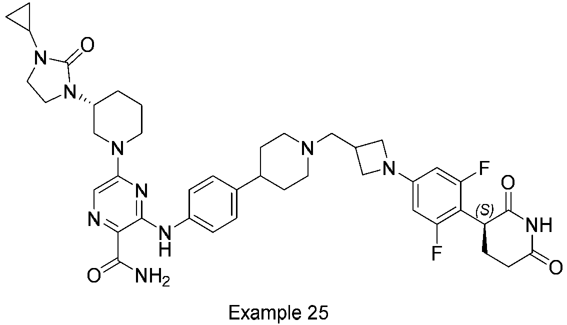

### Example 26:

### 5-((R)-3-(3-cyclopropyl-2-oxoimidazolidin-1-yl)piperidin-1-yl)-3-(4-(1-(4-(R*)-2,6-dioxopiperidin-3-yl)-3,5-difluorophenyl)azetidin-3-yl)methyl)piperidin-4-yl)phenyl)amino)pyrazine-2-carboxamide

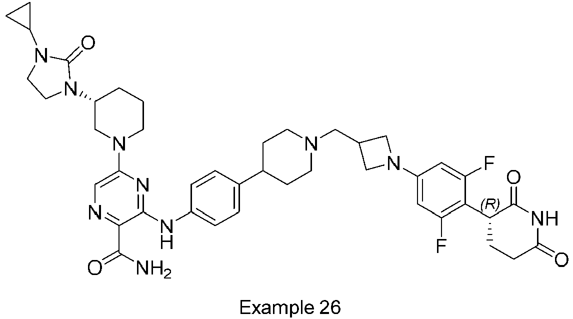

Examples 25 and 26 were synthesized using the same method as Examples 4, 20, and 21. Finally, SFC separation was carried out using a ChiralPak IE column (250 × 30 mm I.D., 10 µm) with mobile phase A (methanol + 0.1% ammonia) and B (acetonitrile) at a flow rate of 120 mL/min under room temperature. Two single-configuration compounds with different retention times were obtained in sequence. After lyophilization, Example 25 (68 mg, yellow solid, retention time: 4.57 min) and Example 26 (75 mg, yellow solid, retention time: 6.38 min) were afforded, respectively.

LC-MS:m/z[M+H]⁺=797.4;
¹HNMR(400MHz,DMSO-*d₆*) δ 11.22(s,1H), 10.85(s,1H), 7.75(s,1H), 7.66(s,1H), 7.50(d,J=8.4Hz,2H), 7.33(s,1H), 7.17(d,J=8.5Hz,2H), 6.10(d,J=11.1Hz,2H), 4.33(m,2H), 4.02(dd,J=12.6,5.0Hz,1H), 3.94(t,J=7.6Hz,2H), 3.66-3.57(m,1H), 3.49(m,2H), 3.28-3.22(m,5H), 3.03-2.88(m,5H), 2.83-2.72(m,1H), 2.58(d,J=7.3Hz,2H), 2.46-2.37(m,2H), 2.13-1.91(m,4H),1.86-1.68(m,5H), 1.68-1.51(m,3H),0.69-0.57(m,4H).

Control Example 1: Control Example 1 was synthesized according to the synthesis method of compound 195 disclosed in patent WO2021113557A1.

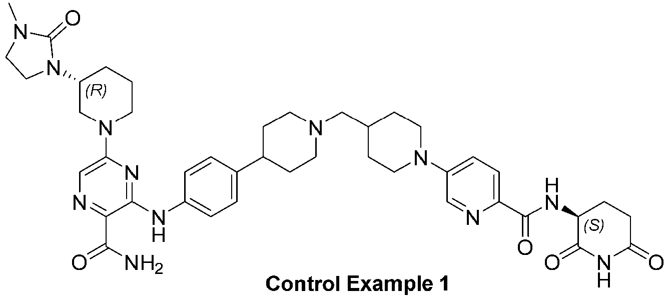

### Experimental Example 1: Mino and TMD8 cell proliferation assay

Mino or TMD8 cells were cultured in RPMI 1640 medium containing 10% fetal bovine serum (Gibco). After reaching the logarithmic growth phase, cells were collected, counted and seeded into 96-well plates at 2000 cells/well. Following overnight incubation, compounds were added to reach final concentrations of 1 µM, 200 nM, 40 nM, 8 nM, 1.6 nM, 0.32 nM, 0.064 nM, 0.0128 nM, 0.00256 nM and 0.00051 nM, with a final DMSO concentration of 0.2%. 0.2% DMSO and blank RPMI 1640 medium (without cells and compounds) were set as vehicle control and negative control, respectively. Two replicates were set for each concentration. After the test compound was mixed, the 96-well plate was placed in a 37°C, 5% CO₂ incubator and cultured (Mino cells were incubated for 96 h, REC-1 and TMD8 cells were incubated for 72 h). After incubation, the plates were placed at room temperature, 50 µL of CTG reagent was added, and the plates were shaken for 2 min and incubated at room temperature for 10 min. The luminescence signal (RLU) in each well was measured using a microplate reader to calculate the cell growth inhibition rate, followed by fitting a dose-response curve to determine the half-inhibitory concentration (IC₅₀).

**Table 1: IC₅₀ of the exemplary compounds against TMD8/Mino cells**

| Compound | TMD8 IC₅₀ (nM) | Mino IC₅₀ (nM) | Compound | TMD8 IC₅₀ (nM) | Mino IC₅₀ (nM) |
|---|---|---|---|---|---|
| Example 1 | 3.9 | - | Example 14 | 1.3 | 1.95 |
| Example 2 | 2.7 | - | Example 15 | 1.3 | 1.56 |
| Example 3 | 8.9 | 9.95 | Example 16 | 0.3 | - |
| Example 4 | 1.4 | 7.73 | Example 17 | 0.4 | - |
| Example 5 | - | 12.71 | Example 18 | 0.4 | - |
| Example 6 | 0.8 | 3.07 | Example 19 | 0.3 | - |
| Example 7 | 9.7 | 2.58 | Example 20 | 0.4 | - |
| Example 8 | - | 7.73 | Example 21 | 4.7 | - |
| Example 9 | 1.7 | 0.2 | Example 22 | 0.3 | - |
| Example 10 | 1.5 | 0.81 | Example 23 | 3.4 | - |
| Example 11 | 8.4 | - | Example 24 | 0.3 | - |
| Example 12 | 3.1 | 0.57 | Example 25 | 1.5 | - |
| Example 13 | 9.9 | 3.62 | Control Example 1 | 2.2 | 11.4 |

| | | | | | |
|---|---|---|---|---|---|
| -indicates the activity was not determined. | | | | | |

All exemplary compounds in the present application demonstrate excellent proliferative inhibitory effects on the respective tumor cells.

### Experimental Example 2: BTK mutant cell inhibitory activity assay

REC-1-BTK-L528W or Baf3-BTK-T474I cells in the logarithmic growth phase were seeded into 96-well plates at a density of 3 × 10³ cells per well. Compound solutions at various concentrations (starting from 2 µM with five-fold serial dilution) were added. The plates were gently mixed and incubated in a cell culture incubator for 72 h. Subsequently, 50 µL Cell Titer-Glo reagent was added. The plates were shaken for 2 min and incubated at room temperature for 10 min. The luminescence signal (RLU) in each well was measured using a microplate reader to calculate the cell growth inhibition rate, followed by fitting a dose-response curve to determine the half-inhibitory concentration (IC₅₀).

**Table 2: IC₅₀ of the exemplary compounds against BTK-L528W/T474I mutant cells**

| Compound | REC-1-BTK-L528W IC₅₀ (nM) | Baf3-BTK-T474I IC₅₀ (nM) |
|---|---|---|
| Control Example 1 | 29 | 12.63 |
| Example 17 | 29 | - |
| Example 19 | - | 3.13 |
| Example 20 | 0.35 | 1.75 |
| Example 22 | 1.0 | 1.15 |
| Example 24 | 5.38 | 4.17 |

| | | |
|---|---|---|
| -indicates not determined. | | |

All exemplary compounds in the present application demonstrate excellent proliferative inhibition capabilities against cells harboring BTK mutations.

### Test Example 3: Single oral administration test in mice

Mice were administered a single oral dose of 10 mpk. On the day of administration, the compound was dissolved in a mixture (10% DMSO, 40% PEG 400, 5% Tween 80 and 45% saline) and vortexed until a clear, transparent solution was obtained. Following oral gavage, whole blood samples were collected at 6 h and 24 h, and spleen samples were collected at 24 h. BTK protein levels were analyzed by Western blotting.

Whole blood samples were pretreated using a whole blood protein extraction kit. According to the manufacturer's instructions, 100× PMSF was added to the protein extract and vortexed thoroughly. An equal volume of the protein extract was combined with the whole blood sample, mixed well, and shaken on ice for 20 min. A tube was inserted into the centrifuge column, and the extract solution was pipetted into the column. Centrifugation was performed at 4 °C and 10,000 × g for 3-5 min. The eluate from the collection tube was collected as total whole blood protein.

Mouse spleen samples were minced, and 150 µL of RIPA lysis buffer containing PMSF was added. The tissues were homogenized on ice using a tissue homogenizer and incubated on ice for an additional 10-20 min for lysis. The samples were centrifuged at 4 °C and 13,000 × g for 30 min. The supernatant was transferred to a fresh centrifuge tube to obtain total spleen protein.

Protein concentration was determined by the BCA assay. After heat denaturation, proteins were separated by sodium dodecyl sulfate-polyacrylamide gel electrophoresis and transferred onto PVDF membranes. The membranes were incubated with blocking buffer for 1 h at room temperature, followed by overnight incubation with primary antibodies against BTK and GAPDH (both diluted 1:1000) at 4°C. After three washes with TBST, HRP-conjugated rabbit anti-goat secondary antibody (diluted 1:10,000) was applied and incubated for 1 h at room temperature. The membranes were then washed three times with TBST. ECL chemiluminescent solution was added, and images were acquired using a gel imaging system. The grayscale values of target protein bands were quantified with Image-Pro Plus 6.0 software. Relative BTK protein levels were compared with those of the vehicle group, and the degradation rate (Deg%) was calculated.

**Table 3 BTK degradation rate in whole blood/spleen of mice after single administration of exemplary compounds of the present application**

| Compound | 6h whole blood BTK Deg% | 24h whole blood BTK Deg% | 24h spleen BTK Deg% |
|---|---|---|---|
| Control Example 1 | B | B | B |
| Example 4 | A | A | - |
| Example 14 | A | A | - |
| Example 17 | B | A | - |
| Example 19 | C | B | C |
| Example 22 | B | A | B |
| Example 24 | C | C | B |

| | | | |
|---|---|---|---|
| A: >80%, B: 60-80%, C: <60%, - indicates not determined. | | | |

Table 3 data indicate that all exemplary compounds in the present application demonstrate strong degradation activity against BTK in mice.

For the purposes of description and disclosure, all patents, patent applications, and other publications are hereby expressly incorporated by reference herein. These publications are included solely because they were disclosed prior to the filing date of the present application. All statements regarding the dates of these documents or the representations of their contents are based on information available to the applicant and do not constitute any admission as to the correctness of the dates of these documents or the contents of these documents. Furthermore, in any country, any reference to these publications herein does not constitute an admission that such publications have become part of the common knowledge in the field.

The examples described above merely illustrate the preferred implementations of the present application and do not limit its scope. Without departing from the design philosophy of the present application, all modifications and improvements to the technical solutions made by those skilled in the art shall fall within the protection scope defined by the claims.

## Claims

1. A compound of formula (I), a pharmaceutically acceptable salt thereof, a deuterated compound thereof, a stereoisomer thereof, a tautomer thereof, or a mixture thereof, 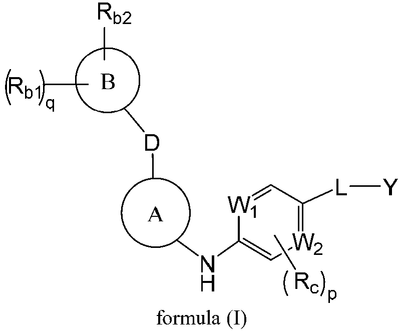 wherein
ring A is selected from the group consisting of 5-12 membered heteroaryl and 5-12 membered heterocyclyl, each optionally substituted with 1, 2, or 3 Rₐ;
D is selected from the group consisting of a bond, -CR₁R₁^{'}-, -S-, -O-, and -NR₂-(CH₂)ₘ-;
ring B is selected from the group consisting of 6-12 membered aryl, 5-12 membered heteroaryl, 5-12 membered heterocyclyl, or C₃-C₁₂ cycloalkyl;
W₁ and W₂ are each independently selected from the group consisting of C and N;
Rₐ is selected from the group consisting of H, C₁-C₆ alkyl, and -C(=O)-NH₂;
R_{b1} is selected from the group consisting of C₁-C₆ haloalkyl, C₁-C₆ alkyl, and halogen;
R_{b2} is selected from the group consisting of 4-6 membered heterocyclyl, -C(=O)-R₃, -NR₄R₅, -S(=O)₂R₃ and -NR₆-C(=O)-R₇; wherein the 4-6 membered heterocyclyl is optionally substituted with 1 or 2 R₈;
R₁, R₁^{'}, R_{c} and R₂ are each independently selected from the group consisting of H, halogen, C₁-C₆ haloalkyl, C₁-C₆ haloalkoxy, C₁-C₆ alkoxy, and C₁-C₆ alkyl;
R₃ is each independently selected from the group consisting of C₃-C₁₀ cycloalkyl, 5-12 membered heterocyclyl, C₁-C₆ alkyl, C₁-C₆ haloalkyl, -S(=O)₂CH₃, and NR₄R₅; wherein the C₃-C₁₀ cycloalkyl, 5-12 membered heterocyclyl, C₁-C₆ alkyl, and C₁-C₆ haloalkyl are each optionally substituted with 1-3 substituents independently selected from the group consisting of halogen, cyano, -SF₅, C₁-C₆ alkyl, C₁-C₆ haloalkoxy, and C₁-C₆ haloalkyl;
R₄, R₅, and R₆ are each independently selected from the group consisting of H, C₁-C₆ alkyl, and -S(=O)₂CH₃;
R₇ is selected from the group consisting of 5-6 membered heteroaryl and phenyl, each optionally substituted with 1-3 R₉;
R₈ is each independently selected from the group consisting of H, C₁-C₆ alkyl, and -S(=O)₂CH₃;
R₉ is selected from the group consisting of halogen, C₁-C₆ alkyl, and C₁-C₆ hydroxyalkyl;
L is -X₁X₂-X₃-X₄-X₅-X₆-;
X₁ is selected from the group consisting of a bond, -C(=O)-, C₁-C₆ alkylene, C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl; the C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl are each optionally substituted with 1-3 substituents independently selected from the group consisting of C₁-C₆ alkyl, C₁-C₆ haloalkyl, cyano, -C(O)NH₂, hydroxyl, and halogen;
X₂ is selected from the group consisting of a bond, -C(=O)-, and C₁-C₆ alkylene;
X₃, X₄, X₅, and X₆ are each independently selected from the group consisting of a bond, 3-12 membered heterocyclyl, C₁-C₆ alkylene; C₃-C₁₀ cycloalkyl, -O-, -NR₂-, and -C(=O)-; wherein not all of X₁, X₂, X₃, X₄, X₅, and X₆ are bonds;
Y is selected from the group consisting of 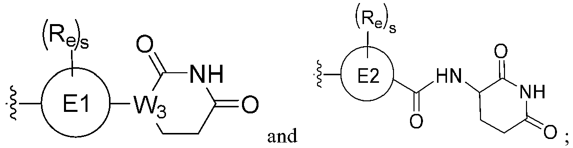 wherein W₃ is selected from the group consisting of N and C;
ring E1 is selected from the group consisting of 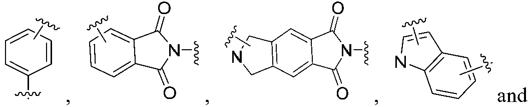 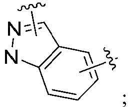 Rₑ is selected from the group consisting of halogen, methyl, methoxy, and trifluoromethyl;
ring E2 is selected from the group consisting of 5-10 membered heteroaryl;
m, p, and q are selected from 0, 1, 2, or 3; and
s is selected from 0, 1, or 2.

2. A compound of formula (I), a pharmaceutically acceptable salt thereof, a deuterated compound thereof, a stereoisomer thereof, a tautomer thereof, or a mixture thereof, 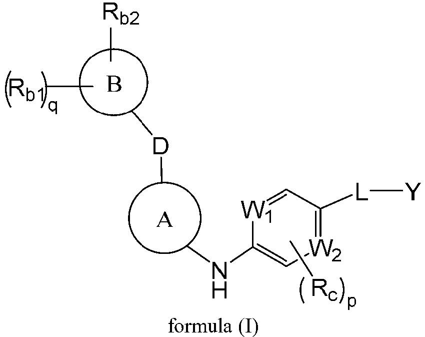 wherein
ring A is selected from the group consisting of 5-12 membered heteroaryl and 5-12 membered heterocyclyl, each optionally substituted with 1, 2, or 3 Rₐ;
D is selected from the group consisting of a bond, -CR₁R₁^{'}-, -S-, -O- and -NR₂-(CH₂)ₘ-;
ring B is selected from the group consisting of 6-12 membered aryl, 5-12 membered heteroaryl, 5-12 membered heterocyclyl, and C₃-C₁₂ cycloalkyl;
W₁ and W₂ are each independently selected from the group consisting of C and N;
Rₐ is selected from the group consisting of H, C₁-C₆ alkyl, and -C(=O)-NH₂;
R_{b1} is selected from the group consisting of C₁-C₆ haloalkyl, C₁-C₆ alkyl, and halogen;
R_{b2} is selected from the group consisting of 4-6 membered heterocyclyl, -C(=O)-R₃, -NR₄R₅, -S(=O)₂R₃ and -NR₆-C(=O)-R₇; wherein the 4-6 membered heterocyclyl is optionally substituted with 1 or 2 R₈;
R₁, R₁^{'}, R_{c} and R₂ are each independently selected from the group consisting of H, halogen, C₁-C₆ haloalkyl, C₁-C₆ haloalkoxy, C₁-C₆ alkoxy, and C₁-C₆ alkyl;
R₃ is each independently selected from the group consisting of C₃-C₁₀ cycloalkyl, 5-12 membered heterocyclyl, C₁-C₆ alkyl, C₁-C₆ alkoxy, C₁-C₆ haloalkyl, -S(=O)₂CH₃, and NR₄R₅; wherein the C₃-C₁₀ cycloalkyl, 5-12 membered heterocyclyl, C₁-C₆ alkyl, and C₁-C₆ haloalkyl are each optionally substituted with 1-3 substituents independently selected from the group consisting of halogen, cyano, -SF₅, C₁-C₆ alkyl, C₁-C₆ haloalkoxy, and C₁-C₆ haloalkyl;
R₄, R₅, and R₆ are each independently selected from the group consisting of H, C₁-C₆ alkyl, and -S(=O)₂CH₃;
R₇ is selected from the group consisting of 5-6 membered heteroaryl and phenyl, each optionally substituted with 1-3 R_{9;}
R₈ is each independently selected from the group consisting of H, C₁-C₆ alkyl, and -S(=O)₂CH₃;
R₉ is selected from the group consisting of halogen, C₁-C₆ alkyl, and C₁-C₆ hydroxyalkyl;
L is -X₁-X₂-X₃-X₄-X₅-X₆-;
X₁ is selected from the group consisting of a bond, -C(=O)-, C₁-C₆ alkylene, C₅-C₈ monocycloalkyl, C₇-C₁₂ spirocycloalkyl; 7-12 membered spiroheterocyclyl; 5-12 membered bridged heterocyclyl; and 3-8 membered monocyclic heterocyclyl; the C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl are each optionally substituted with 1-3 substituents independently selected from the group consisting of C₁-C₆ alkyl, C₁-C₆ haloalkyl, cyano, -C(O)NH₂, hydroxyl, and halogen;
X₂ is selected from the group consisting of a bond, -C(=O)-, and C₁-C₆ alkylene;
X₃, X₄, X₅, and X₆ are each independently selected from the group consisting of a bond, 3-12 membered heterocyclyl, C₁-C₆ alkylene; C₃-C₁₀ cycloalkyl, -O-, -NR₂-, and -C(=O)-; wherein not all of X₁, X₂, X₃, X₄, X₅, and X₆ are bonds;
Y is selected from the group consisting of 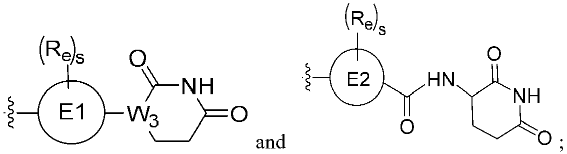 where W₃ is selected from the group consisting of N and C;
ring E1 is selected from the group consisting of 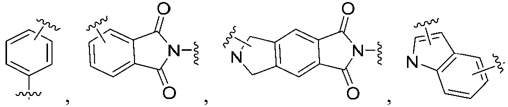
and 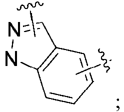 Rₑ is selected from the group consisting of halogen, methyl, methoxy, and trifluoromethyl;
ring E2 is selected from 5-10 membered heteroaryl;
m, p, and q are each independently selected from 0, 1, 2, or 3; and
s is selected from 0, 1, or 2.

3. The compound of formula (I), pharmaceutically acceptable salt thereof, deuterated compound thereof, stereoisomer thereof, tautomer thereof, or mixture thereof according to claim 1 or 2, wherein ring A is selected from the group consisting of 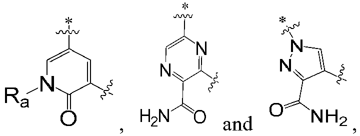 wherein * indicates the position of attachment to D, provided that: ring A is selected from the group consisting of 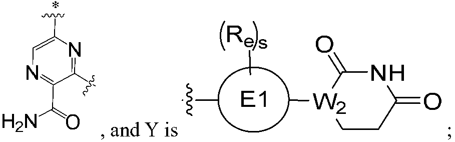
Rₐ is selected from the group consisting of H, C₁-C₃ alkyl and -C(=O)-NH₂, preferably, Rₐ is selected from the group consisting of C₁-C₃ alkyl and -C(=O)-NH₂, more preferably, Rₐ is selected from the group consisting of -CH₃ and -C(=O)-NH₂;
R_{b1} is selected from the group consisting of C₁-C₆ haloalkyl, C₁-C₆ alkyl, and halogen, preferably selected from the group consisting of C₁-C₃ haloalkyl, C₁-C₃ alkyl, and halogen, more preferably C₁-C₃ alkyl and halogen.

4. The compound of formula (I), pharmaceutically acceptable salt thereof, deuterated compound thereof, stereoisomer thereof, tautomer thereof, or mixture thereof according to claim 1 to 3, wherein R_{b2} is selected from the group consisting of 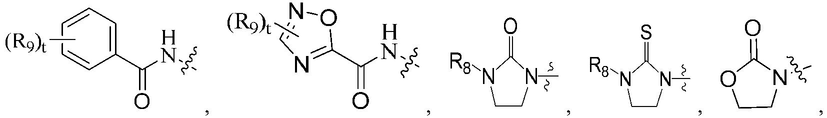 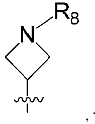 -C(=O)-R₃, -NR₄R₅ and -S(=O)₂R₃;
R₃ is each independently selected from the group consisting of C₃-C₅ cycloalkyl, C₁-C₃ alkyl, and C₁-C₃ alkoxy, each optionally substituted with 1-3 substituents independently selected from the group consisting of halogen, cyano, -SF₅, C₁-C₆ alkyl, C₁-C₆ haloalkoxy, and C₁-C₆ haloalkyl;
R₉ is selected from the group consisting of halogen, C₁-C₆ alkyl, and C₁-C₆ hydroxyalkyl, preferably C₁-C₃ alkyl and C₁-C₃ hydroxyalkyl;
R₈ is each independently selected from the group consisting of H, C₁-C₆ alkyl, C₁-C₆ haloalkyl, and -S(=O)₂CH₃, preferably, R₈ is each independently selected from the group consisting of H and C₁-C₃ alkyl;
t is selected from the group consisting of 0, 1, or 2.

5. The compound of formula (I), pharmaceutically acceptable salt thereof, deuterated compound thereof, stereoisomer thereof, tautomer thereof, or mixture thereof according to any one of claims 1 to 4, wherein Y is selected from the group consisting of 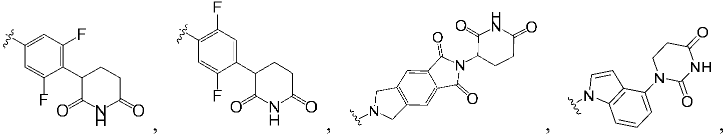 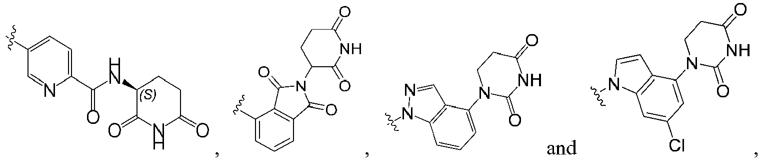 preferably, Y is selected from the group consisting of

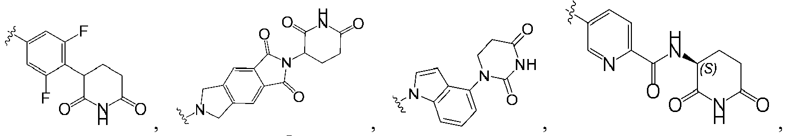

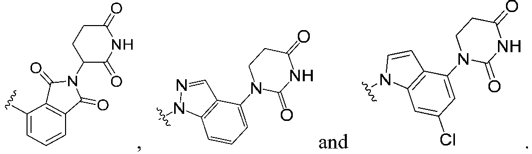

6. The compound of formula (I), pharmaceutically acceptable salt thereof, deuterated compound thereof, stereoisomer thereof, tautomer thereof, or mixture thereof according to any one of claims 1 to 5, wherein it has the structure shown in formula (II): 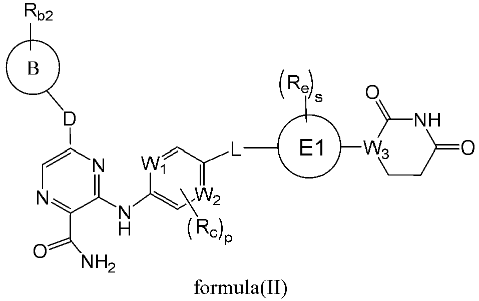 wherein
W₁, W₂, and W₃ are each independently selected from the group consisting of C and N;
D is selected from the group consisting of a bond, -CR₁R₁'-, -S-, -O-, and -NR₂-(CH₂)ₘ-;
ring B is selected from the group consisting of 6-12 membered aryl, 5-12 membered heteroaryl, 5-12 membered heterocyclyl, and C₃-C₆ cycloalkyl;
R_{b2} is selected from the group consisting of 4-6 membered heterocyclyl, -C(=O)-R₃, -NR₄R₅, -S(=O)₂R₃, and -NR₆-C(=O)-R₇; wherein the 4-6 membered heterocyclyl is optionally substituted with 1 or 2 R₈;
R₁, R₁^{'}, Rc, and R₂ are each independently selected from the group consisting of H, halogen, C₁-C₆ haloalkyl, C₁-C₆ haloalkoxy, C₁-C₆ alkoxy, and C₁-C₆ alkyl;
R₃ is each independently selected from the group consisting of C₃-C₁₀ cycloalkyl, 5-12 membered heterocyclyl, C₁-C₆ alkyl, C₁-C₆ haloalkyl, -S(=O)₂CH₃, and NR₄R₅; wherein the C₃-C₁₀ cycloalkyl, 5-12 membered heterocyclyl, C₁-C₆ alkyl, and C₁-C₆ haloalkyl are each optionally substituted with 1-3 substituents independently selected from the group consisting of halogen, cyano, -SF₅, C₁-C₆ alkyl, C₁-C₆ haloalkoxy, and C₁-C₆ haloalkyl;
R₄, R₅, and R₆ are each independently selected from the group consisting of H, C₁-C₆ alkyl, and -S(=O)₂CH₃;
R₇ is selected from the group consisting of 5-6 membered heteroaryl and phenyl, each optionally substituted with 1-3 R₉;
R₈ is each independently selected from the group consisting of H, C₁-C₆ alkyl, and -S(=O)₂CH₃;
R₉ is selected from the group consisting of halogen, C₁-C₆ alkyl, and C₁-C₆ hydroxyalkyl;
ring E1 is selected from the group consisting of 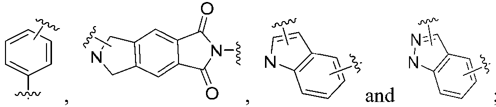 Rₑ is selected from the group consisting of halogen, methyl, methoxy, and trifluoromethyl;
m, s, and p are selected from 0, 1, or 2.

7. The compound of formula (I), pharmaceutically acceptable salt thereof, deuterated compound thereof, stereoisomer thereof, tautomer thereof, or mixture thereof according to any one of claims 2 to 5, wherein it has the structure shown in formula (II): 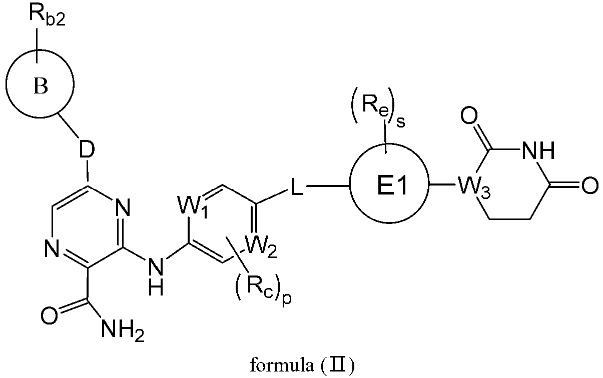 wherein
W₁, W₂, and W₃ are each independently selected from the group consisting of C and N;
D is selected from the group consisting of a bond, -CR₁R₁^{'}-, -S-, -O-, and -NR₂-(CH₂)ₘ-;
ring B is selected from the group consisting of 6-12 membered aryl, 5-12 membered heteroaryl, 5-12 membered heterocyclyl, and C₃-C₆ cycloalkyl;
R_{b2} is selected from the group consisting of 4-6 membered heterocyclyl, -C(=O)-R₃, -NR₄R₅, -S(=O)₂R₃, and -NR₆-C(=O)-R₇; wherein the 4-6 membered heterocyclyl is optionally substituted with 1 or 2 R₈;
R₁, R₁^{'}, Rc, and R₂ are each independently selected from the group consisting of H, halogen, C₁-C₆ haloalkyl, C₁-C₆ haloalkoxy, C₁-C₆ alkoxy, and C₁-C₆ alkyl;
R₃ is each independently selected from the group consisting of C₃-C₁₀ cycloalkyl, 5-12 membered heterocyclyl, C₁-C₆ alkyl, C₁-C₆ alkoxy, C₁-C₆ haloalkyl, -S(=O)₂CH₃, and NR₄R₅; wherein the C₃-C₁₀ cycloalkyl, 5-12 membered heterocyclyl, C₁-C₆ alkyl, and C₁-C₆ haloalkyl are each optionally substituted with 1-3 substituents independently selected from the group consisting of halogen, cyano, -SF₅, C₁-C₆ alkyl, C₁-C₆ haloalkoxy, and C₁-C₆ haloalkyl;
R₄, R₅, and R₆ are each independently selected from the group consisting of H, C₁-C₆ alkyl, and -S(=O)₂CH₃;
R₇ is selected from the group consisting of 5-6 membered heteroaryl and phenyl, each optionally substituted with 1-3 R₉;
R₈ is each independently selected from the group consisting of H, C₁-C₆ alkyl, and -S(=O)₂CH₃;
R₉ is selected from the group consisting of halogen, C₁-C₆ alkyl, and C₁-C₆ hydroxyalkyl;
L is -X₁-X₂-X₃-X₄-X₅-X₆-;
X₁ is selected from the group consisting of a bond, -C(=O)-, C₁-C₆ alkylene, C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl; the C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl are each optionally substituted with 1-3 substituents independently selected from the group consisting of C₁-C₆ alkyl, C₁-C₆ haloalkyl, cyano, -C(O)NH₂, hydroxyl, and halogen;
X₂ is selected from the group consisting of a bond, -C(=O)-, and C₁-C₆ alkylene;
X₃, X₄, X₅, and X₆ are each independently selected from the group consisting of a bond, 3-12 membered heterocyclyl, C₁-C₆ alkylene, C₃-C₁₀ cycloalkyl, -O-, -NR₂-, and -C(=O)-; wherein not all of X₁, X₂, X₃, X4, X₅, and X₆ are bonds;
ring E1 is selected from the group consisting of 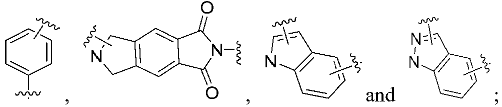
Rₑ is selected from the group consisting of halogen, methyl, methoxy, and trifluoromethyl;
m, s, and p are selected from 0, 1, or 2.

8. The compound of formula (I), pharmaceutically acceptable salt thereof, deuterated compound thereof, stereoisomer thereof, tautomer thereof, or mixture thereof according to any one of claims 1 to 7, wherein ring E1 is selected from the group consisting of 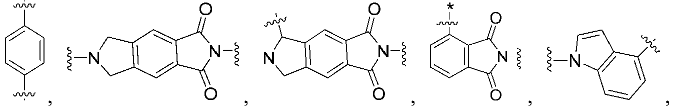 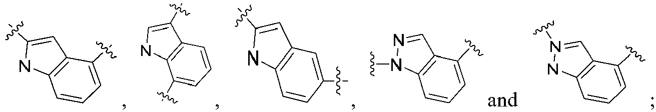 preferably, ring E1 is selected from the group consisting of 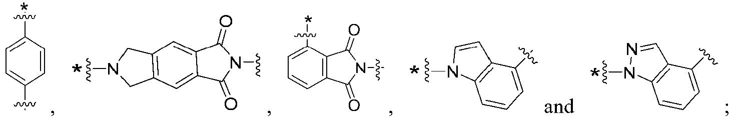 wherein * indicates the position of attachment to L; preferably, ring B is selected from the group consisting of phenyl, 5-6 membered heteroaryl, 5-6 membered heterocyclyl, and C₃-C₆ cycloalkyl; more preferably, ring B is selected from the group consisting of 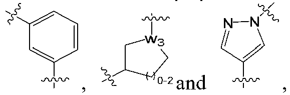 and W₃ is selected from the group consisting of C and N; further preferably, ring B is selected from the group consisting of 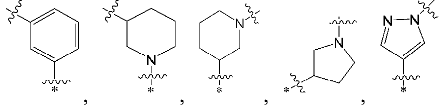 and 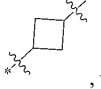 wherein * indicates the position of attachment to D.

9. The compound of formula (I), pharmaceutically acceptable salt thereof, deuterated compound thereof, stereoisomer thereof, tautomer thereof, or mixture thereof according to any one of claims 2 to 5 or 8, wherein ring A is selected from the group consisting of 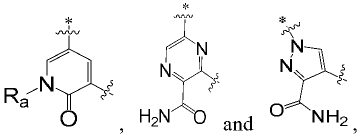 wherein * indicates the position of attachment to D, provided that when ring A is selected from the group consisting of 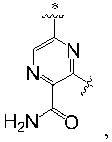 Y is 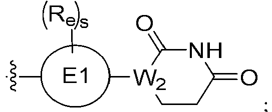
Rₐ is selected from the group consisting of C₁-C₃ alkyl and -C(=O)-NH₂, preferably -CH₃ and -C(=O)-NH₂;
D is selected from the group consisting of a bond, -NH-, -N(CH₃)-, and -NH-CH₂-; preferably, D is selected from the group consisting of a bond, -NH-, and -N(CH₃)-;
W₁ and W₂ are each independently selected from the group consisting of C and N;
ring B is selected from the group consisting of 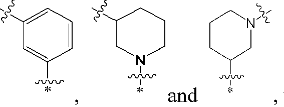 wherein * indicates the position of attachment to D;
R_{b1} is selected from the group consisting of C₁-C₃ alkyl and halogen, preferably, R_{b1} is selected from the group consisting of -CH₃ and F;
R_{b2} is selected from the group consisting of 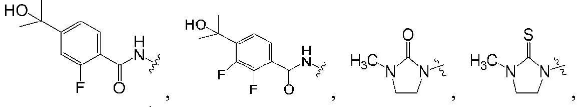 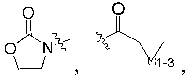 and -S(=O)₂CH₃, or alternatively from 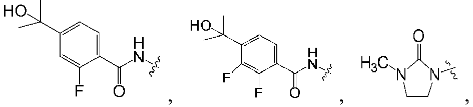 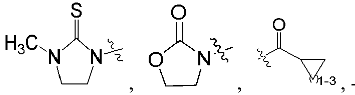 -C(=O)OCH₃ and -S(=O)₂CH₃;
q is selected from 0, 1, or 2;
Y is selected from the group consisting of 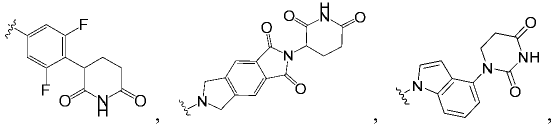 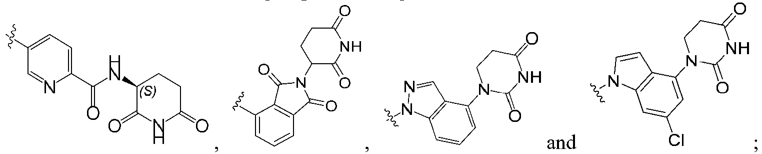
L is selected from the group consisting of 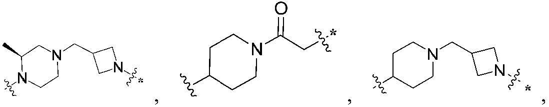 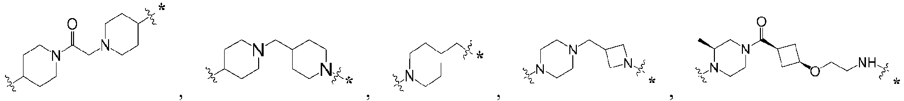 and 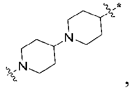 wherein * indicates the position of attachment to Y.

10. The compound of formula (I), pharmaceutically acceptable salt thereof, deuterated compound thereof, stereoisomer thereof, tautomer thereof, or mixture thereof according to any one of claims 6 to 8, wherein D is selected from the group consisting of a bond, -NH-, -N(CH₃)-, and -NH-CH₂-, preferably, D is selected from the group consisting of a bond, -NH-, and -N(CH₃)-;
W₁, W₂, and W₃ are each independently selected from the group consisting of C and N;
ring B is selected from the group consisting of 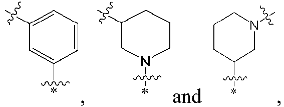 preferably, ring B is selected from the group consisting of 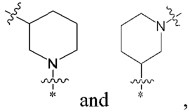 wherein * indicates the position of attachment to D;
R_{b2} is selected from the group consisting of 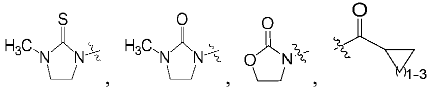 and -S(=O)₂CH₃; or alternatively from 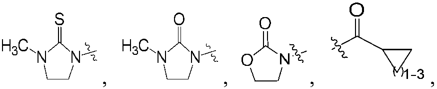 -C(=O)OCH₃ and -S(=O)₂CH₃;
ring E1 is selected from the group consisting of 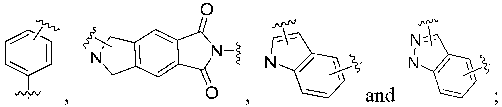
L is selected from the group consisting of 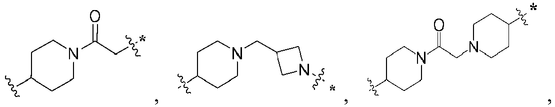 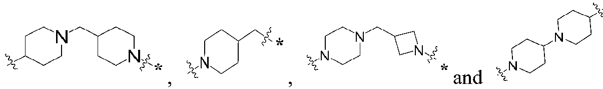 , wherein * indicates the position of attachment to Y.

11. The compound of formula (I), pharmaceutically acceptable salt thereof, deuterated compound thereof, stereoisomer thereof, tautomer thereof, or mixture thereof according to any one of claims 1 to 5, wherein the compound has the structure shown in formulas (I-1)-(I-4) 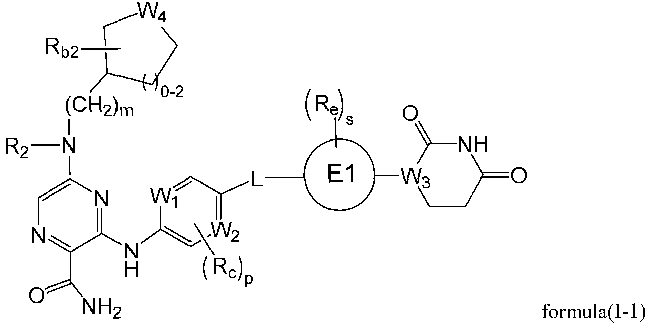 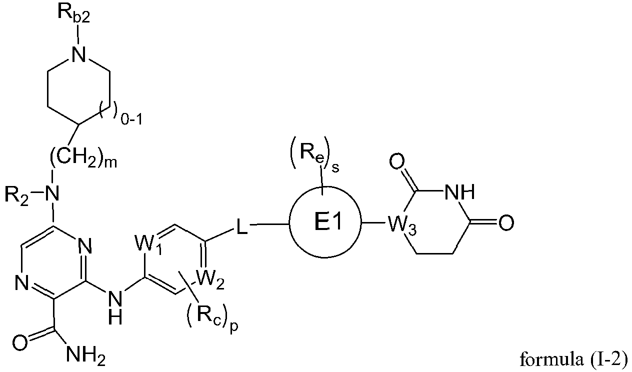 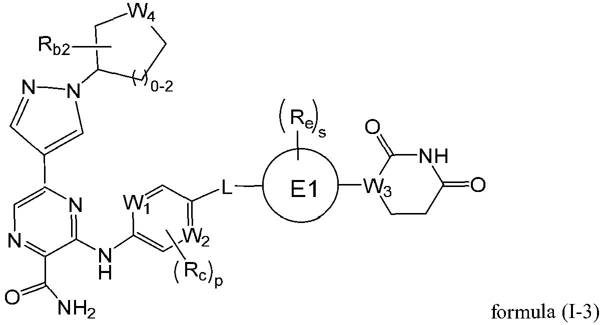 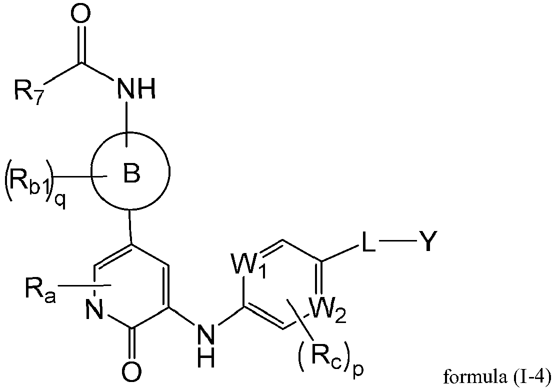 wherein
W₄ is selected from the group consisting of C and N;
W₁, W₂, W₃, R_{c}, R₂, R_{b2}, Rₑ, Rₐ, R₇, ring B, L, Y, ring E1, m, p, q, and s are defined as in any one of claims 1 to 5.

12. The compound of formula (I), pharmaceutically acceptable salt thereof, deuterated compound thereof, stereoisomer thereof, tautomer thereof, or mixture thereof according to any one of claims 1 to 9, wherein
L is -X₁-X₂-X₃-X₄-X₅-X₆-*, wherein * indicates the position of attachment to Y;
X₁ is selected from the group consisting of a bond, -C(=O)-, C₁-C₆ alkylene, C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl; wherein the C₅-C₈ monocycloalkyl, C₇-C₁₂ spirocycloalkyl, 7-12 membered spiroheterocyclyl, 5-12 membered bridged heterocyclyl, and 3-8 membered monocyclic heterocyclyl are optionally substituted with 1-3 substituents independently selected from the group consisting of C₁-C₆ alkyl, C₁-C₆ haloalkyl, cyano, -C(O)NH₂, hydroxyl, and halogen;
X₂ is selected from the group consisting of a bond and -C(=O)-;
X₃, X₄, X₅, and X₆ are each independently selected from the group consisting of a bond, 4-6 membered heterocyclyl, C₁-C₃ alkylene, C₃-C₆ cycloalkyl, -O-, -NH-, and -C(=O)- , and not all of X₁, X₂, X₃, X₄, X₅, and X₆ are bonds; preferably, L is selected from the group consisting of

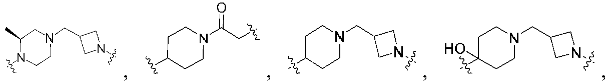

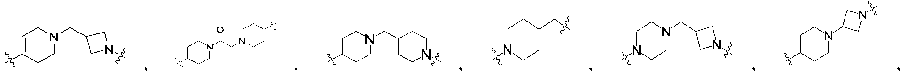

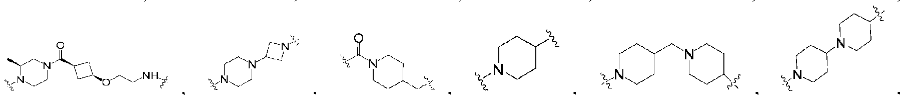

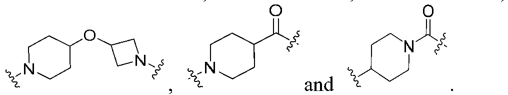

13. The compound of formula (I), pharmaceutically acceptable salt thereof, deuterated compound thereof, stereoisomer thereof, tautomer thereof, or mixture thereof according to any one of claims 1 to 12, the compound is selected from the group consisting of

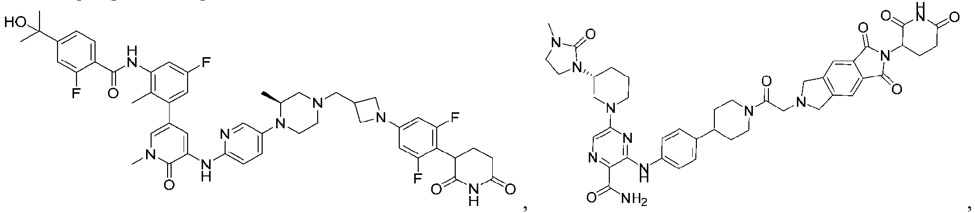

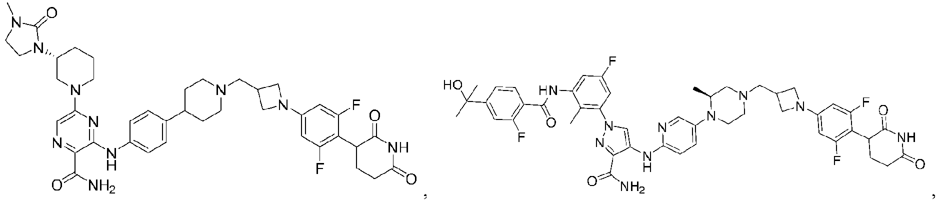

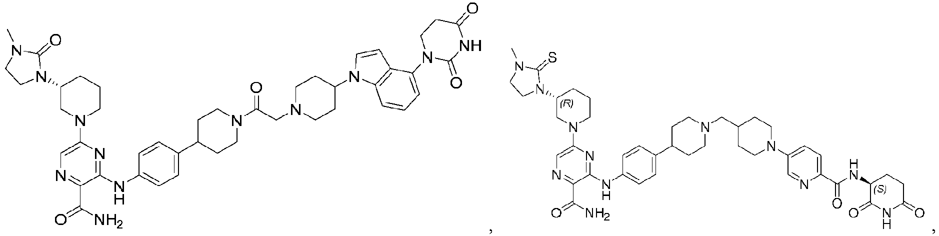

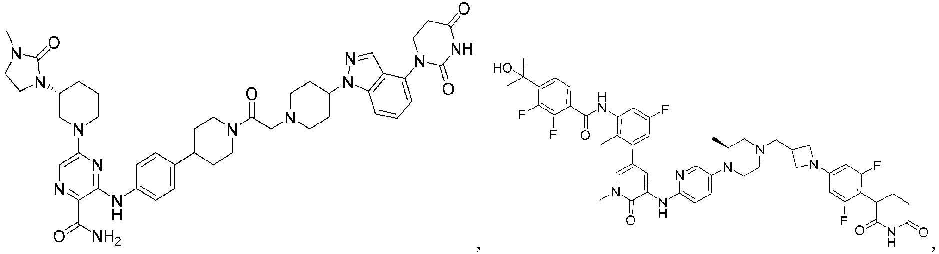

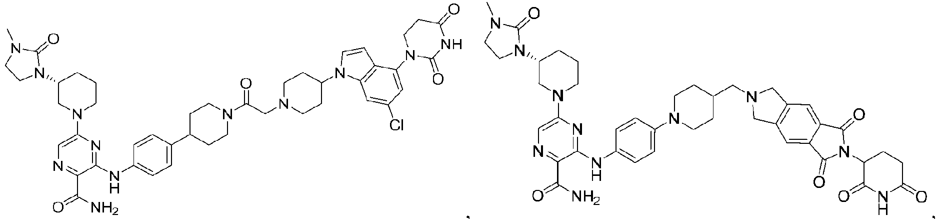

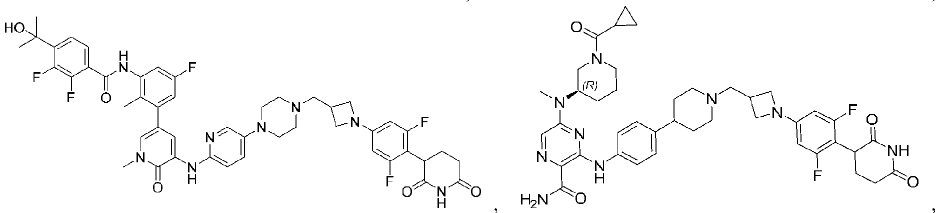

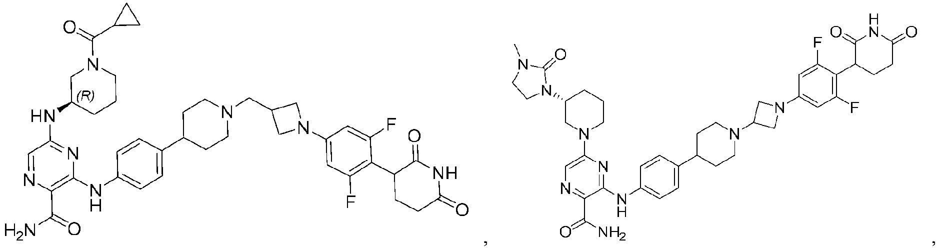

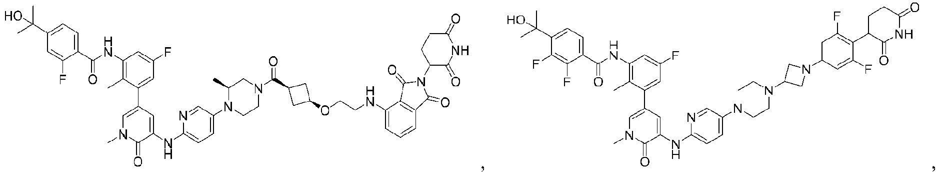

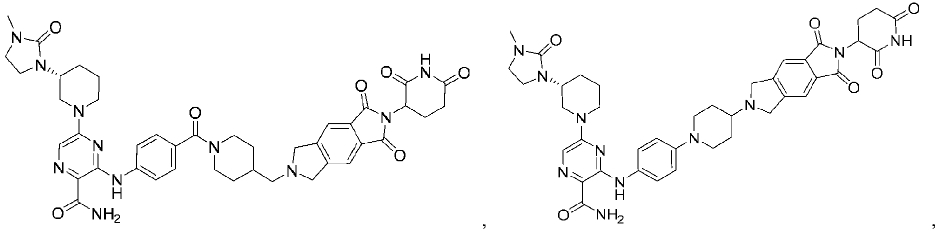

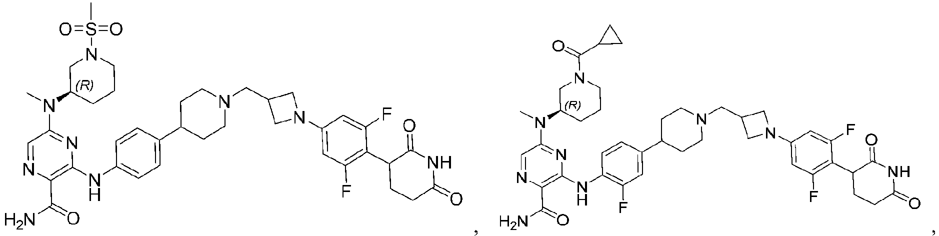

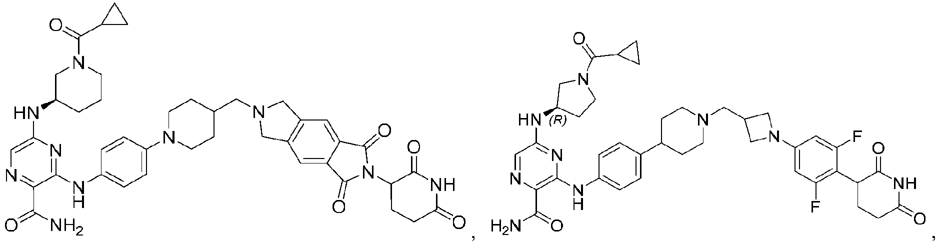

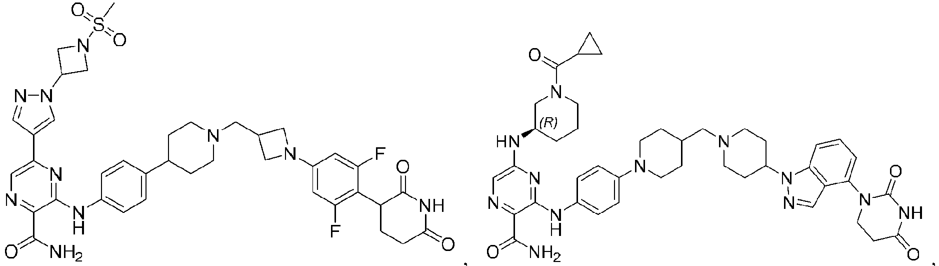

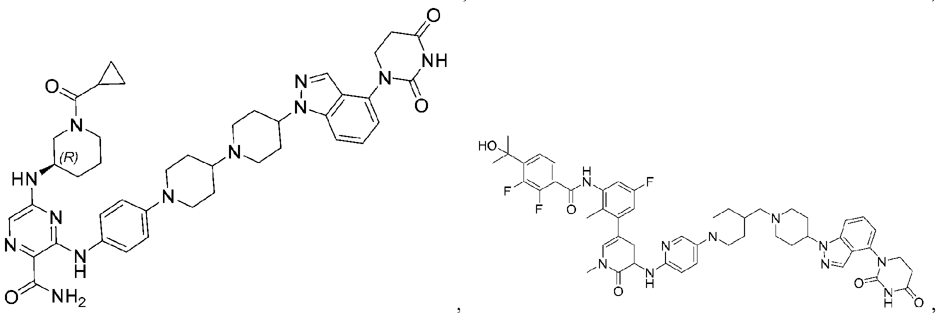

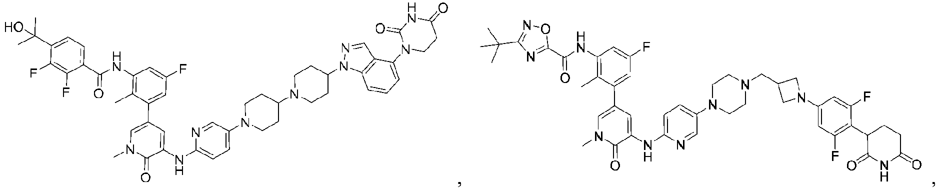

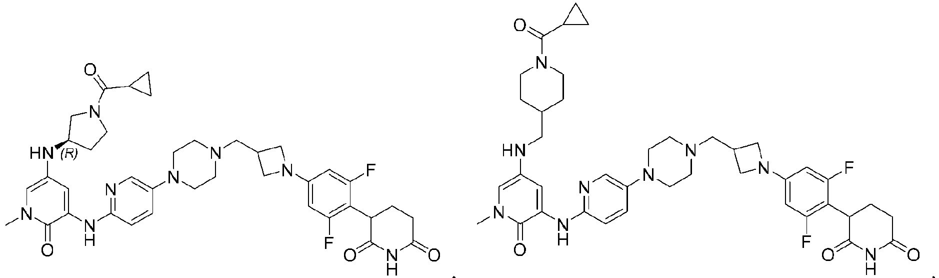

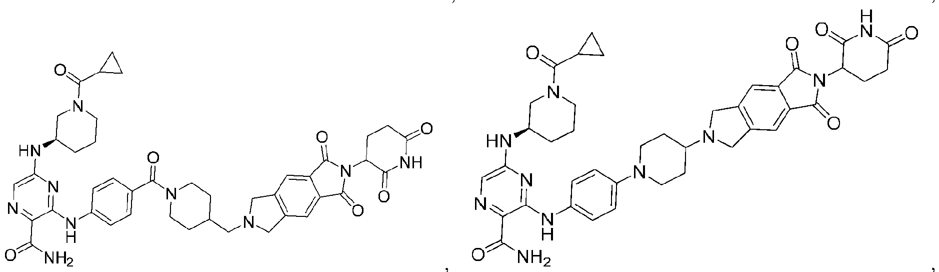

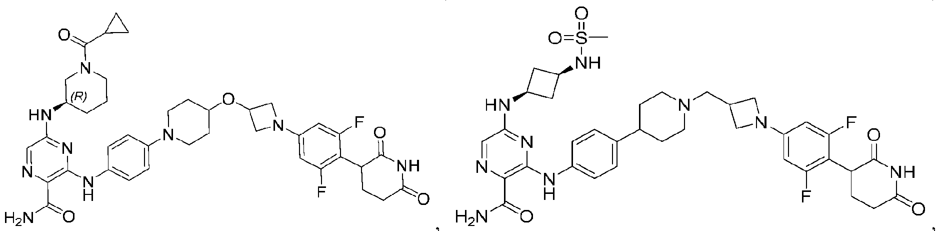

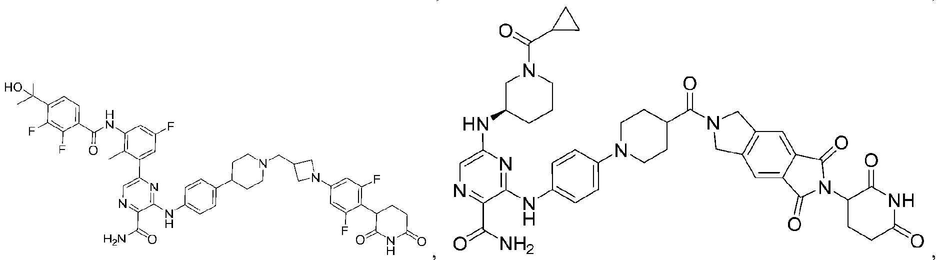

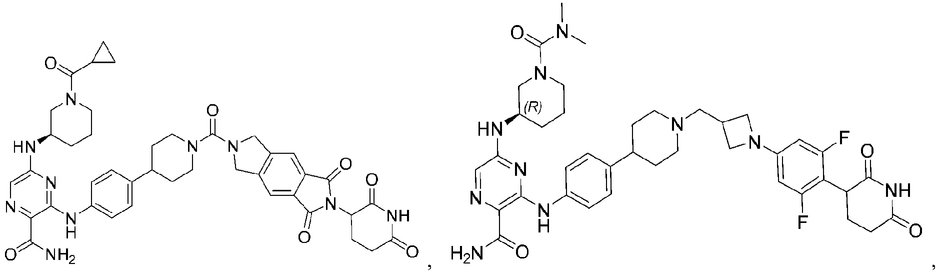

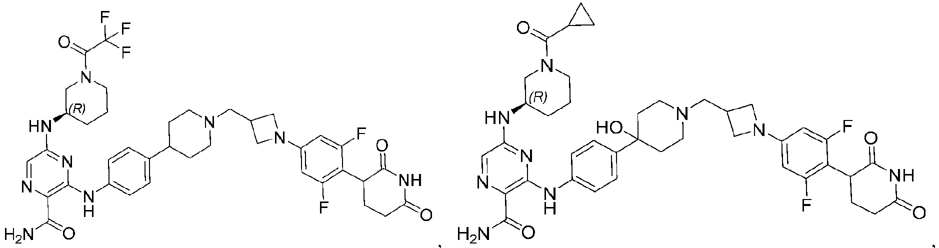

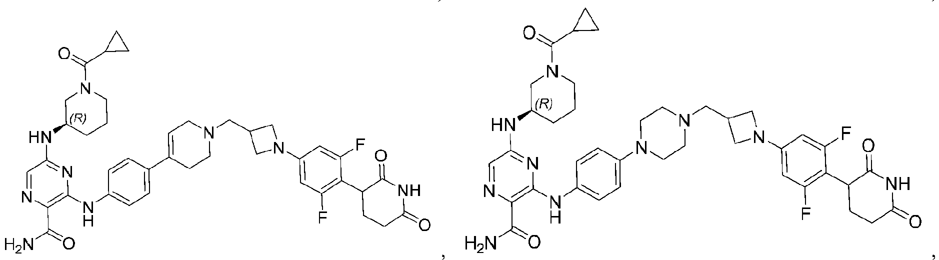

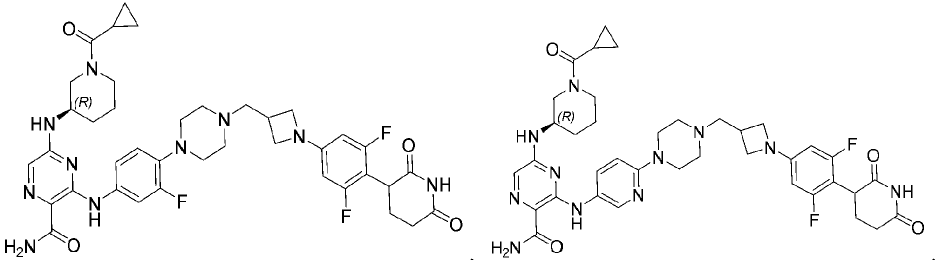

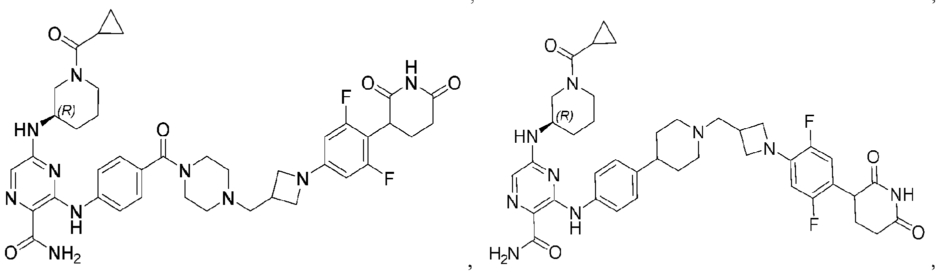

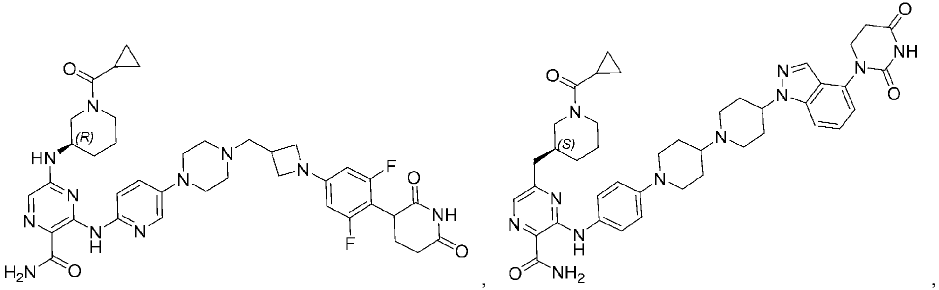

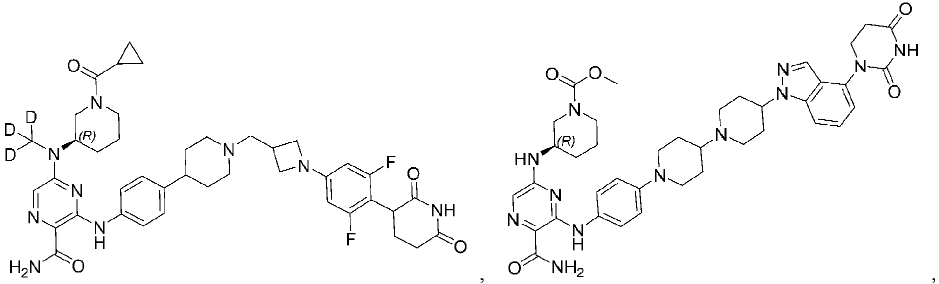

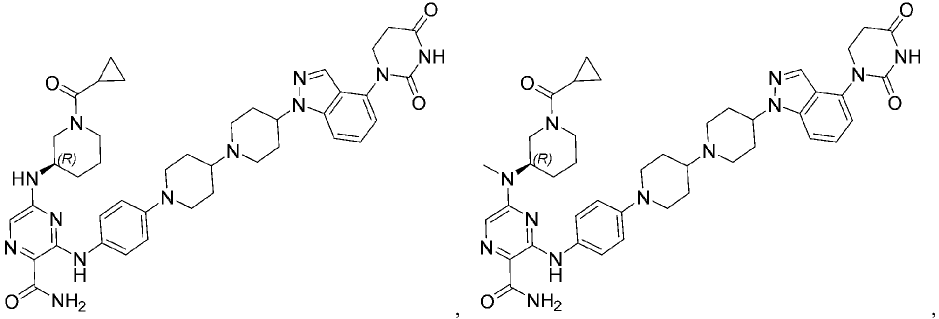

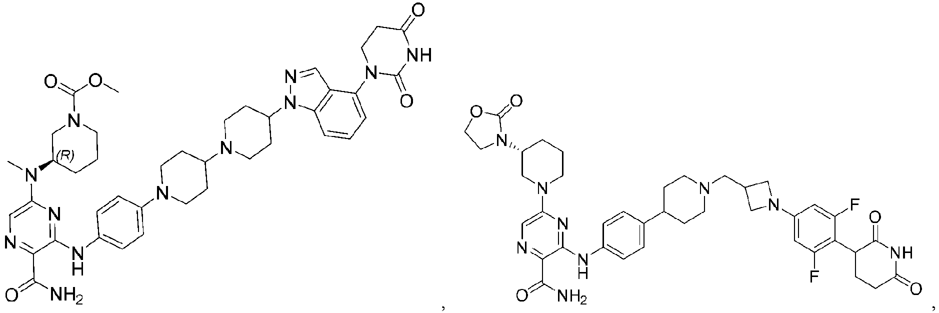

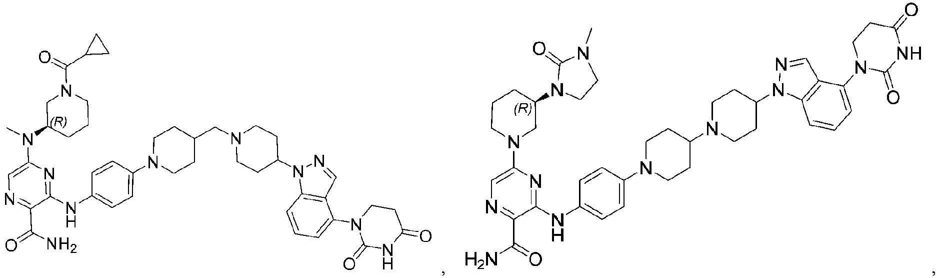

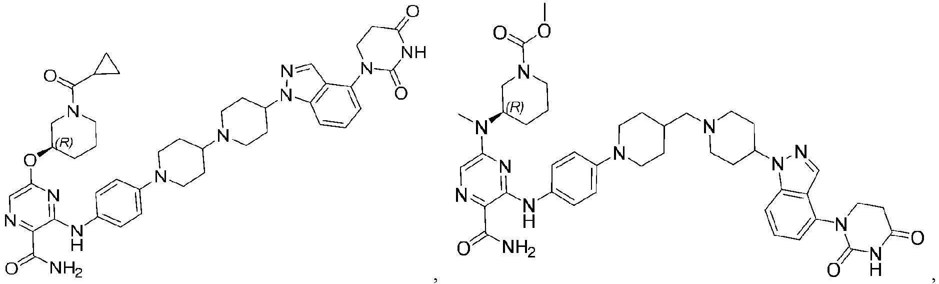

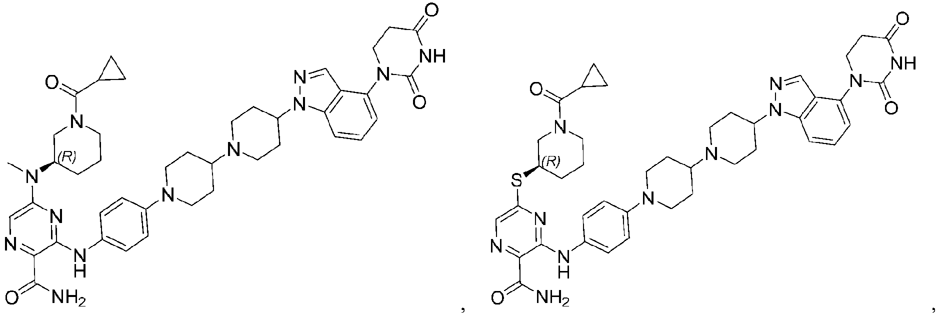

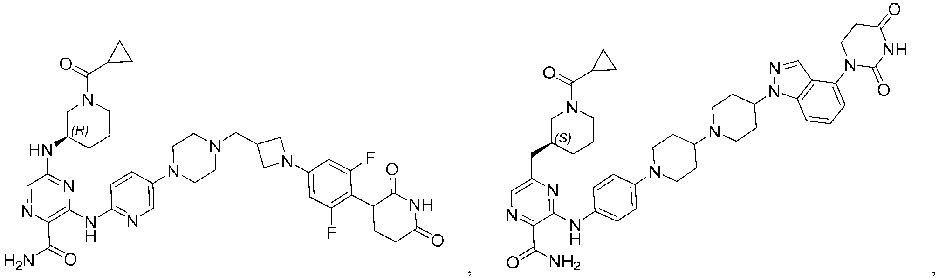

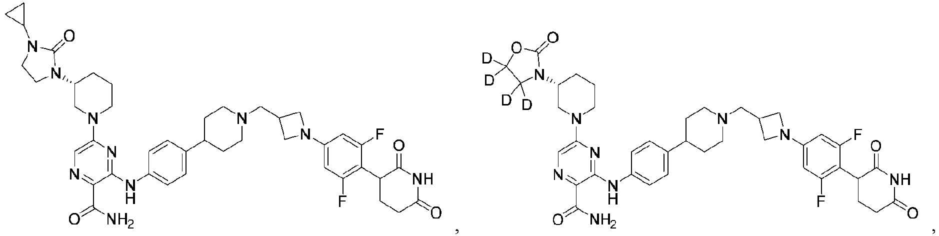

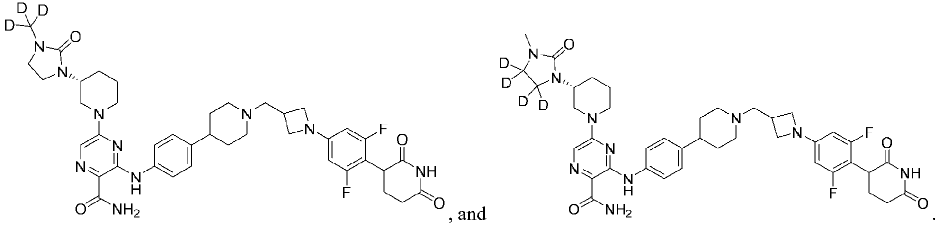

14. The compound of formula (I), pharmaceutically acceptable salt thereof, deuterated compound thereof, stereoisomer thereof, tautomer thereof, or mixture thereof according to any one of claims 1 to 13, for preventing and/or treating a disease or disorder mediated by degradation of Bruton^{'}s tyrosine kinase (BTK).

15. The compound of formula (I), pharmaceutically acceptable salt thereof, deuterated compound thereof, stereoisomer thereof, tautomer thereof, or mixture thereof for use according to claim **14,**
wherein the disease or disorder is cancer or an autoimmune disease; preferably, the cancer comprises myeloid leukemia (acute and chronic), acute lymphoblastic leukemia, chronic lymphocytic leukemia, myeloproliferative disease, multiple myeloma, myelodysplastic syndrome, Hodgkin disease, non-Hodgkin lymphoma (malignant lymphoma), hairy cell leukemia, mantle cell lymphoma, Waldenstrom macroglobulinemia, marginal zone lymphoma and follicular lymphoma; the autoimmune disease comprises inflammatory bowel disease, arthritis, lupus, rheumatoid arthritis, psoriatic arthritis, osteoarthritis, Still's disease, juvenile arthritis, diabetes mellitus, myasthenia gravis, Hashimoto's thyroiditis, Ord's thyroiditis, Graves' disease, rheumatoid arthritis syndrome, multiple sclerosis, infectious neuronitis, acute disseminated encephalomyelitis, Addison's disease, opsoclonus-myoclonus syndrome, ankylosing spondylitis, antiphospholipid antibody syndrome, aplastic anemia, autoimmune hepatitis, celiac disease, Goodpasture syndrome, immune thrombocytopenic purpura, optic neuritis, scleroderma, primary biliary cirrhosis, Reiter's syndrome, Takayasu arteritis, temporal arteritis, warm autoimmune hemolytic anemia, Wegener's granulomatosis, psoriasis, alopecia universalis, Behget's disease, chronic fatigue syndrome, familial dysautonomia, endometriosis, interstitial cystitis, neuromyotonia, scleroderma or vulvodynia, and chronic graft-versus-host disease.
